# EUROPEAN PATENT APPLICATION

(11) **EP 2 738 172 A1**
(43) Date of publication of application: **04.06.2014**
(21) Application number: 12382472.4
(22) Date of filing: 28.11.2012
(51) Int. Cl.: C07D 471/04, C07D 487/04, A61K 31/407, A61P 37/00

(54) **New bicyclic compounds as crac channel modulators**

(71) Applicant: Almirall, S.A., 08022 Barcelona (ES)
(72) Inventor: Vidal Juan, Bernat, 08980 Sant Feliu de Llobregat (ES); Gonzalez Rodriguez, Jacob, 08980 Sant Feliu de Llobregat (ES); Gual Roig, Silvia, 08980 Sant Feliu de Llobregat (ES); Esteve Trias, Cristina, 08980 Sant Feliu de Llobregat (ES); Vidal Gispert, Laura, 08980 Sant Feliu de Llobregat (ES)
(74) Representative: Elzaburu Marquez, Alberto

(57) **Abstract**

The present invention relates to novel compounds which are inhibitors of CRAC channel activity. This invention also relates to pharmaceutical compositions containing them, process for their preparation and their use in therapy.

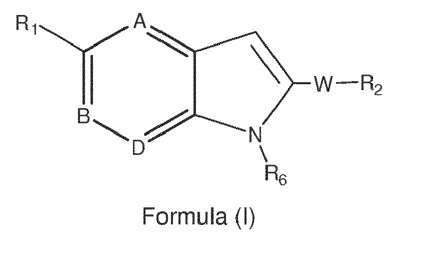

## Description

### FIELD OF THE INVENTION

The present invention relates to novel compounds which are inhibitors of CRAC channel activity. This invention also relates to pharmaceutical compositions containing them, process for their preparation and their use in therapy.

### BACKGROUND OF THE INVENTION

The regulation of intracellular calcium is a key element in the transduction of signals into and within cells. Cellular responses to growth factors, neurotransmitters, hormones and other signal molecules are initiated through Calcium-dependent processes. Virtually all cell types depend upon the generation of cytoplasmic calcium signals to regulate cell function or to trigger specific responses. Cytosolic calcium signals control a wide array of cellular functions ranging from short-term responses such as contraction and secretion to longer-term regulation of cell growth and proliferation. These signals involve combination of Calcium release from intracellular stores such as the endoplasmic reticulum (ER), and influx of Calcium across the plasma membrane.

In immune cells the molecular mechanisms of calcium signalling are especially well characterized. Cell activation through immunoreceptor engagement induces a rise in cytosolic calcium levels mainly through a selective channel-based process known as Store-Operated Calcium Entry (SOCE). Calcium Release-activated calcium (CRAC) channels are a type of SOC channels present in B and T-lymphocytes, NK cells, macrophages, DC and mast cells among the immune cells. CRAC channels are characterized by an extremely high ion selectivity for Ca²⁺ and a low conductance, and are activated through the Ca²⁺ sensors proteins in the ER called stromal interaction molecule (STIM) that bind to the pore forming unit of the CRAC channel in the plasma membrane, called ORAI. Upon antigen binding to the T-cell receptor, the activation of phospholipase C (PLCγ) generates the lipid metabolite InsP3, which promotes the release of Ca²⁺ from the ER stores. STIM proteins sense the calcium depletion in the ER, oligomerize and redistribute into discrete puncta located in junctional ER sites in close proximity to the plasma membrane. STIM proteins directly interact with ORAI1 in the plasma membrane opening the CRAC channel and allowing a sustained calcium entry across the plasma membrane (see Oh-hora, M. Immuno. Rev. 2009, 231, 210; Vig, M. Science 2006, 312, 1220; Vig, M. Nature Immunol. 2008, 9 (1), 89). Such a prolonged intracellular calcium increase activates short term processes such as degranulation, and cytosolic signal transduction processes involving gene transcription through NFAT to produce lipid mediators, several cytokines Th1, Th2, and Th17, matrix metalloproteinases, all of which participate in the pathogenesis of autoimmune and inflammation-based diseases.

Therefore, the involvement of deregulated lymphocyte Ca²⁺ signaling in the pathogenesis of autoimmune and inflammatory immune disorders suggests that interference with Ca²⁺ signaling through the CRAC channel may be a useful approach to treat autoimmune diseases, such as SLE or rheumatoid arthritis, and inflammatory disorders, such as respiratory diseases, psoriasis or ulcerative colitis, and to prevent allograft rejection in transplantation (Feske, S. Nature Reviews 2007, 7, 690).

More specifically, impaired CRAC channel activity has been linked to a growing number of diseases, including asthma, allergic diseases, autoimmunity, polyposis, inflammatory bowel disease and certain cancers. Therefore, CRAC channel inhibitors can be of considerable clinical benefit (Parekh, A. B. Nat. Rev. Drug Discov. 2010, 9, 399; Feske, S. Ann. N. Y. Acad. Sci. 2011, 1238, 74).

Patents and patent applications related to modulation of CRAC channel include WO2005009539, WO2006034402, WO2006081389, WO2007087427, WO2008039520, WO2009017818, WO2009035818 WO2009038775, W020120025295, W020100034003, WO20100039236, WO2010122088, WO2010122089, W02011139489, WO2012027710, WO2012064808, WO2012052458, WO2012052459, WO2012079020, WO2012151355. Patent application WO2011036130 discloses indole derivatives as CRAC modulators.

While progress has been made in this field, there remains the need to identify further compounds which are CRAC channel modulators.

The present invention relates to novel bicyclic derivatives which are inhibitors of CRAC channel activity. Such derivatives have potential therapeutic benefit in the treatment of disorders associated with inappropriate CRAC activity, in particular in the treatment and prevention of allergic, inflammatory and autoimmune disorders including asthma, chronic obstructive pulmonary disease, bronchiectasis, cystic fibrosis, nasal polyposis (Di Capite J. L. Curr. Opin. Allergy Clin. Immunol. 2011, 11, 33), allergic rhinitis (Wang, Y. Exp. Mol. Med. 2012, 44 (3), 177), cough (Sutovska, M., Advances in Experimental Medicine and Biology, Chapter 6, DOI 10.1007/978-94-007-4549-0_6), allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, myeloproliferative diseases, chronic lymphocytic leaukaemia, B cell lymphoma, breast and prostate cancer, immune thrombocytopenic purpura, macular degeneration (Shaw, P. Frontiers in Bioscience E4 2012, 2253; Parekh, A. B. Nat. Rev. Drug Discov. 2010, 9, 399).

In view of the numerous conditions that are contemplated to benefit by treatment involving the inhibition of calcium release-activated calcium channel (CRAC), it is immediately apparent that new compounds that inhibit CRAC pathways and use of these compounds should provide substantial therapeutic benefits to a wide variety of patients.

It has now been found that bicyclic heteroaryl derivatives are novel and potent CRAC ORAI1 inhibitors and can therefore be used in the treatment or prevention of these diseases.

### SUMMARY OF THE INVENTION

Thus, the present invention is directed to compounds of formula (I), or pharmaceutically acceptable salts, N-oxides, or isotopically-labeled derivates thereof. 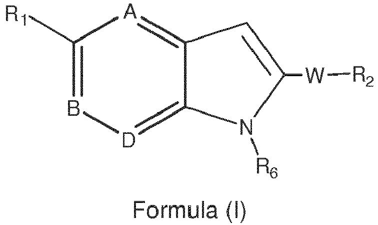 wherein,
A, B and D each independently represent a nitrogen atom or a -CH group, wherein at least one of A, B and D represents a nitrogen atom, and wherein at least one of A, B and D represents a -CH group;
W represents a linker selected from a direct bond and a -CH₂- group,
R₁ represents a monocyclic or bicyclic C₆-C₁₄ aryl group, a monocyclic or bicyclic 5- to 14- membered heteroaryl group containing at least one heteroatom selected from O, S and N, a tetrahydropyridinyl group or a bicyclyl group which is a monocyclic C₆-C₉ aryl or 5- to 9- membered heteroaryl group fused to a saturated or unsaturated C₅₋₉ cycloalkyl or to a saturated or unsaturated 5- to 9- membered heterocyclyl group, said heteroaryl or heterocyclyl group containing at least one heteroatom selected from O, S and N,
wherein the aryl, heteroaryl, tetrahydropyridinyl and the bicyclyl group are unsubstituted or substituted with one or more substituents selected from a halogen atom, a cyano group, a -(CH₂)₁₋₃CN group, a -(CH₂)_{n'}-OR₃ group, a -(CH₂)_{n'}-C(O)-(CH₂)₁₋₃-CN group, a -(CH₂)_{n'}-C(O)-(CH₂)ₙ-R₃ group, a -(CH₂)_{n'}-C(O)-(CH₂)ₙ-NR₄R₅ group, a -(CH₂)_{n'}-S(O)₂(CH₂)ₙR₃ group or a -(CH₂)_{n'}-S(O)₂(CH₂)ₙNR₄R₅ group, a linear or branched C₁-C₆ alkyl group, a C₁-C₄ haloalkyl group, a C₁-C₄ hydroxyalkyl group, a C₃-C₇ cycloalkyl group, a monocyclic or bicyclic C₆-C₁₄ aryl group, a monocyclic or bicyclic 5- to 14- membered heteroaryl group containing at least one heteroatom selected from O, S and N and a 5- to 14- membered heterocyclyl group containing at least one heteroatom selected from O, S and N,
R₂ represents a linear or branched C₁-C₉ alkyl group, a monocyclic or bicyclic C₃-C₁₀ cycloalkyl group, a monocyclic or bicyclic C₃-C₁₀ cycloalkenyl group, a monocyclic or bicyclic C₆-C₁₄ aryl group, a monocylic or bicyclic 5- to 14-membered heteroaryl group containing at least one heteroatom selected from N, O and S, a monocylic saturated or unsaturated 4- to 8- membered heterocyclyl group containing at least one heteroatom selected from O, S and N,
wherein the cycloalkyl, cycloalkenyl, aryl, heteroaryl and heterocyclyl groups are unsubstituted or substituted by one or more substituents selected from a halogen atom; a cyano group; a hydroxyl group; an oxo group, an ethylenedioxy group, a - (CH₂)₁₋₃-CN group, a -(CH₂)_{n'}-OR₃ group, a -(CH₂)_{n'}-C(O)-(CH₂)₁₋₃-CN group, a - (CH₂)_{n'}-C(O)O-R₃ group, a -(CH₂)_{n'}-C(O)-(CH₂)ₙ-R₃ group, a -(CH₂)_{n'}-C(O)-(CH₂)ₙ-NR₄R₅ group, a (CH₂)_{n'}-S(O)₂(CH₂)ₙR₃ group or a (CH₂)_{n'}-S(O)₂(CH₂)ₙNR₄R₅ group, a linear or branched C₁-C₆ alkyl group; a linear or branched C₁-C₄ haloalkyl group; a linear or branched C₁-C₄ hydroxyalkyl group; a C₃-C₇ cycloalkyl group; a monocyclic or bicyclic C₆-C₁₄ aryl group; a 5- to 14- membered heteroaryl group containing at least one heteroatom selected from O, S and N and a 5- to 14-membered heterocyclyl group containing at least one heteroatom selected from O, S and N;
each n' and n are 0, 1 or 2,
R₃ represents a linear or branched C₁-C₄ haloalkyl group, a linear or branched C₁-C₄ hydroxyalkyl group or a linear or branched C₁-C₆ alkyl group,
R₄ and R₅ each independently represent a hydrogen atom, a linear or branched C₁-C₄ haloalkyl group, a linear or branched C₁-C₄ hydroxyalkyl group or a linear or branched C₁-C₆ alkyl group, or R₄ and R₅ together with the nitrogen atom to which both R₄ and R₅ groups are bonded form a 4- to 7- membered, saturated N-containing heterocyclyl group optionally containing a further heteroatom selected from O, N and S and which heterocyclyl group is unsubstituted or substituted with one or more substituents selected from a halogen atom, a hydroxyl group, a cyano group, a -CHF₂ group or a - CF₃ group; R₆ is selected from the group consisting of a hydrogen atom and a C group, wherein C represents a prodrug-functional group,
wherein the compound of formula (I) is not one of 2,5-diphenyl-3H-pyrrolo[2,3-c]pyridine, 2-methyl-5-phenyl-1H-pyrrolo[2,3-b]pyridine, 2-ethyl-5-(3-fluorophenyl)-1H-pyrrolo[2,3-b]pyridine and 2-methyl-5-phenyl-1H-pyrrolo[2,3-c]pyridine.

The invention also provides synthetic processes and intermediates described herein, which are useful for preparing compounds of the invention.

The invention further provides a pharmaceutical composition comprising at least a compound of the invention and a pharmaceutically acceptable carrier.

The invention also provides a compound of the invention as described herein for use in the treatment of human or animal body by therapy.

The invention is also directed to the compounds as described herein, for use in the treatment of a pathological condition or disease associated with CRAC activity in particular wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease.

The invention also provides the use of the compounds of the invention as described herein, for the manufacture of a medicament for the treatment of a pathological condition or disease associated with CRAC activity, in particular wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease.

The invention is also directed to a method of treatment of a pathological condition or disease associated with CRAC activity, in particular wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, comprising administering a therapeutically effective amount of the compounds of the invention or a pharmaceutical composition of the invention to a subject in need of such treatment.

The invention also provides a combination product comprising (i) at least a compound of the invention as described herein; and (ii) one or more therapeutic active ingredients for simultaneous, separate or sequential use in the treatment of the human or animal body.

### DETAILED DESCRIPTION OF THE INVENTION.

When describing the compounds, compositions and methods of the invention, the following terms have the following meanings, unless otherwise indicated.

As used herein the term C₁-C₆ alkyl embraces linear or branched radicals having 1 to 6 carbon atoms. Examples include methyl, ethyl, n-propyl, i-propyl, n-butyl, sea-butyl , t-butyl, pentyl and hexyl radicals.

As used herein, the term C₁-C₄ haloalkyl group is a C₁-C₄ linear or branched alkyl group, which is substituted by one or more, preferably 1, 2 or 3 halogen atoms. Examples of such radicals include chlorometyl, chloroethyl, fluroromethyl, dicloroethyl difluoroethyl and trifluoromethyl.

As used herein, the term C₁-C₄ hydroxyalkyl embraces linear or branched alkyl radicals having 1 to 4 carbon atoms, any one of which may be substituted with one or more hydroxyl radicals. Examples of such radicals include hydroxymethyl, hydroxyethyl, hydroxypropyl,and hydroxybutyl.

As used herein, the term C₆-C₁₄ aryl radical embraces typically a C₆-C₁₄, preferably a C₆-C₁₀ monocyclic or polycyclic aryl radical such as phenyl, naphthyl, naphthalenyl, anthranyl and phenanthryl.

As used herein, the term 5- to 14- membered heteroaryl radical embraces typically a 5-to 14- membered ring system comprising at least one heteroaromatic ring and containing at least one heteroatom selected from O, S and N. A 5- to 14- membered heteroaryl radical may be a single ring or two or more fused rings wherein at least one ring contains a heteroatom.

Examples include pyridyl, pyrazinyl, pyrimidinyl, pyridazinyl, furyl, benzofuranyl, oxadiazolyl, oxazolyl, isoxazolyl, benzoxazolyl, imidazolyl, benzimidazolyl, thiazolyl, thiadiazolyl, thienyl, pyrrolyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2H-pyrazolo[3,4-d]pyrimidinyl, 1H-pyrazolo[3,4-d]pyrimidinyl, thieno[2,3-d] pyrimidinyl and the various pyrrolopyridyl radicals.

As used herein, the term C₃-C₁₀ cycloalkyl radical embraces saturated monocyclic or polycyclic carbocyclic radicals having from 3 to 10 carbon atoms, preferably from 3 to 7 carbon atoms. Polycyclic cycloalkyl radicals contain two or more fused cycloalkyl groups, preferably two cycloalkyl groups. Typically, polycyclic cycloalkyl radicals are selected from decahydronaphthyl (decalyl), bicyclo[2.2.2]octyl, adamantyl, camphyl or bornyl groups. Examples of monocyclic cycloalkyl groups include cyclopropyl, cyclobutyl, cyclopentyl, cyclohexyl, cycloheptyl, cyclooctyl, cyclononyl and cyclodecyl.

As used herein, the term C₃-C₁₀ cycloalkenyl radical embraces partially unsaturated carbocyclic radicals having from 3 to 10 carbon atoms, preferably from 3 to 7 carbon atoms. Examples include cyclobutenyl, cyclopentenyl, cyclohexenyl, cycloheptenyl, cyclooctenyl, cyclononenyl and cyclodecenyl.

As used herein, the term 4 to 8-membered heterocyclyl radical embraces typically a monocyclic non-aromatic, saturated or unsaturated C₄-C₈ carbocyclic ring system in which one or more, for example 1, 2 or 3 of the carbon atoms preferably 1 or 2 of the carbon atoms are replaced by a heteroatom selected from N, O and S.

It is to be understood that the term unsaturated encompases both partially and fully insaturated ring. In this regards, the insaturated (or non-saturated ring) may have one or more double bonds.

Examples of 4 to 8-membered heterocyclic radicals include piperidyl, pyrrolidyl, pyrrolinyl, piperazinyl, morpholinyl, thiomorpholinyl, pyrrolyl, pyrazolinyl, pirazolidinyl, quinuclidinyl, triazolyl, pyrazolyl, tetrazolyl, imidazolidinyl, imidazolyl, oxiranyl, thiaranyl, aziridinyl, oxetanyl, thiatanyl, azetidinyl and 4,5-dihydro-oxazolyl.

As used herein, the term halogen atom embraces chlorine, fluorine, bromine and iodine atoms. A halogen atom is typically a fluorine, chlorine or bromine atom, most preferably chlorine or fluorine. The term halo when used as a prefix has the same meaning.

Also included within the scope of the invention are the isomers, polymorphs, pharmaceutically acceptable salts, N-oxides, isotopes, solvates and prodrugs of the compounds of formula (I). Any reference to a compound of formula (I) throughout the present specification includes a reference to any isomer, polymorph, pharmaceutically acceptable salt, N-oxide, isotope, solvate or prodrug of such compound of formula (I).

### Isomers

Compounds containing one or more chiral centre may be used in enantiomerically or diastereoisomerically pure form, in the form of racemic mixtures and in the form of mixtures enriched in one or more stereoisomer. The compounds of Formula (I) as described and claimed encompass the racemic forms of the compounds as well as the individual enantiomers, diastereomers, and stereoisomer-enriched mixtures.

Conventional techniques for the preparation/isolation of individual enantiomers include chiral synthesis from a suitable optically pure precursor or resolution of the racemate using, for example, chiral high pressure liquid chromatography (HPLC). Alternatively, the racemate (or a racemic precursor) may be reacted with a suitable optically active compound, for example, an alcohol, or, in the case where the compound contains an acidic or basic moiety, an acid or base such as tartaric acid or 1-phenylethylamine. The resulting diastereoisomeric mixture may be separated by chromatography and/or fractional crystallization and one or both of the diastereoisomers converted to the corresponding pure enantiomer(s) by means well known to one skilled in the art. Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% isopropanol, typically from 2 to 20%, and from 0 to 5% of an alkylamine, typically 0.1 % diethylamine. Concentration of the eluate affords the enriched mixture. Stereoisomer conglomerates may be separated by conventional techniques known to those skilled in the art. See, e.g. "Stereochemistry of Organic Compounds" by Ernest L. Eliel (Wiley, New York, 1994).

Atropisomers are stereoisomers resulting from hindered rotation about single bonds where the steric strain barrier to rotation is high enough to allow for the isolation of the conformers. Oki (Oki, M; Topics in Stereochemistry 1983, 1) defined atropisomers as conformers that interconvert with a half-life of more than 1000 seconds at a given temperature. The scope of the invention as described and claimed encompasses the racemic forms of the compounds as well as the individual atropisomers (an atropisomer "substantially free" of tis corresponding enantionmer) and stereoisomer-enriched mixtures, i.e. mixtures of atropisomers.

Separation of atropisomers is possibly by chiral resolution methods such as selective crystallization. In an atropo-enantioselective or atroposelective synthesis one atropisomer is formed at the expense of the other. Atroposelective synthesis may be carried out by use of chiral auxiliaries like a Corey-Bakshi-Shibata (CBS) catalyst (asymmetric catalyst derived from proline) in the total synthesis of knipholone or by approaches based on thermodynamic equilibration when an isomerization reaction favors one atropisomer over the other.

The compounds of Formula (1) may exhibit the phenomena of tautomerism and structural isomerism. Tautomers exist as mixtures of a tautomeric set in solution. In solid form, usually one tautomer predominates. Even though one tautomer may be described, the present invention includes all tautomers of the compounds of Formula (1) -

### Polymorphs

The compounds of formula (I) may exist in different physical forms, i.e. amorphous and crystalline forms.

Moreover, the compounds of the invention may have the ability to crystallize in more than one form, a characteristic which is known as polymorphism. Polymorphs can be distinguished by various physical properties well known in the art such as X-ray diffraction pattern, melting point or solubility. All physical forms of the compounds of formula (I), including all polymorphic forms ("polymorphs") thereof, are included within the scope of the invention.

### Pharmaceutically acceptable salts

As used herein, the term pharmaceutically acceptable salt refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid; and organic acids, for example citric, fumaric, gluconic, glutamic, lactic, maleic, malic, mandelic, mucic, ascorbic, oxalic, pantothenic, succinic, tartaric, benzoic, acetic, methanesulphonic, ethanesulphonic, benzenesulphonic, p-toluenesulphonic acid, xinafoic (1-hydroxy-2-naphthoic acid), napadisilic (1,5-naphthalenedisulfonic acid) and the like. Particularly preferred are salts derived from fumaric, hydrobromic, hydrochloric, acetic, sulfuric, methanesulfonic, xinafoic, and tartaric acids.

Salts derived from pharmaceutically-acceptable inorganic bases include aluminum, ammonium, calcium, copper, ferric, ferrous, lithium, magnesium, manganic, manganous, potassium, sodium, zinc and the like. Particularly preferred are ammonium, calcium, magnesium, potassium and sodium salts.

Salts derived from pharmaceutically-acceptable organic bases include salts of primary, secondary and tertiary amines, including alkyl amines, arylalkyl amines, heterocyclyl amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

Other preferred salts according to the invention are quaternary ammonium compounds wherein an equivalent of an anion (X⁻) is associated with the positive charge of the N atom. X⁻ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and *p*-toluenesulphonate. X⁻ is preferably an anion selected from chloride, bromide, iodide, sulphate, nitrate, acetate, maleate, oxalate, succinate or trifluoroacetate. More preferably X⁻ is chloride, bromide, trifluoroacetate or methanesulphonate.

### N-oxides

As used herein, an N-oxide is formed from the tertiary basic amines or imines present in the molecule, using a convenient oxidising agent.

### Isotopes

The invention also includes isatapically-labeled derivatives of the compounds of the invention, wherein one or more atoms is replaced by an atom having the same atomic number, but an atomic mass or mass number different from the atomic mass or mass number usually found in nature. Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulfur, such as ³⁵S. Certain isotopically-labeled compounds of the invention, for example, those incorporating a radioactive isotope, are useful in drug and/or substrate tissue distribution studies. The radioactive isotopes tritium, ³H, and carbon-14, ¹⁴C , are particularly useful for this purpose in view of their ease of incorporation and ready means of detection. Substitution with heavier isotopes such as deuterium, ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled derivatives of the compounds of the invention can generally be prepared by conventional techniques known to those skilled in the art or by processes analogous to those described herein, using an appropriate isotopically-labeled reagent in place of the non-labeled reagent otherwise employed.

Preferred isotopically-labeled derivatives include deuterated derivatives of the compounds of the invention. As used herein, the term deuterated derivative embraces compounds of the invention where in a particular position at least one hydrogen atom is replaced by deuterium. Deuterium (D or ²H) is a stable isotope of hydrogen which is present at a natural abundance of 0.015 molar %.

### Solvates

The compounds of the invention may exist in both unsolvated and solvated forms. The term solvate is used herein to describe a molecular complex comprising a compound of the invention and an amount of one or more pharmaceutically acceptable solvent molecules. The term hydrate is employed when said solvent is water. Examples of solvate forms include, but are not limited to, compounds of the invention in association with water, acetone, dichloromethane, 2-propanol, ethanol, methanol, dimethylsulfoxide (DMSO), ethyl acetate, acetic acid, ethanolamine, or mixtures thereof. It is specifically contemplated that in the present invention one solvent molecule can be associated with one molecule of the compounds of the present invention, such as a hydrate.

Furthermore, it is specifically contemplated that in the present invention, more than one solvent molecule may be associated with one molecule of the compounds of the present invention, such as a dihydrate. Additionally, it is specifically contemplated that in the present invention less than one solvent molecule may be associated with one molecule of the compounds of the present invention, such as a hemihydrate. Furthermore, solvates of the present invention are contemplated as solvates of compounds of the present invention that retain the biological effectiveness of the non-solvate form of the compounds.

### Prodrugs

Prodrugs of the compounds described herein are also within the scope of the invention. Thus certain derivatives of the compounds of the present invention, which derivatives may have little or no pharmacological activity themselves, when administered into or onto the body may be converted into compounds of the present invention having the desired activity, for example, by hydrolytic cleavage. Such derivatives are referred to as 'prodrugs'. Further information on the use of prodrugs may be found in Pro-drugs as Novel Delivery Systems, Vol. 14, ACS Symposium Series (T. Higuchi and W. Stella) and Bioreversible Carriers in Drug Design, Pergamon Press, 1987 (ed. E. B. Roche, American Pharmaceutical Association).

Prodrugs in accordance with the invention can, for example, be produced by replacing appropriate functionalities present in the compounds of the present invention with certain moieties known to those skilled in the art as 'pro-moieties' as described, for example, in Design of Prodrugs by H. Bundgaard (Elsevier, 1985).

Typically, A is a nitrogen atom, B is a -CH group and D is a -CH group; or A is a nitrogen atom, B is a -CH group and D is a nitrogen atom; or A is a -CH group, B is a - CH group and D is a nitrogen atom; or A is a -CH group, B is a nitrogen atom and D is a -CH group; or A is a nitrogen atom, B is a nitrogen atom and D is a -CH group; or A is a -CH group, B is a nitrogen atom and D is a nitrogen atom.

Preferably, A is a nitrogen atom, B is a -CH group and D is a -CH group; or A is a nitrogen atom, B is a -CH group and D is a nitrogen atom; or A is a -CH group, B is a - CH group and D is a nitrogen atom; or A is a -CH group, B is a nitrogen atom and D is a -CH group.

Typically, W is a direct bond.

Typically R₁ represents a monocyclic C₆-C₈ aryl group, a monocyclic 5- to 8- membered heteroaryl group containing at least one heteroatom selected from O, S and N, a tetrahydropyridinyl group or a bicyclyl group which is a monocyclic C₆-C₉ aryl fused to a monocyclic C₅₋₉ cycloalkyl or to a monocyclic 5- to 9- membered heterocyclyl group, said heterocyclyl group containing at least one heteroatom selected from O, S and N,
wherein the aryl, heteroaryl, tetrahydropyridinyl and the bicyclyl group are unsubstituted or substituted by one or more substituents selected from a halogen atom, a cyano group, a -(CH₂)_{n'}-OR₃ group, a -(CH₂)_{n'}-C(O)-(CH₂)ₙ-R₃ group, a - (CH₂)_{n'}-C(O)-(CH₂)ₙ-NR₄R₅ group, a -(CH₂)ₙ-S(O)₂(CH₂)ₙR₃ group or a -(CH₂)_{n'}-S(O)₂(CH₂)ₙNR₄R₅ group, a linear or branched C₁-C₄ alkyl group, a C₁-C₄ haloalkyl group, a C₁-C₄ hydroxyalkyl group, a C₃-C₇ cycloalkyl group, a monocyclic C₆-C₈ aryl group, a monocyclic 5- to 8- membered heteroaryl group containing at least one heteroatom selected from O, S and N, a 5- to 14- membered heterocyclyl group containing at least one heteroatom selected from O, S and N, wherein each n' and n are 0, 1 or 2.

Preferably, R₁ represents a monocyclic C₆-C₈ aryl group, a monocyclic 5- to 8-membered heteroaryl group containing at least one heteroatom selected from O, S and N or a bicyclyl group which is a monocyclic C₆-C₉ aryl fused to a 5- to 9- membered heterocyclyl group, said heterocyclyl group containing at least one heteroatom selected from O, S and N, wherein the aryl, heteroaryl and the bicyclyl group are unsubstituted or substituted by one or more substituents selected from a halogen atom, a -(CH₂)_{n'}-OR₃ group, a - (CH₂)_{n'}-C(O)-(CH₂)ₙ-NR₄R₅ group or a -(CH₂)_{n'}-S(O)₂(CH₂)ₙNR₄R₅ group, wherein each n' and n are 0, 1 or 2, a C₁-C₂ alkyl group, and a C₁-C₂ haloalkyl group, wherein R₃ represent a C₁-C₂ haloalkyl group, a C₁-C₂ hydroxyalkyl group and a C₁₋₂ alkyl group, and R₄ and R₅ independently are selected from the group consisting of a hydrogen atom, a C₁-C₂ haloalkyl group, and a C₁₋₂ alkyl group, or R₄ and R₅ together with the nitrogen atom to which both R₄ and R₅ groups are bonded form a 4- to 6- membered, saturated N-containing heterocyclyl group optionally containing a further heteroatom selected from O, N and S and which heterocyclyl group is unsubstituted or substituted with one or more substituents selected from a halogen atom, a hydroxyl group, a cyano group or a -CF₃ group;

More preferably, R₁ represents a monocyclic 5- to 8- membered N-containing heteroaryl group and further containing at least one heteroatom selected from O, S and N or a bicyclyl group which is a phenyl group fused to a 5- to 6- membered heterocyclyl group containing at least one heteroatom selected from O, S and N, wherein the aryl and the bicyclyl group are unsubstituted or substituted by one or two substituents selected from a chlorine atom, fluorine atom, a -OR₃ group, a -S(O)₂NR₄R₅ group, a C₁-C₂ alkyl group, and a C₁-C₂ haloalkyl group, wherein R₃ represent a R₃ represents a C₁₋₂ alkyl group and a C₁-C₂ haloalkyl group, and R₄ and R₅ independently are selected from the group consisting of a hydrogen atom and a C₁₋₂ alkyl group, more preferably, the aryl and the bicyclyl group are unsubstituted or substituted by one or two substituents selected from a chlorine atom, fluorine atom, a methyl group, a methoxy group, a CF₃ group and a -S(O)₂NR₄R₅ group, wherein R₄ and R₅ independently are selected from the group consisting of a hydrogen atom and a methyl group.

Typically, R₂ represents a linear or branched C₁-C₆ alkyl group optionally substituted with one or more halogen atom (fluorine), a monocyclic C₃-C₇ cycloalkyl group, a monocyclic C₆-C₈ aryl group, a monocylic 5- to 8-membered heteroaryl group containing at least one heteroatom sleected from O and N, a saturated or unsaturated 4- to 8- membered heterocyclyl group containing at least one heteroatom selected from O and N, wherein the cycloalkyl, aryl, heteroaryl and heterocyclyl groups are unsubstituted or substituted by one or more substituents selected from a halogen atom; a cyano group; a hydroxyl group; an oxo group, a -(CH₂)_{n'}-OR₃ group, a -(CH₂)_{n'}-C(O)O-R₃ group, a -(CH₂)_{n'}-C(O)-(CH₂)ₙ-NR₄R₅ group, a -(CH₂)_{n'}-S(O)₂(CH₂)ₙR₃ group or a -(CH₂)_{n'}-S(O)₂(CH₂)ₙNR₄R₅ group, wherein each n' and n are 0, 1 or 2; a linear or branched C₁-C₄ alkyl group; a C₁-C₂ haloalkyl group; a C₁-C₂ hydroxyalkyl group; wherein R₃ represent a linear or branched C₁-C₄ haloalkyl group, a linear or branched C₁-C₄ hydroxyalkyl group and a C₁₋₂ alkyl group, and R₄ and R₅ independently are selected from the group consisting of a hydrogen atom, a C₁-C₂ haloalkyl group, and a C₁₋₂ alkyl group, or R₄ and R₅ together with the nitrogen atom to which both R₄ and R₅ groups are bonded form a 4- to 6- membered, saturated N-containing heterocyclyl group optionally containing a further heteroatom selected from O and N and which heterocyclyl group is unsubstituted or substituted with one or more substituents selected from a halogen atom, a hydroxyl group, a cyano group or a -CF₃ group;

Preferably, R₂ represents a monocyclic C₄-C₆ cycloalkyl group and a monocyclic C₆-C₈ aryl group, wherein the cycloalkyl, and the aryl groups are unsubstituted or substituted with one or two substituents selected from a fluorine atom, chlorine atom, a hydroxyl group; a methoxy group, a methyl group and a -CF₃ group, preferably R₂ represents a phenyl group , a cyclopentyl group and a cyclohexyl group which are optionally substituted with one or two substituents selected from a chlorine atom, a fluorine atom, a methyl group and a methoxy group.

Typically, C is selected from the group consisiting of a -(CH₂)-O-P(O)(OH)₂ and a - (CH₂)-O-(CO)-R₇, wherein R₇ is selected from the group consisting of a linear or branched C₁₋₄ alkyl group and a C₃₋₇ cycloalkyl group, preferably C represents -(CH₂)-O-P(O)(OH)₂ group.

Typically, R₆ is selected from a hydrogen atom and a -(CH₂)-O-P(O)(OH)₂ group.

Typically, compounds of the present invention have the following formulae: 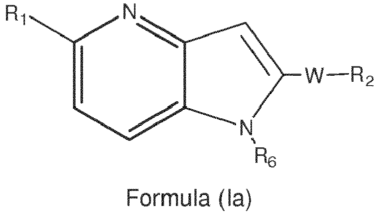 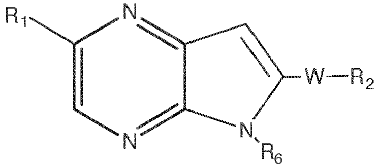 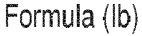 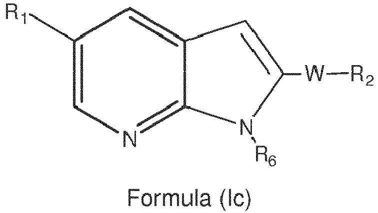 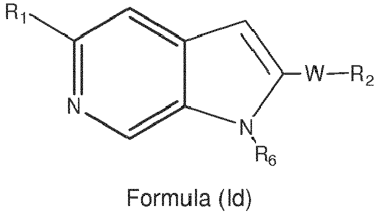 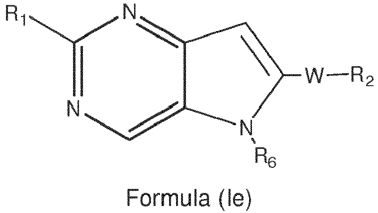 or 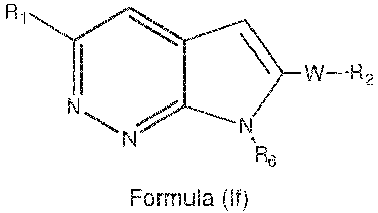 wherein W, R₁, R₂ and R₆ are as defined above.

Preferably, compounds of the present invention are selected from compounds of formula (Ia); 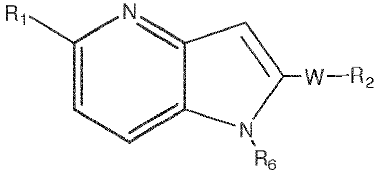 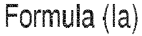 compounds of formula (Ib); 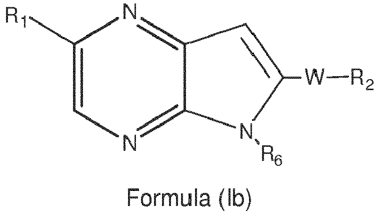 or compounds of formula (Ic);

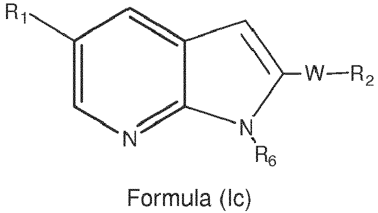

In another embodiment of the present invention,
A is a nitrogen atom, B is a -CH group and D is a -CH group; or A is a nitrogen atom, B is a -CH group and D is a nitrogen atom; or A is a -CH group, B is a -CH group and D is a nitrogen atom; or A is a -CH group, B is a nitrogen atom and D is a -CH group
W represents a direct bond or a -CH₂- group,
R₁ represents a phenyl group, a monocyclic 5- to 6- membered N-containing heteroaryl group and optionally further containing one or two nitrogen atom as heteroatom, or a bicyclyl group which is a phenyl group fused to a 5- to 9-membered saturated or unsaturated heterocyclyl group, said heterocyclyl group containing at least one heteroatom selected from O and N,
wherein the phenyl, heteroaryl and the bicyclyl group are substituted with two or three substituents selected from a halogen atom, a -OR₃ group, a -S(O)₂NR₄R₅ group, a linear or branched C₁-C₆ alkyl group, a C₁-C₄ haloalkyl group and a monocyclic 5- to 7- membered heteroaryl group containing at least one heteroatom selected from O and N,
R₂ represents a linear or branched C₁-C₄ alkyl group, a C₃-C₁₀ cycloalkyl group, a C₄-C₆ cycloalkenyl group, monocyclic C₆-C₁₄ aryl group, a monocylic a 5- to 6-membered heteroaryl group containing at least one heteroatom selected from O and N, a monocyclic saturated or unsaturated 4- to 8- membered heterocyclyl group containing at least one heteroatom selected from O and N,
wherein the cycloalkyl, cycloalkenyl, aryl, heteroaryl and heterocyclyl groups are unsubstituted or substituted with one or more substituents selected from a halogen atom; a cyano group; a hydroxyl group; an oxo group, an ethylenedioxy group, a - OR₃ group, a -C(O)O-R₃ group, a -S(O)₂NR₄R₅ group; a linear or branched C₁-C₆ alkyl group; a C₁-C₄ hydroxyalkyl group;
R₃ represents a C₁-C₄ haloalkyl group, a C₁-C₄ hydroxyalkyl group, a linear or branched C₁-C₄ alkyl group,
R₄ and R₅ each independently represent a hydrogen atom, or a linear or branched C₁-C₆ alkyl group, or R₄ and R₅ together with the nitrogen atom to which both R₄ and R₅ groups are bonded form a 4- to 7- membered, an unsubstituted saturated N-containing heterocyclyl group optionally containing a further heteroatom selected from O.
R₆ is selected from the group consisting of a hydrogen atom and a -(CH₂)-OP(O)(OH)₂.

Particular individual compounds of the invention include:
2-((2-ch)oro-6-fluorophenyl)-5-(6-methoxy-4-methylpyridin-3-yl)-1H-pyrrolo[2,3-b]pyridine,
6-((2-chloro-6-fluorophenyl)-2-(6-methoxy-4-methylpyridin-3-yl)-5H-pyrrolo[2,3-b]pyrazine,
6-((2-chloro-6-fluorophenyl)-2-(1-methyl-3-(trifluoromethyl)-1H-pyrazol-5-yl)-5H-pyrrolo[2,3-b]pyrazine,
2-((2,6-difluorophenyl)-5-(6-methoxy-4-methylpyridin-3-yl)-1H-pyrrolo[2,3-b]pyridine,
2-cyclohexyl-5-(6-methoxy-4-methylpyridin-3-yl)-1H-pyrrolo[2,3-b]pyridine,
5-((6-methoxy-4-methylpyridin-3-yl)-2-(4-methylpyridin-3-yl)-1H-pyrrolo[2,3-b]pyridine,
6-((2-chloro-6-fluorophenyl)-2-(1-ethyl-3-(trifluoromethyl)-1H-pyrazol-5-yl)-5H-pyrrolo[2,3-b]pyrazine,
4-((6-(2-chloro-6-fluorophenyl)-5H-pyrrolo[2,3-b]pyrazin-2-yl)-N,N,3-trimethylbenzenesulfonamide,
3-((3,5-difluoro-4-(5-(6-methoxy-4-methylpyridin-3-yl)-1H-pyrrolo[2,3-b]pyridin-2-yl)phenoxy)propan-1-ol,
6-cyclohexyl-2-(1-methyl-3-(trifluoromethyl)-1H-pyrazol-5-yl)-5H-pyrrolo[2,3-b]pyrazine,
2-((2-chloro-6-fluorophenyl)-5-(6-methoxy-4-methylpyridin-3-yl)-1H-pyrrolo[3,2-b]pyridine,
2-((2-chloro-6-fluorophenyl)-5-(1-methyl-3-(trifluoromethyl)-1H-pyrazol-5-yl)-1H-pyrrolo[2,3-b]pyridine,
5-((6-chloro-2,2-difluorobenzo[d][1,3]dioxol-5-yl)-2-(2-chloro-6-fluorophenyl)-1H-pyrrolo[2,3-b]pyridine,
2-((2-chloro-6-fluorophenyl)-5-(1-methyl-3-(trifluoromethyl)-1H-pyrazol-5-yl)-1H-pyrrolo[3,2-b]pyridine,
6-cyclohexyl-2-(6-methoxy-4-methylpyridin-3-yl)-5H-pyrrolo[2,3-b]pyrazine,
2-cyclohexyl-5-(1-methyl-3-(trifluoromethyl)-1H-pyrazol-5-yl)-1H-pyrrolo[3,2-b]pyridine,
2-cyclohexyl-5-(6-methoxy-4-methylpyridin-3-yl)-1H-pyrrolo[3,2-b]pyridine,
2-((2-chloro-6-fluorophenyl)-5-(1,3-dimethyl-1H-pyrazol-5-yl)-1H-pyrrolo[3,2-b]pyridine,
2-((4-methyl-3-(2-propyl-1H-pyrrolo[3,2-b]pyridin-5-yl)phenyl)oxazole,
2-((2-chloro-6-fluorophenyl)-5-(1-ethyl-3-(trifluoromethyl)-1H-pyrazol-5-yl)-1H-pyrrolo[3,2-b]pyridine,
2-((2,6-difluorophenyl)-5-(1-methyl-3-(trifluoromethyl)-1H-pyrazol-5-yl)-1H-pyrrolo[3,2-b]pyridine,
5-((1-methyl-3-(trifluoromethyl)-1H-pyrazol-5-yl)-2-propyl-1H-pyrrolo[3,2-b]pyridine,
5-((5-methyl-3-(trifluoromethyl)-1H-pyrazol-1-yl)-2-propyl-1H-pyrrolo[3,2-b]pyridine,
2-cyclopentyl-5-(1-methyl-3-(trifluoromethyl)-1H-pyrazol-5-yl)-1H-pyrrolo[3,2-b]pyridine,
2-cyclohexyl-5-(5-methyl-3-(trifluoromethyl)-1H-pyrazol-1-yl)-1H-pyrrolo[3,2-b]pyridine,
2-((2-chlorophenyl)-5-(1-methyl-3-(trifluoromethyl)-1H-pyrazol-5-yl)-1H-pyrrolo[3,2-b]pyridine,
1-((5-(1-methyl-3-(trifluoromethyl)-1H-pyrazol-5-yl)-1H-pyrrolo[3,2-b]pyridin-2-yl)cyclohexanol,
5-((1-methyl-3-(trifluoromethyl)-1H-pyrazol-5-yl)-2-(4-methylpyridin-3-yl)-1H-pyrrolo[3,2-b]pyridine,
2-((2,6-difluorophenyl)-5-(5-methyl-3-(trifluoromethyl)-1H-pyrazol-1-yl)-1H-pyrrolo[3,2-b]pyridine,
2-cyclohexyl-5-(5-(difluoromethoxy)-3-(trifluoromethyl)-1H-pyrazol-1-yl)-1H-pyrrolo[3,2-b]pyridine,
5-((1-methyl-3-(trifluoromethyl)-1H-pyrazol-5-yl)-2-(1,4-dioxaspiro[4.5]dec-7-en-8-yl)-1H-pyrrolo[3,2-b]pyridine,
tert-butyl 4-(5-(5-methyl-3-(trifluoromethyl)-1H-pyrazo)-1-yl)-1H-pyrrolo[3,2-b]pyridin-2-yl)piperidine-1-carboxylate,
2-((5-(1-methyl-3-(trifluoromethyl)-1H-pyrazol-5-yl)-1H-pyrrolo[3,2-b]pyridin-2-yl)benzonitrile,
4-((5-(1-methyl-3-(trifluoromethyl)-1H-pyrazol-5-yl)-1H-pyrrolo[3,2-b]pyridin-2-yl)cyclohexanol,
2-((2-fluorophenyl)-5-(1-methyl-3-(trifluoromethyl)-1H-pyrazol-5-yl)-1H-pyrrolo[3,2-b]pyridine,
(2-((5-(1-methyl-3-(trifluoromethyl)-1H-pyrazol-5-yl)-1H-pyrrolo[3,2-b]pyridin-2-yl)phenyl)methanol,
2-((3,5-difluoro-4-(5-(1-methyl-3-(trifluoromethyl)-1H-pyrazol-5-yl)-1H-pyrrolo[3,2-b]pyridin-2-yl)phenoxy)ethanol,
3-((3,5-difluoro-4-(5-(1-methyl-3-(trifluoromethyl)-1H-pyrazol-5-yl)-1H-pyrrolo[3,2-b]pyridin-2-yl)phenoxy)propan-1-ol,
4-(((4-(2-(2-chloro-6-fluorophenyl)-1H-pyrrolo[2,3-b]pyridin-5-yl)-3-methylphenyl)sulfonyl)morpholine,
5-((1-methyl-3-(trifluoromethyl)-1H-pyrazol-5-yl)-2-(o-tolyl)-1H-pyrrolo[3,2-b]pyridine,
5-((1-methyl-3-(trifluoromethyl)-1H-pyrazol-5-yl)-2-(1,4-dioxaspiro[4.5]decan-8-yl)-1H-pyrrolo[3,2-b]pyridine,
2-((3,6-dihydro-2H-pyran-4-yl)-5-(1-methyl-3-(trifluoromethyl)-1H-pyrazol-5-yl)-1H-pyrrolo[3,2-b]pyridine,
4-((5-(1-methyl-3-(trifluoromethyl)-1H-pyrazol-5-yl)-1H-pyrrolo[3,2-b]pyridin-2-yl)cyclohexanone,
4-((2-(2-chloro-6-fluorophenyl)-1H-pyrrolo[2,3-b]pyridin-5-yl)-N,N,3-trimethylbenzenesulfonamide,
2-((2-methoxyphenyl)-5-(1-methyl-3-(trifluoromethyl)-1H-pyrazol-5-yl)-1H-pyrrolo[3,2-b]pyridine,
5-((1-methyl-3-(trifluoromethyl)-1H-pyrazol-5-yl)-2-(tetrahydro-2H-pyran-4-yl)-1H-pyrrolo[3,2-b]pyridine,
N,N,3-trimethyl-4-(5-(1-methyl-3-(trifluoromethyl)-1H-pyrazol-5-yl)-1H-pyrrolo[3,2-b]pyridin-2-yl)benzenesulfonamide,
5-fluoro-2-(5-(1-methyl-3-(trifluoromethyl)-1H-pyrazo)-5-yl)-1H-pyrrolo[3,2-b]pyridin-2-yl)benzonitrile,
2-((4,4-difluorocyclohexyl)-5-(1-methyl-3-(trifluoromethyl)-1H-pyrazol-5-yl)-1H-pyrrolo[3,2-b]pyridine,
5-((1-methyl-3-(trifluoromethyl)-1H-pyrazol-5-yl)-2-phenyl-1H-pyrrolo[3,2-b]pyridine,
2-((4-butoxy-2-methylphenyl)-5-(1-methyl-3-(trifluoromethyl)-1H-pyrazol-5-yl)-1H-pyrrolo[3,2-b]pyridine,
2-isobutyl-5-(1-methyl-3-(trifluoromethyl)-1H-pyrazol-5-yl)-1H-pyrrolo[3,2-b]pyridine,
2-((4-fluoro-2-methylphenyl)-5-(1-methyl-3-(trifluoromethyl)-1H-pyrazol-5-yl)-1H-pyrrolo[3,2-b]pyridine,
2-((5-fluoro-2-methylphenyl)-5-(1-methyl-3-(trifluoromethyl)-1H-pyrazol-5-yl)-1H-pyrrolo[3,2-b]pyridine,
2-((2,3-difluorophenyl)-5-(1-methyl-3-(trifluoromethyl)-1H-pyrazol-5-yl)-1H-pyrrolo[3,2-b]pyridine,
2-fluoro-6-(5-(1-methyl-3-(trifluoromethyl)-1H-pyrazol-5-yl)-1H-pyrrolo[3,2-b]pyridin-2-yl)benzonitrile,
2-((5-Chloro-2-methylphenyl)-5-[1-methyl-3-(trifluoromethyl)-1*H*-pyrazol-5-yl]-1*H-*pyrrolo[3,2-*b*]pyridine,
2-((3-fluoropyridin-4-yl)-5-(1-methyl-3-(trifluoromethyl)-1H-pyrazol-5-yl)-1H-pyrrolo[3,2-b]pyridine,
2-((2-chloro-6-fluorophenyl)-5-(5-methyl-3-(trifluoromethyl)-1H-pyrazal-1-yl)-1H-pyrrolo[2,3-b]pyridine,
4-((2-(2-chlorophenyl)-1H-pyrrolo[3,2-b]pyridin-5-yl)-N,N,3-trimethylbenzenesulfonamide,
4-(((4-(6-(2-chloro-6-fluorophenyl)-5H-pyrrolo[2,3-b]pyrazin-2-yl)-3-methylphenyl)sulfonyl)morpholine,
2-((3-fluoro-2-methylphenyl)-5-(1-methyl-3-(trifluoromethyl)-1H-pyrazol-5-yl)-1H-pyrrolo[3,2-b]pyridine,
2-((2-chlorophenyl)-5-(6-methoxy-4-methylpyridin-3-yl)-1H-pyrrolo[2,3-c]pyridine,
2-((2-fluoro-6-methylphenyl)-5-(1-methyl-3-(trifluoromethyl)-1H-pyrazol-5-yl)-1H-pyrrolo[3,2-b]pyridine,
4-((6-(2-chloro-6-fluorophenyl)-5H-pyrrolo[2,3-b]pyrazin-2-yl)-3-methylbenzenesulfonamide,
2-((2-chloro-4-fluorophenyl)-5-(1-methyl-3-(trifluoromethyl)-1H-pyrazol-5-yl)-1H-pyrrolo[3,2-b]pyridine,
6-cyclopentyl-2-(6-methoxy-4-methylpyridin-3-yl)-5H-pyrrolo[2,3-b]pyrazine,
6-((2,6-difluorophenyl)-2-(6-methoxy-4-methylpyridin-3-yl)-5H-pyrrolo[2,3-b]pyrazine,
4-((6-(2-chloro-6-fluorophenyl)-5H-pyrrolo[2,3-b]pyrazin-2-yl)-N,3-dimethylbenzenesulfonamide,
2-((2-chloro-6-methylphenyl)-5-(1-methyl-3-(trifluoromethyl)-1H-pyrazol-5-yl)-1H-pyrrolo[3,2-b]pyridine,
2-((2-chloro-5-fluorophenyl)-5-(1-methyl-3-(trifluoromethyl)-1H-pyrazol-5-yl)-1H-pyrrolo[3,2-b]pyridine,
2-benzyl-5-(1-methyl-3-(trifluoromethyl)-1H-pyrazol-5-yl)-1H-pyrrolo[3,2-b]pyridine,
5-chloro-6-(2-(2-chloro-6-fluorophenyl)-1H-pyrrolo[2,3-b]pyridin-5-yl)-2-methylbenzo[d]oxazole,
2-((2-chloro-3-fluorophenyl)-5-(1-methyl-3-(trifluoromethyl)-1H-pyrazol-5-yl)-1H-pyrrolo[3,2-b]pyridine,
2-((2-chlorophenyl)-5-(6-methoxy-4-methylpyridin-3-yl)-1H-pyrrolo[2,3-b]pyridine,
2-((2-fluorophenyl)-5-(6-methoxy-4-methylpyridin-3-yl)-1H-pyrrolo[2,3-b]pyridine,
2-((2-chloro-6-fluorophenyl)-5-(5-methyl-3-(trifluoromethyl)-1H-1,2,4-triazol-1-yl)-1H-pyrrolo[3,2-b]pyridine,
3,5-dimethyl-4-(5-(1-methyl-3-(trifluoromethyl)-1H-pyrazol-5-yl)-1H-pyrrolo[3,2-b]pyridin-2-yl)isoxazole,
6-((2,6-difluorophenyl)-2-(1-methyl-3-(trifluoromethyl)-1H-pyrazol-5-yl)-5H-pyrrolo[2,3-b]pyrazine,
2-((2-fluorophenyl)-5-(6-methoxy-4-methylpyridin-3-yl)-1H-pyrrolo[2,3-c]pyridine,
2-((2-chloro-5-fluorophenyl)-5-(6-methoxy-4-methylpyridin-3-yl)-1H-pyrrolo[2,3-b]pyridine,
2-((4-isopropoxy-2-methylphenyl)-5-(1-methy)-3-(trifluoromethyl)-1H-pyrazol-5-yl)-1H-pyrrolo[3,2-b]pyridine,
2-((4-ethoxy-2-methylphenyl)-5-(1-methyl-3-(trifluoromethyl)-1H-pyrazol-5-yl)-1H-pyrrolo[3,2-b]pyridine,
2-((2-chloro-6-fluorophenyl)-5-(6-methoxy-4-methylpyridin-3-yl)-1H-pyrrolo[2,3-c]pyridine,
2-((2,6-difluorophenyl)-5-[1-methyl-3-(trifluoromethyl)-1*H*-pyrazol-5-yl]-1*H-*pyrrolo[2,3-*c*]pyridine,
2-((2-chloro-6-fluorophenyl)-5-(4-methyl-6-(trifluoromethyl)pyridin-3-yl)-1H-pyrrolo[2,3-b]pyridine,
2-((2-chloro-6-fluorophenyl)-5-(4,6-dimethoxypyridin-3-yl)-1H-pyrrolo[2,3-b]pyridine,
{2-((2-Chloro-6-fluorophenyl)-5-[1-methyl-3-(trifluoromethyl)-1*H*-pyrazol-5-yl]-1*H-*pyrrolo[3,2-b]pyridin-1-yl}methyl dihydrogen phosphate,
2-Cyclopentyl-5-(1-methyl-3-(trifluoromethyl)-1*H*-pyrazol-5-yl)-1*H*-pyrrolo[2,3-*b*]pyridine,
2-((2-Chloro-6-fluorophenyl)-5-(4,6-dimethoxypyridin-3-yl)-1*H*-pyrrolo[3,2-*b*]pyridine, 2-(2,6-Difluorophenyl)-5-(1-methyl-3-(trifluoromethyl)-1*H*-pyrazol-5-yl)-1*H-*pyrrolo[2,3-*b*]pyridine,
2-((2-Chloro-6-fluorophenyl)-5-(2-methyl-4-(methylsulfonyl)phenyl)-1*H*-pyrrolo[3,2-b]pyridine,
2-((2-Chlorobenzyl)-5-(1-methyl-3-(trifluoromethyl)-1*H*-pyrazol-5-yl)-1*H*-pyrrolo[3,2-*b*]pyridine,
2-((2-Chloro-6-fluorophenyl)-5-(1-methyl-5-(trifluoromethyl)-1*H*-1,2,4-triazol-3-yl)-1*H-*pyrrolo[3,2-*b*]pyridine,
4-((2-(2-Chlorophenyl)-1*H*-pyrrolo[3,2-*b*]pyridin-5-yl)-N,3-dimethylbenzenesulfonamide,
5-Chloro-6-(2-(2-chloro-6-fluorophenyl)-1H-pyrrolo[3,2-b]pyridin-5-yl)-2-methylbenzo[*d*]oxazole,
4-((2-(2-Chloro-6-fluorophenyl)-1*H*-pyrrolo[3,2-b]pyridin-5-yl)-*N*-methyl-3-(trifluoromethyl)benzenesulfonamide,
2-((2-Chloro-6-fluorophenyl)-5-(4-methyl-6-(trifluoromethyl)pyridin-3-yl)-1*H-*pyrrolo[3,2-*b*]pyridine,
4-((2-(2-Chloro-6-fluorophenyl)-1H-pyrrolo[3,2-b]pyridin-5-yl)-N,3-dimethylbenzamide,
Methyl 3-(2-(2-chloro-6-fluorophenyl)-1*H*-pyrrolo[2,3-b]pyridin-5-yl)-4-methyl-5,6-dihydropyridine-1(2*H*)-carboxylate,
5-((2-(2-Chloro-6-fluorophenyl)-1*H*-pyrrolo[2,3-b]pyridin-5-yl)-*N*,4-dimethylpyridin-2-amine,
1-((4-(2-(2-Chloro-6-fluorophenyl)-1*H*-pyrrolo[2,3-b]pyridin-5-yl)-3-methylphenyl)-*N-*methylmethanesulfonamide,
2-Cyclopentyl-5-(6-(difluoromethoxy)-4-methylpyridin-3-yl)-1*H*-pyrrolo[3,2-*b*]pyridine,
2-((*tert-*Butyl)-5-(1-methyl-3-(trifluoromethyl)-1*H*-pyrazol-5-yl)-1*H*-pyrrolo[3,2-b]pyridine,
5-Chloro-6-(2-cyclopentyl-1*H*-pyrrolo[2,3-*b*]pyridin-5-yl)-2-methylbenzo[*d*]oxazole, 6-(2-Chloro-6-fluorophenyl)-2-(4-methyl-6-(trifluoromethyl)pyridin-3-yl)-5H-pyrrolo[2,3-*b*]pyrazine,
4-((6-(2-Chloro-6-fluorophenyl)-5*H*-pyrrolo[2,3-b]pyrazin-2-yl)-*N*-methyl-3-(trifluoromethyl)benzenesulfonamide, and
2-((2-Chloro-6-fluorophenyl)-5-(3-methyl-1-(phenylsulfonyl)-1*H*-pyrazol-4-yl)-1*H-*pyrrolo[2,3-*b*]pyridine,
and Pharmaceutical acceptable salts, N-oxides, and isotopically-labeled derivates thereof.

Of outstanding interest are:
2-((2-chloro-6-fluorophenyl)-5-(6-methoxy-4-methylpyridin-3-yl)-1H-pyrrolo[2,3-b]pyridine,
6-((2-chloro-6-fluorophenyl)-2-(6-methoxy-4-methylpyridin-3-yl)-5H-pyrrolo[2,3-b]pyrazine,
2-((2-chloro-6-fluorophenyl)-5-(1-methyl-3-(trifluoromethyl)-1H-pyrazol-5-yl)-1H-pyrrolo[2,3-b]pyridine,
2-((2-chloro-6-fluorophenyl)-5-(1-methyl-3-(trifluoromethyl)-1H-pyrazol-5-yl)-1H-pyrrolo[3,2-b]pyridine,
2-cyclohexyl-5-(1-methyl-3-(trifluoromethyl)-1H-pyrazol-5-yl)-1H-pyrrolo[3,2-b]pyridine,
2-cyclopentyl-5-(1-methyl-3-(trifluoromethyl)-1H-pyrazol-5-yl)-1H-pyrrolo[3,2-b]pyridine,
4-((6-(2-chloro-6-fluorophenyl)-5H-pyrrolo[2,3-b]pyrazin-2-yl)-N,3-dimethylbenzenesulfonamide,
5-chloro-6-(2-(2-chloro-6-fluorophenyl)-1H-pyrrolo[2,3-b]pyridin-5-yl)-2-methylbenzo[d]oxazole,
2-((2,6-difluorophenyl)-5-[1-methyl-3-(trifluoromethyl-1*H*-pyrazol-5-yl]-1*H-*pyrrolo[2,3-*c*]pyridine, and
2-((2-chloro-6-fluorophenyl)-5-(4-methyl-6-(trifluoromethyl)pyridin-3-yl)-1H-pyrrolo[2,3-b]pyridine,
and Pharmaceutical acceptable salts, N-oxides and and isotopically-labeled derivates thereof.

The invention also provides a compound of the invention as described herein for use in the treatment of human or animal body by therapy.

The invention is also directed to the compounds as described herein, for use in the treatment of a pathological condition or disease associated with CRAC activity in particular wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease.

The invention also provides the use of the compounds of the invention as described herein, for the manufacture of a medicament for the treatment of a pathological condition or disease associated with CRAC activity, in particular wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease.

The invention is also directed to a method of treatment of a pathological condition or disease associated with CRAC activity, in particular wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, comprising administering a therapeutically effective amount of the compounds of the invention or a pharmaceutical composition of the invention to a subject in need of such treatment.

The invention also provides a combination product comprising (i) at least a compound of the invention as described herein; and (ii) one or more other therapeutic active ingredients, for simultaneous, separate or sequential use in the treatment of the human or animal body.

As used herein, the term therapeutically effective amount refers to an amount sufficient to effect treatment when administered to a patient in need of treatment.

As used herein, the term treatment refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

As used herein, the term disease or condition associated with CRAC activity includes all disease states and/or conditions that are acknowledged now, or that are found in the future, to be associated with CRAC activity. Such disease states include, but are not limited to, an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease.

### GENERAL SYNTHETIC PROCEDURES

The compounds of the invention can be prepared using the methods and procedures described herein, or using similar methods and procedures. It will be appreciated that where typical or preferred process conditions (i.e., reaction temperatures, times, mole ratios of reactants, solvents, pressures, etc.) are given; other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group, as well as suitable conditions for protection and deprotection, are well known in the art. For example, numerous protecting groups, and their introduction and removal are described in T. W. Greene and G. M. Wuts, Protecting Groups in Organic Synthesis, Third Edition, Wiley, New York, 1999, and references cited therein.

Processes for preparing compounds of the invention are provided as further embodiments of the invention and are illustrated by the procedures below.

Compounds of general formula (I) may be prepared following the synthetic scheme depicted in Scheme 1.

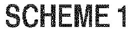

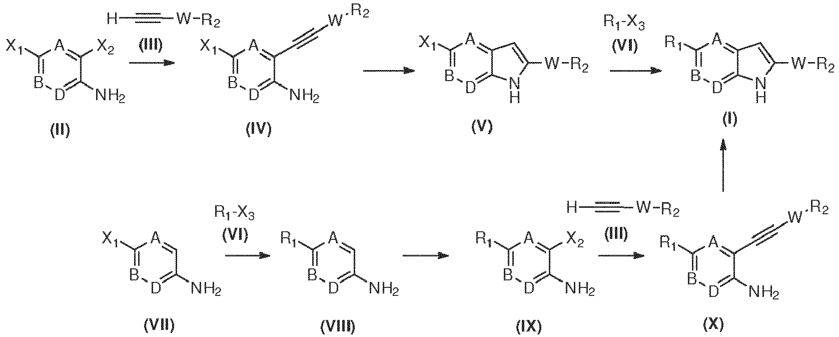

Regioselective Sonogashira coupling reaction between intermediate (II) where X₁ and X₂ represent different halogen atoms with alkyne (III) in a solvent that is inert to the reaction conditions such as THF, using an organic base, preferably triethylamine, copper (preferably copper (I) iodide) and palladium (such as dichlorobis(triphenylphosphine)palladium (II)) as catalysts at a range of temperatures between 70ºC to 150ºC provides intermediates of general formula (IV). These compounds can be converted into compounds of formula (V) by cyclization mediated by the use of a suitable catalyst e.g. copper (preferably copper (I) iodide) or palladium in polar aprotic solvents such as dimethylformamide and at a temperature range of 70ºC to 150ºC. Alternatively, cyclization from (IV) to (V) can be performed in the presence of a bsae such as potassium *tert*-butoxyde in a polar aprotic solvent such as *N*-methyl-2-pyrrolidone.

Further Suzuki type reaction (Miyaura, N.; Suzuki, A. Chem. Rev. 1995, 95, 2457) of intermediate (V) with R₁-X₃ (VI) where X₃ represents a boronic or boronate groups provide compounds of general formula (I). These reactions may be catalyzed by a palladium catalyst like [1,1'-bis(diphenylphosphino)-ferrocene] dichloropalladium (II) complex with dichloromethane (1:1), tetrakis(triphenylphosphine)-palladium(0), bis(triphenylphosphine)palladium(II) chloride or tris(dibenzylideneacetone)-dipalladium(0) in an aprotic organic solvent such as dioxane, toluene, DMF or DME and in the presence of a base such as cesium carbonate, sodium carbonate or potassium phosphate at a temperature from 80ºC to 140ºC.

Alternatively, when X₃ is hydrogen in intermediate (VI), compounds of formula (I) can be obtained by Buchwald-Hartwig-type *N*-arylation of the corresponding nitrogen containing heterocyclic ring (VI). These reactions may be catalyzed by copper iodide and a ligand such as *trans*-*N*,*N*'-dimethylcyclohexane-1,2-diamine in an organic solvent such as toluene, *N*-methylpyrrolidone or dioxane and in the presence of a base such as potassium phosphate or cessium carbonate at a temperature from 60ºC to 150ºC, using conventional heating or microwave reactor.

Similarly, Stille-type cross coupling of intermediate (V) using the organotin derivative of (VI) in the presence of palladium catalysts such as tetrakis(triphenylphosphine) palladium (0) in solvents such as xylene or dimethylformamide at a temperature between 25ºC to 200ºalso provides compounds of general formula (I).

Similarly, Negishi-type cross coupling of intermediate (V) using the organozinc derivative of (VI) in the presence of palladium catalysts such as tetrakis (triphenylphosphine)palladium (0) in solvents such as tetrahydrofuran at a temperature between 25ºC to 180ºC also provides compounds of general formula (I).

In another synthetic pathway, aminoheterocycles (VIII) can be prepared starting from haloheterocycles (VI) by Suzuki, Stille, Negishi or Buckwatd-Hartwig-type coupling reactions using the corresponding R₁-X₃ reagent under the standard procedures for described above to yield the compounds of formula (VIII). Subsequent regioselective bromination or iodination of the compounds of formula (VIII) using reagents such as Br₂, I₂ or *N*-halosuccinimide in polar aprotic solvents such as DMF and at temperatures ranging from 0ºC to 100ºC, yield the compounds of formula (IX).

A further Sonogashira-type reaction of the compounds of formula (IX) using the alkyne derivative (III) under the standard procedures described above provides the compounds of formula (X) that upon cyclization using the standard conditions described above provides compounds of general formula (I).

In another synthetic pathway, compounds of general formula (Ia) may also be prepared following the synthetic scheme depicted in Scheme 2.

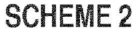

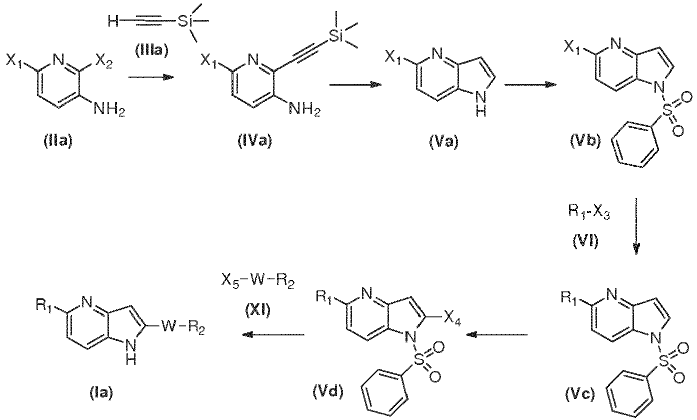

Regioselective Sonogashira-type reaction of trimethylsilylacetylene (IIIa) with dihaloaminopyridine (IIa) under the standard conditions described above followed by copper or base catalyzed cyclization of intermediate (lVa) under the above conditions provides intermediate (Va) usually with spontaneous desilylation. Further reaction of (Va) with benzenesulphonyl chloride in the presence of a base such as sodium hydride provides protected compound (Vb). Suzuki, Stille, Negishi or Buckwatd-Hartwig-type coupling reactions using the corresponding R₁-X₃ reagent (VI) under the standard procedures described above to yield the compounds of formula (Vc). Compounds of general formula (Vd) where X₄ is halogen can be obtained by regioselective lithiation of compounds of formula (Vc) with a solution of BuLi in hexanes and subsequent addition of iodine or bromine at a temperature from -78ºC to room temperature.

Intermediate (Vd) where X4 is halogen can be converted to compounds of general formula (Ia) by Suzuki, Stille, Negishi or Buckwald-Hartwig-type coupling reactions using the corresponding derivatives of general formula (XI). Additionally, intermediate (Vd) can be converted to the corresponding boronate in X₄ with a suitable reagent such as bis(pinacolato)diboron in the presence of a suitable base such as potassium acetate and a palladium catalyst such as [1,1'-bis(diphenylphosphino)ferrocene]dichloropalladium(II) complex with dichloromethane (1:1) in an aprotic organic solvent such as dioxane, *N,N'*-dimethylformamide or dimethylsulphoxide at a temperature ranging from 80 to 120 ºC. Further Suzuki-type reaction with derivatives of general formula (XI) where X₅ is an halogen provides compounds of general formula (Ia).

Specific intermediates of general formula (VIIIa) may also be prepared following the synthetic scheme depicted in Scheme 3.

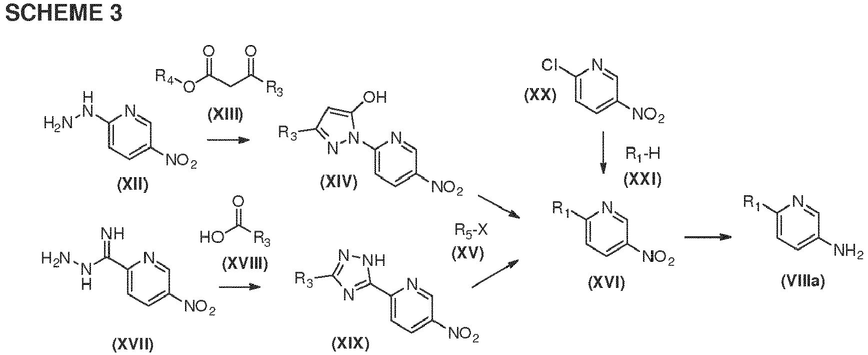

Condensation of 2-hydrazinyl compound (XII) with ketoester (XIII) under acidic conditions (for instance under in a saturated solution of hydrochloric acid in dioxane) or condensation of imidohidrazide (XVII) with carboxylic acid (XVIII) provide key intermediates (XIV) and (XIX) respectively. Further alkylation reaction with haloderivatives (XV) in the presence of a base such as potassium carbonate and a polar aprotic solvent such as dimethylformamide leads to nitropyridines of general formula (XVI).

Additionally, nitropyridines (XVI) can be prepared by direct arylarion of 2-chloropyridine (XX) using, for instance Buckwald-Hartwig-type reaction conditions. Further reduction of (XVI) using iron and ammonium chloride under reflux of ethanol and water provides key amino intermediate of general formula (VIIIa).

In another synthetic pathway, compounds of general formula (Ic) may be prepared following the synthetic scheme depicted in Scheme 4.

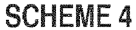

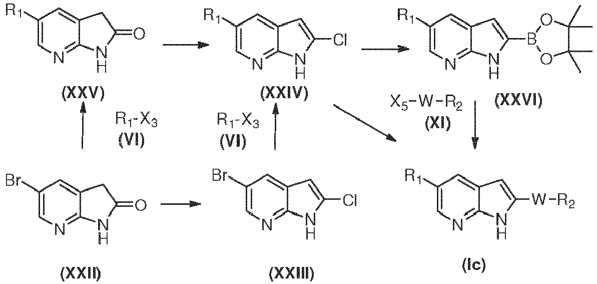

5-Bromo-1*H*-pyrrolo[2,3-b]pyridin-2(3*H*)-one (XXII) can be converted into the 2-chloro analogue of formula (XXIII) by treatment with chlorinating agents such as POCl₃, PCl₅ or PhPOCl₂ or by using a combination of such reagents. A further chemoselective Suzuki, Stille, Negishi or Buckwald-Hartwig-type coupling using the corresponding 1-X₃ reagent (VI) under the standard procedures described above provides the 2-chloro-7-azaindoles (XXIV). Alternatively intermediate (XVIV) can be prepared by initial coupling of lactam (XXII) to provide intermediate of formula (XXV) and then chlorination to provide intermediate (XXIV) following the conditions described above. Intermediate of formula (XXIV) can be converted either directly to compounds of general formula (Ic) using the same conditions as described for the synthesis of (Ia) from (Vd) or by sequential boronate formation by reaction for instance with bis(pinnacolato)diboron, [1,1'-bis(diphenylphosphino)ferrocene]-dichloropalladium (II) complex (PdCl₂(ppdf)) and potassium acetate in a solvent such as dimethylformamide, or with isopropyl magnesium chloride and triisopropyl in a solvent such as tetrahydrofuran and subsequent Suzuki-type coupling with intermediate (XI) where X₅ is an halogen.

### EXAMPLES

The syntheses of the compounds of the invention are illustrated by the following Examples (1 to 88) including Preparations (1 to 64) which do not limit the scope of the invention in any way.

### General

Reagents, starting materials, and solvents were purchased from commercial suppliers and used as received. Concentration or evaporation refers to evaporation under vacuum using a Büchi rotatory evaporator.

Reaction products were purified, when necessary, by flash chromatography on silica gel (40-63 µm) with the solvent system indicated. Purifications in reverse phase were made in a Biotage SP1® automated purification system equipped with a C₁₈ column and using a gradient of water/acetonitrile/MeOH (1:1) (0.1% v/v ammonium formate both phases) from 0% to 100% acetonitrile/MeOH (1:1) in 40 column volumes. The appropriate fractions were collected and the solvents evaporated under reduced pressure and/or liofilized.

Preparative HPLC-MS were performed on a Waters instrument equipped with a 2767 injector/cor/collector, a 2525 binary gradient pump, a 2996 PDA detector, a 515 pump as a make-up pump and a ZQ4000 Mass spectrometer detector.

The chromatographic separations were obtained using a Waters 2795 system equipped with a Symmetry C₁₈ (2.1 x 50 mm, 3.5 µm) column for methods A , and C and a Symmetry C₁₈ (2.1 x 100 mm, 3.5 µm) for method D. The mobile phase was formic acid (0.4 ml), ammonia (0.1 ml), methanol (500 ml) and acetonitrile (500 ml) (B) and formic acid (0.5 ml), ammonia (0.125 ml) and water (1000 ml) (A), the gradients are specified in the following table for each method used.

| Method | Run Time | 0% B | 0 to 95% B | 95% B |
|---|---|---|---|---|
| A | 5 min | 0.2 min | 3 min | 0.8 min |
| B | 9 min | 0.5 min | 6.5 min | 1 min |
| C | 15 min | 0 min | 10.5 min | 1.5 min |

The flow rate was 0.8 ml/min for method A and 0.4 ml/min for method B, C and D. The injection volume was 5 microliter. A Waters 2996 diode array was used as a UV detector. Chromatograms were processed at 210 nM or 254 nM. Mass spectra of the chromatograms were acquired using positive and negative electrospray ionization in a Micromass ZMD or in a Waters ZQ detectors coupled to the HPLC.

¹H Nuclear Magnetic Resonance Spectra were recorded on a Varian Gemini-2000 spectrometer operating at a frequency of 300 MHz for the ¹H spectra or in a Varian Mercury plus operating at a frequency of 400 MHz for the ¹H spectra. Samples were dissolved in the specified deuterated solvent. Tetramethylsilane was used as reference.

Mass Spectra (m/z) were recorded on a Micromass ZMD or in a Waters ZQ mass spectrometer using ESI ionization.

### Abbreviations:

- DMF: Dimethylformamide
- DMSO: Dimethylsulfoxide
- CDCl₃: Deuterated chloroform
- NMR: Nuclear magnetic resonance
- s: Singlet
- d: Doublet
- dd: Doublet doublet
- td: Triple doublet
- br: Broad
- q: Quarted
- t: Triplet
- m: Multiplet
- LRMS: Low resolution mass spectrometry
- h: hour
- min: minutes

### PREPARATION 1

### 5-Bromo-2-chloro-1H-pyrrolo[2,3-b]pyridine

### a) 3,3,5-Tribromo-1,3-dihydro-2H-pyrrolo[2,3-b]pyridin-2-one

To a solution of 1*H*-pyrralo[2,3-b]pyridine (5.9 g, 48.94 mmol) in 380 ml *tert*-butanol and 380 ml water, bromine (31.7 ml, 612 mmol) was slowly added. The mixture was stirred overnight at room temperature and then *tert*-butanol was removed. The mixture was basified with saturated aqueous sodium hydrogencarbonate solution, the solid was filtered, washed with water and dried in the vacuum oven to give the title compound (14 g, 69% yield).
LRMS (m/z): 369, 371 (M+1)⁺.

### b) 5-Bromo-1,3-dihydro-2H-pyrrolo[2,3-b]pyridin-2-one

Zinc dust (24.7 g, 377.73 mmol) was added in portions to a stirred solution of 3,3,5-tribromo-1,3-dihydro-2*H*-pyrrolo[2,3-b]pyridin-2-one (Preparation 1a, 14 g, 37.75 mmol) in 288 ml acetic acid and the reaction was stirred overnight at room temperature. Solvent was removed and crude was basified with saturated aqueous sodium hydrogencarbonate solution and it was extracted with chloroform/methanol 9:1, organic layer was dried (Na₂SO₄) and concentrated to give the title compound as a solid (6.5 g, 81% yield).
LRMS (m/z): 213, 215 (M+1)⁺.

### c) 5-Bromo-2-chloro-1H-pyrrolo[2,3-b]pyridine

A suspension of 5-bromo-1,3-dihydro-2*H*-pyrrolo[2,3-b]pyridin-2-one (Preparation 1b, 6.5 g, 30.51 mmol) in phosphorous oxychloride (28.2 ml, 304.98 mmol) was stirred at 100°C for 5h. After that time 4% aqueous sodium hydrogencarbonate solution was added and it was extracted with chloroform-methanol 9:1, organic layer was dried (Na₂SO₄) and concentrated to give the title compound as a brown solid (6.92 g, 95% yield).
LRMS (m/z): 231, 233 (M+1)⁺.

### PREPARATION 2

### (6-Methoxy-4-methylpyridin-3-yl)boronic acid

Butyllithium solution (1.6M in hexane, 15.3 ml, 22.27 mmol) was added under argon to a solution of 5-bromo-2-methoxy-4-methylpyridine (4.5 g, 22.3 mmol) in 150 ml tetrahydrofuran at -78°C. The mixture was stirred at -78°C for 1h and after that triisopropyl borate (6.2 ml, 26.7 mmol) was added. The reaction was warmed to room temperature slowly and stirred overnight. The mixture was poured into a saturated aqueous ammonium chloride solution / water (1/1) and it was extracted with ethyl acetate, organic layers were put together, washed with brine, dried and concentrated. After washing the crude residue with diethyl ether the title compound was obtained as a white solid (2.12 g, 56.6% yield).
LRMS (m/z): 168 (M+1)⁺.

### PREPARATION 3

### 5-(6-Methoxy-4-methylpyridin-3-yl)-2-(4,4,5,5-tetramethyl-1,3,2-dioxaborolan-2-yl)-1H-pyrrolo[2,3-b]pyridine

### a) 2-Chloro-5-(6-methoxy-4-methylpyridin-3-yl)-1H-pyrrolo[2,3-b]pyridine

An oven dried resealable Schlenk tube was charged with 5-bromo-2-chloro-1*H-*pyrrolo[2,3-*b*]pyridine (Preparation 1c, 0.5 g, 2.07 mmol), (6-methoxy-4-methylpyridin-3-yl)boronic acid (Preparation 2, 0.35 g, 2.08 mmol), cesium carbonate (2.027 g, 6.22 mmol), 5.5 ml dioxane and 5.5 ml water. The Schlenk tube was subjected to three cycles of evacuation-backfilling with argon, and 1,1'-bis(diphenylphosphino)ferrocene-palladium(II) dichloride dichloromethane complex (0.085 g, 0.10 mmol) was added. After three further cycles of evacuation-backfilling with argon, the Schlenk tube was capped and placed in an oil bath at 100 °C. After 6h, the mixture was cooled and the solvent was removed in vacuo. Water was added and product was extracted with dichloromethane and organic layer was dried (Na₂SO₄). The residue was purified by flash chromatography (10%, dichloromethane-methanol) to give the title compound (0.353 g, 62% yield) as a solid.
LRMS (m/z):274 (M+1)⁺.

### b) 5-(6-Methoxy-4-methylpyridin-3-yl)-2-(4,4,5,5-tetramethyl-1,3,2-dioxaborolan-2-yl)-1H-pyrrolo[2,3-b]pyridine

A microwave tube was charged with 2-chloro-5-(6-methoxy-4-methylpyridin-3-yl)-1*H-*pyrrolo[2,3-*b*]pyridine (Preparation 3a, 0.175 mg, 0.63 mmol), 4,4,4',4',5,5,5',5'-octamethyl-2,2'-bi(1,3,2-dioxaborolane) (0.246 g, 0.95 mmol), tricyclohexylphosphine (0.032 g, 0.11 mmol), potassium acetate (0.145 g, 1.46 mmol), tris(dibenzylideneacetone)dipalladium(0) (0.030 g, 0.03 mmol) and 1.7 ml 1,2-dimethoxyethane. Vial was capped and it was subjected to three cycles of evacuation-backfilling with argon and reaction was heated at 120ºC. After 1h, saturated aqueous ammonium chloride solution was added and product was extracted with ethyl acetate. Organic layer was dried (Na₂SO₄) and evaporated to give the desired product 66% pure (0.5 g, 100% yield).
LRMS (m/z): 366 (M+1)⁺. Boronic acid

### PREPARATION 4

### 7-Bromo-2-(6-methoxy-4-methylpyridin-3-yl)-5H-pyrrolo[2,3-b]pyrazin-6-amine

### a) 5-(6-Methoxy-4-methylpyridin-3-yl)pyrazin-2-amine

Obtained as a white solid (77% yield) from (6-methoxy-4-methylpyridin-3-yl)boronic acid (preparation 2) and 5-bromopyrazin-2-amine following the experimental procedure as described in preparation 3a, followed by purification by flash chromatography (10% to 100%, heptane - ethyl acetate).
LRMS (m/z): 217 (M+1)⁺.

### b) 3-Bromo-5-(6-methoxy-4-methylpyridin-3-yl)pyrazin-2-amine

*N*-bromosuccinimide (0.091 mg, 0.509 mmol) was added to a solution of 5-(6-methoxy-4-methylpyridin-3-yl)pyrazin-2-amine (Preparation 4a, 0.100 g, 0.462 mmol) in 2 ml DMSO and 0.1 ml water. The reaction was stirred at room temperature for 3h. Reaction mixture was partitioned between water and ethyl acetate, organic layer was washed with brine, dried (Na₂SO₄), filtered and solvent evaporated in vacuo. Purification by flash chromatography (10% to 70% heptane - ethyl acetate) afforded the desired product as a white solid (0.070 mg, 51 % yield).
LRMS (m/z): 295, 297 (M+1)⁺.

### PREPARATION 5

### 1-Chloro-2-ethynyl-3-fluorobenzene

### a) [(2-Chloro-6-fluorophenyl)ethynyl](trimethyl)silane

In a microwave vial 1-chloro-3-fluoro-2-iodobenzene (1 g, 3.90 mmol) was dissolved in 9 ml *N*,*N-*dimethylformamide (dry) and triethylamine (4.5 ml, 32.4 mmol). Argon was bubbled through the solution for 10 minutes. Next the copper (I) iodide (0.030 mg, 0.156 mmol) and bis(triphenylphosphine)palladium (II) chloride (0.055 g, 0.078 mmol) were added, followed by trimethylsilylacetylene (1.665 ml, 11.70 mmol). The reaction was stirred at 80°C for 30 minutes and it was partitioned between brine and heptane, organic layer was washed with brine, dried (Na₂SO₄), filtered and solvent evaporated in vacuo to afford the desired product (0.7 g, 79% yield) as a yellow oil.
LRMS (m/z): 228 (M+1)⁺.

### b) 1-Chloro-2-ethynyl-3-fluorobenzene

[(2-chloro-6-fluorophenyl)ethynyl](trimethyl)silane (Preparation 5a, 0.700 mg, 3.09 mmol) was dissolved in 20 ml methanol, potassium carbonate (1 g, 7.24 mmol) was added and the mixture was stirred at room temperature for 20 min. Reaction mixture was partitioned between water and hexane, organic layer was washed with brine, dried (Na₂SO₄), filtered and solvent evaporated in vacuo to afford the desired product (0.35 g, 2.264 mmol, 73% yield).
LRMS (m/z): 154 (M+1)⁺.

### PREPARATION 6

### 3-Bromo-5-[1-methyl-3-(trifluoromethyl)-1H-pyrazol-5-yl]pyrazin-2-amine

### a) 5-[1-Methyl-3-(trifluoromethyl)-1H-pyrazol-5-yl]pyrazin-2-amine

Obtained as a brown solid (67% yield) from [1-methyl-3-(trifluoromethyl)-1*H*-pyrazol-5-yl]boronic acid and 5-bromopyrazin-2-amine following the experimental procedure as described in Preparation 3a.
LRMS (m/z): 244 (M+1)⁺.

### b) 3-Bromo-5-[1-methyl-3-(trifluoromethyl)-1H-pyrazol-5-yl]pyrazin-2-amine

Obtained (30% yield) from 5-[1-methyl-3-(trifluoromethyl)-1*H*-pyrazol-5-yl]pyrazin-2-amine (Preparation 6a) following the experimental procedure as described in Preparation 4b, followed by reverse phase purification using SP1 Purification System.
LRMS (m/z): 322, 324 (M+1)⁺.

### PREPARATION 7

### 5-Bromo-6'-methoxy-4'-methyl-3,3'-bipyridin-6-amine

### a) 6'-Methoxy-4'-methyl-3,3'-bipyridin-6-amine

Obtained as a green oil (74% yield) from 5-bromo-2-methoxy-4-methylpyridine and 5-(4,4,5,5-tetramethyl-1,3,2-dioxaborolan-2-yl)pyridin-2-amine following the experimental procedure as described in Preparation 3a.
LRMS (m/z): 216 (M+1)⁺.

### b) 5-Bromo-6'-methoxy-4'-methyl-3,3'-bipyridin-6-amine

6'-methoxy-4'-methyl-3,3'-bipyridin-6-amine (0.879 g, 4.08 mmol) was dissolved in 10 ml acetic acid, bromine (0.231 ml, 4.49 mmol) was added slowly and the resulting suspension was heated to 70°C for 2h. Reaction was diluted with 2N sodium hydroxide aqueous solution and extracted with dichloromethane. Combined organic layers were dried and concentrated in vacuo to give the desired product (0.091 g, 46% yield).
LRMS (m/z): 294, 296 (M+1)⁺.

### PREPARATION 8

### [1-Ethyl-3-(trifluoromethyl)-1H-pyrazol-5-yl]boronic acid

### a) 1-Ethyl-3-(trifluoromethyl)-1H-pyrazole

3-(Trifluoromethyl)-1*H*-pyrazole (1g, 7.35 mmol), iodoethane (0.712 g, 8.82 mmol) and potassium carbonate (1.523 g, 11.02 mmol) were dissolved in 20 ml *N,N-*dimethylformamide. The mixture was heated to 100°C for 4h. Water was added and product was extracted with ethyl acetate, organic layer was washed with brine and it was dried (Na₂SO₄), filtered and concentrated. Purification of the crude residue by flash chromatography afforded the desired product (0.2 g, 17% yield) as an oil.
LRMS (m/z): 165 (M+1)⁺.

### b) [1-Ethyl-3-(trifluoromethyl)-1H-pyrazol-5-yl]boronic acid

Obtained (71% yield) as a yellow oil from 1-ethyl-3-(trifluoromethyl)-1*H*-pyrazole (Preparation 8a) following the experimental procedure as described in Preparation 2.
LRMS (m/z): 209 (M+1)⁺.

### PREPARATION 9

### 3-Bromo-5-[1-ethyl-3-(trifluoromethyl)-1H-pyrazol-5-yl]pyrazin-2-amine

### a) 5-[1-Ethyl-3-(trifluoromethyl)-1H-pyrazol-5-yl]pyrazin-2-amine

Obtained (26% yield) from [1-ethyl-3-(trifluoromethyl)-1*H*-pyrazol-5-yl]boronic acid (Preparation 8b) and 5-bromopyrazin-2-amine following the experimental procedure as described in Preparation 3a, using sodium carbonate as a base.
LRMS (m/z): 258 (M+1)⁺.

### b) 3-Bromo-5-[1-ethyl-3-(trifluoromethyl)-1H-pyrazol-5-yl]pyrazin-2-amine

Obtained (75% yield) from 5-[1-ethyl-3-(trifluoromethyl)-1*H*-pyrazol-5-yl]pyrazin-2-amine (Preparation 18a) following the experimental procedure as described in Preparation 4b.
LRMS (m/z): 336,338 (M+1)⁺.

### PREPARATION 10

### 4-(5-Amino-6-bromopyrazin-2-yl)-N,N,3-trimethylbenzenesulfonamide

### a) 4-(5-Aminopyrazin-2-yl)-N,N,3-trimethylbenzenesulfonamide

4-Bromo-*N*,*N*,3-trimethylbenzenesulfonamide (0.5 g, 1.797 mmol), potassium acetate (0.176 g, 1.797 mmol) and 4,4,4',4',5,5,5',5'-octamethyl-2,2'-bi(1,3,2-dioxaborolane) (0.456 g, 1.797 mmol) were dissolved in 10 ml 1,4-dioxane and degassed with nitrogen for 5 min. 1,1'-Bis(diphenylphosphino)ferrocene-palladium(II) dichloride (0.066 g, 0.09 mmol) was added and the mixture was stirred under microwave irradiation for 1 h. Water (2 ml), cesium carbonate (0.703 g, 2.157 mmol), 5-bromopyrazin-2-amine (0.32 g, 1.797 mmol) and an additional amount of 1,1'-bis(diphenylphosphino)ferrocene-palladium(II) dichloride (0.066 g, 0.09 mmol) were added and the mixture was heated under microwave irradiation at 100°C for 5h. Reaction mixture was partitioned between water and ethyl acetate, organic layer was separated, washed with brine, dried (Na₂SO₄), filtered and solvent evaporated in vacuo. Purification by flash chromatography (10% to 100% heptane - ethyl acetate) afforded the desired product (0.120g,%) as a white solid.
LRMS (m/z): 293 (M+1)⁺.

### b) 4-(5-Amino-6-bromopyrazin-2-yl)-N,N,3-trimethylbenzenesulfonamide

Obtained (100% yield) as an oil from 4-(5-aminopyrazin-2-yl)-*N*,*N*,3-trimethylbenzene sulfonamide following the experimental procedure as described in Preparation 4b.
LRMS (m/z): 371,373 (M+1)⁺.

### PREPARATION 11

### 3-(3,5-Difluoro-4-iodophenoxy)propan-1-ol

3,5-Difluoro-4-iodophenol (0.5 g, 1.95 mmol), 3-bromopropan-1-ol (0.247 ml, 2.73 mmol) and potassium carbonate (1.079 g, 7.81 mmol) were dissolved in 10ml *N,N-*dimethylformamide under nitrogen conditions and the mixture was heated to 70ºC overnight. The crude was partitioned between water and dichloromethane, organic layer was washed with brine, dried and concentrated. Purification by flash chromatography (0% to 10%, dichloromethane - methanol) afforded the desired product (0.344 g, 56% yield).
LRMS (m/z): Non ionizable
¹H NMR (400 MHz, CHLOROFORM-*d*) d ppm 2.00 - 2.08 (m, 2 H), 3.85 (q, *J*=5.86 Hz, 2 H), 4.10 (t, J=6.06 Hz, 2 H), 6.50 - 6.55 (m, 2 H).

### PREPARATION 12

### 5-[1-Methyl-3-(trifluoromethyl)-1H-pyrazol-5-yl]-2-(4,4,5,5-tetramethyl-1,3,2-dioxaborolan-2-yl)-1H-pyrrolo[2,3-b]pyridine

### a) 2-Chloro-5-[1-methyl-3-(trifluoromethyl)-1H-pyrazol-5-yl]-1H-pyrrolo[2,3-b] pyridine

Obtained (18% yield) as a yellow solid from 5-bromo-2-chloro-1*H*-pyrrolo[2,3-*b*]pyridine (Preparation 1c) and (1-methyl-3-(trifluoromethyl)-1*H*-pyrazol-5-yl)boronic acid following the experimental procedure as described in Preparation 3a.
LRMS (m/z): 301 (M+1)⁺.

### b) 5-[1-Methyl-3-(trifluoromethyl)-1H-pyrazol-5-yl]-2-(4,4,5,5-tetramethyl-1,3,2-dioxaborolan-2-yl)-1H-pyrrolo[2,3-b]pyridine

Obtained (100% yiled) from 2-chloro-5-[1-methyl-3-(trifluoromethyl)-1*H*-pyrazol-5-yl]-1*H*-pyrrolo[2,3-*b*]pyridine (Preparation 12a) following the experimental procedure as described in Preparation 3b.
LRMS (m/z): 393 (M+1)⁺.

### PREPARATION 13

### 3-Bromo-5-(6-chloro-2,2-difluoro-1,3-benzodioxol-5-yl)pyridin-2-amine

### a) 5-(6-Chloro-2,2-difluoro-1,3-benzodioxol-5-yl)pyridin-2-amine

Obtained (57% yield) from 5-bromo-6-chloro-2,2-difluorobenzo[*d*][1,3]dioxole and 5-(4,4,5,5-tetramethyl-1,3,2-dioxaborolan-2-yl)pyridin-2-amine following the experimental procedure as described in Preparation 3a, followed by reverse phase using SP1 Purification System.
LRMS (m/z): 285 (M+1)⁺.

### b) 3-Bromo-5-(6-chloro-2,2-difluoro-1,3-benzodioxol-5-yl)pyridin-2-amine

Obtained (100% yield) as a white solid from 5-(6-chloro-2,2-difluoro-1,3-benzodioxol-5-yl)pyridin-2-amine (Preparation 13a) following the experimental procedure as described in Preparation 7b.
LRMS (m/z): 363, 365 (M+1)⁺.

### PREPARATION 14

### 5-Bromo-2-(2-chloro-6-fluorophenyl)-1H-pyrrolo[3,2-b]pyridine

### a) 2,6-Dibromopyridin-3-amine

Obtained (89% yield) from pyridin-3-amine following the experimental procedure as described in Preparation 4b.
LRMS (m/z): 251, 253 (M+1)⁺.

### b) 6-Bromo-2-[(2-chloro-6-fluorophenyl)ethynyl]pyridin-3-amine

Obtained (29% yield) from 2,6-dibromopyridin-3-amine (Preparation 14a) and 1-chloro-2-ethynyl-3-fluorobenzene (Preparation 5) following the experimental procedure as described in Preparation 5a, using thermal conditions instead of microwave irradiation and followed by flash chromatography purification (0-100%, hexane - ethyl acetate).
LRMS (m/z): 325, 327 (M+1)⁺.

### c) 5-Bromo-2-(2-chloro-6-fluorophenyl)-1H-pyrrolo[3,2-b]pyridine

A solution of 6-bromo-2-[(2-chloro-6-fluorophenyl)ethynyl]pyridin-3-amine (Preparation 14b, 0.738 g, 2.27 mmol) in 3 ml *N*-methyl-2-pyrrolidone was added to potassium *tert-*butoxide (0.568 g, 4.81 mmol) in 3 ml *N*-methyl-2-pyrrolidone and the mixture was stirred at room temperature overnight. Reaction mixture was poured into water and it was extracted with ethyl acetate, organic layer was washed with brine, dried (Na₂SO₄), filtered and concentrated. Purification of the crude residue by flash chromatography (0% to 100%, hexane - ethyl acetate) afforded the title compound (0.387 g, 52%) as a yellow solid.
LRMS (m/z): 325, 327 (M+1)⁺.

### PREPARATION 15

### 5-Bromo-2-cyclohexyl-1H-pyrrolo[3,2-b]pyridine

### a) 6-Bromo-2-(cyclohexylethynyl)pyridin-3-amine

Obtained (34% yield) as a solid from 2,6-dibromopyridin-3-amine (Preparation 14a) and ethynylcyclohexane following the experimental procedure as described in Preparation 5a, using thermal conditions instead of microwave irradiation and followed by flash chromatography purification (0% to 100%, hexane - ethyl acetate) and then reverse phase purification using SP1 Purification System.
LRMS (m/z): 279, 280 (M+1)⁺.

### b) 5-Bromo-2-cyclohexyl-1H-pyrrolo[3,2-b]pyridine

Obtained (73% yield) as a solid from 6-bromo-2-(cyclohexylethynyl)pyridin-3-amine (Preparation 15a) following the experimental procedure as described in Preparation 14c, obtaining the desired product by precipitation in water.
LRMS (m/z): 279, 281 (M+1)⁺.

### PREPARATION 16

### 2-Ethynyl-1,3-difluorobenzene

### a) [(2,6-Difluorophenyl)ethynyl](trimethyl)silane

Obtained (100% yield) from 1,3-difluoro-2-iodobenzene and ethynyltrimethylsilane following the experimental procedure as described in Preparation 5a.
LRMS (m/z): Non ionizable
¹H NMR (300 MHz, CHLOROFORM-*d*) d ppm 0.02 (s, 9 H), 6.55 - 6.65 (m, *J*=8.22, 7.04 Hz, 2 H), 6.91 - 7.03 (m, 1 H).

### b) 2-Ethynyl-1,3-difluorobenzene

Obtained (35% yield) as an oil from [(2,6-difluorophenyl)ethynyl](trimethyl)silane (Preparation 16a) following the experimental procedure as described in Preparation 5b.
LRMS (m/z): Non ionizable
¹H NMR (300 MHz, CHLOROFORM-*d*) d ppm 3.52 (s, 1 H), 6.88 - 6.98 (m, *J*=8.51, 7.34 Hz, 2 H), 7.28 - 7.37 (m, 1 H).

### PREPARATION 17

### 5-Bromo-2-(2,6-difluorophenyl)-1H-pyrrolo[3,2-b]pyridine

### a) 6-Bromo-2-[(2,6-difluorophenyl)ethynyl]pyridin-3-amine

Obtained (34% yield) as a solid from 2,6-dibromopyridin-3-amine (Preparation 14a) and 2-ethynyl-1,3-difluorobenzene (Preparation 16b) following the experimental procedure as described in Preparation 5a, using thermal conditions instead of microwave irradiation and followed by flash chromatography using SP1 Purification System (0% to 100% hexane - diethyl ether).
LRMS (m/z): 309, 311 (M+1)⁺.

### b) 5-Bromo-2-(2,6-difluorophenyl)-1H-pyrrolo[3,2-b]pyridine Obtained (39% yield) from 6-bromo-2-[(2,6-difluorophenyl)ethynyl]pyridin-3-amine

(Preparation 17a) following the experimental procedure as described in Preparation 14c.
LRMS (m/z): 309, 311 (M+1)⁺.

### PREPARATION 18

### 5-Bromo-2-propyl-1H-pyrrolo[3,2-b]pyridine

### a) 6-Bromo-2-pent-1-yn-1-ylpyridin-3-amine

Obtained (51% yield) as an oil from 2,6-dibromopyridin-3-amine (Preparation 14a) and pent-1-yne following the experimental procedure as described in Preparation 5a, using thermal conditions instead of microwave irradiation and followed by flash chromatography purification (0-100%, hexane - diethyl ether) and then reverse phase chromatography using SP1 Purification System.
LRMS (m/z): 239, 241 (M+1)⁺.

### b) 5-Bromo-2-propyl-1H-pyrrolo[3,2-b]pyridine

Obtained (76% yield) as a white solid from 6-bromo-2-pent-1-yn-1-ylpyridin-3-amine (Preparation 18a) following the experimental procedure as described in Preparation 14c, followed by purification by flash chromatography (0% to 100% hexane - ethyl acetate).
LRMS (m/z): 239, 241 (M+1)⁺.

### PREPARATION 19

### 2-Bromo-6-[1-methyl-3-(trifluoromethyl)-1H-pyrazol-5-yl]pyridin-3-amine

### a) 6-[1-Methyl-3-(trifluoromethyl)-1H-pyrazol-5-yl]pyridin-3-amine

Obtained (30% yield) as a white solid from 6-bromopyridin-3-amine and 1-methyl-3-(trifluoromethyl)-1*H*-pyrazol-5-ylboronic acid following the experimental procedure as described in Preparation 3a, using sodium carbonate as a base and followed by reverse phase using SP1 Purification System.
LRMS (m/z): 243 (M+1)⁺..

### b) 2-Bromo-6-[1-methyl-3-(trifluoromethyl)-1H-pyrazol-5-yl]pyridin-3-amine

Obtained (82% yield) as a solid from 6-[1-methyl-3-(trifluoromethyl)-1*H*-pyrazol-5-yl]pyridin-3-amine (Preparation 19a) following the experimental procedure as described in Preparation 4b, using acetronitrile as solvent.
LRMS (m/z): 321, 323 (M+1)⁺.

### PREPARATION 20

### 2-Bromo-6-[5-methyl-3-(trifluoromethyl)-1H-pyrazol-1-yl]pyridin-3-amine

### a) 6-[5-Methyl-3-(trifluoromethyl)-1H-pyrazol-1-yl]pyridin-3-amine

In a Schlenk vessel 5-methyl-3-(trifluoromethyl)-1*H*-pyrazole (174 mg, 1.16 mmol), 6-bromopyridin-3-amine (200 mg, 1.16 mmol), cooper (I) iodide (33 mg, 0.17 mmol) and potassium phosphate (488 mg, 2.3 mmol) were dissolved in 4 ml dioxane. The reaction was submitted to three vacuum-argon cycles and was then heated at 130°C overnight. The mixture was filtered through a plug of Celite and evaporated under reduced pressure. The crude was purified by flash chromatography (0% to 100%, hexane - diethyl ether) using SP1 Purification System to obtain 226 mg (79% yield) of the title compound.
LRMS (m/z): 243 (M+1)⁺.

### b) 2-Bromo-6-[5-methyl-3-(trifluoromethyl)-1H-pyrazol-1-yl]pyridin-3-amine

Obtained (75% yield) as a solid from 6-[5-methyl-3-(trifluoromethyl)-1*H*-pyrazol-1-yl]pyridin-3-amine (Preparation 20a) following the experimental procedure as described in Preparation 4b, using acetronitrile as a solvent.
LRMS (m/z): 321, 323 (M+1)⁺.

### PREPARATION 21

### 5-Chloro-1-(phenylsulfonyl)-1H-pyrrolo[3,2-b]pyridine

### a) 6-Chloro-2-iodopyridin-3-amine

N-lodosuccinimide (5.25 g, 20 mmol) was added to a solution of 6-chloropyridin-3-amine (2.5 g, 20 mmol) in 40 ml *N*,*N*-dimethylformamide and the mixture was stirred overnight at room temperature. The reaction mixture was partitioned between water and ethyl acetate and organic layer was washed with brine, dried (MgSO₄), filtered and concentrated. The crude residue was suspended in dichloromethane; solid was filtered, re-dissolved in ethyl acetate and washed with aqueous saturated solution of sodium thiosulfate. Organic layer was dried, filtered and concentrated to afford the desired product as a brown solid (1.93 g, 39% yield).
LRMS (m/z): 255 (M+1)⁺.

### b) 6-Chloro-2-((trimethylsilyl)ethynyl)pyridin-3-amine

Obtained (91% yield) as a yellow solid from 6-chloro-2-iodopyridin-3-amine (Preparation 21 a) and ethynyltrimethylsilane following the experimental procedure as described in Preparation 5a, using thermal conditions (60°C) instead of microwave irradiation and followed by flash chromatography purification (0% to 100%, hexane - diethyl ether).
LRMS (m/z): 225 (M+1)⁺.

### c) 5-Chloro-1H-pyrrolo[3,2-b]pyridine

Obtained (79% yield) as a brown solid from 6-chloro-2-((trimethylsilyl)ethynyl)pyridin-3-amine (Preparation 21 b) following the experimental procedure as described in Preparation 14c.
LRMS (m/z): 153 (M+1)⁺.

### d) 5-Chloro-1-(phenylsulfonyl)-1H-pyrrolo[3,2-b]pyridine

5-Chloro-1*H*-pyrrolo[3,2-*b*]pyridine (Preparation 21 c, 2 g, 13.11 mmol) was added to a suspension of sodium hydride (60%, 0.70 g, 17.5 mmol) in 10 ml *N,N-*dimethylformamide at 0°C and mixture was stirred for 30 min. Then benzenesulfonyl chloride (2 ml, 15.51 mmol) was added and reaction was stirred at room temperature for 1 h. The mixture was partitioned between ethyl acetate and water, organic layer was dried (MgSO₄), filtered and concentrated. Purification by normal phase chromatography (0% to 100%, hexane - diethyl ether) afforded the title compound as a white solid (2.85 g, 74% yield).
LRMS (m/z): 293 (M+1)⁺.

### PREPARATION 22

### 2-Iodo-5-[1-methyl-3-(trifluoromethyl)-1H-pyrazol-5-yl]-1-(phenylsulfonyl)-1H-pyrrolo[3,2-b]pyridine

### a) 5-[1-Methyl-3-(trifluoromethyl)-1H-pyrazol-5-yl]-1-(phenylsulfonyl)-1H-pyrrolo[3,2-b]pyridine

Obtained (49% yield) as a solid from 5-chloro-1-(phenylsulfonyl)-1*H*-pyrrolo[3,2-b]pyridine (Preparation 21d) and 1-methyl-3-(trifluoromethyl)-1*H*-pyrazol-5-ylboronic acid following the experimental procedure as described in Preparation 3a, with sodium carbonate as a base.
LRMS (m/z): 407 (M+1)⁺.

### b) 2-Iodo-5-[1-methyl-3-(trifluoromethyl)-1H-pyrazol-5-yl]-1-(phenylsulfonyl)-1H-pyrrolo[3,2-b]pyridine

To a solution of 2,2,6,6-tetramethylpiperidine (0.094 ml, 0.56 mmol) was dissolved in 4 ml tetrahydrofuran. At -78°C butyllithium (1.6 M in hexane, 0.322 ml, 0.52 mmol) was added under argon and the reaction was stirred at -78°C for 30 min. 5-[1-methyl-3-(trifluoromethyl)-1*H*-pyrazol-5-yl]-1-(phenylsulfonyl)-1*H*-pyrrolo[3,2-*b*]pyridine (Preparation 22a, 0.175 g, 0.43 mmol) was then added in 5 ml tetrahydrofuran. The mixture was stirred at -78ºC for 1h and iodine (0.218 g, 0.86 mmol) in tetrahydrofuran (2 ml) was added dropwise. After stirring at -78ºC for 3h, the reaction was warmed to room temperature slowly and stirred at this temperature overnight. Aqueous saturated solution of ammonium chloride was added and product was extracted with ethyl acetate, organic layer was dried (MgSO₄), filtered and concentrated. Purification of the crude residue by normal phase chromatography (0% to 100%, hexane - diethyl ether) afforded the desired product as a solid (0.179 g, 74% yield).
LRMS (m/z): 533 (M+1)⁺.

### PREPARATION 23

### 2-Bromo-6-[5-(difluoromethoxy)-3-(trifluoromethyl)-1H-pyrazol-1-yl]pyridin-3-amine

### a) 1-(5-Nitropyridin-2-yl)-3-(trifluoromethyl)-1H-pyrazol-5-ol

To a solution of 2-hydrazinyl-5-nitropyridine (1 g, 6.49 mmol) and ethyl 4,4,4-trifluoro-3-oxobutanoate (1.19 g, 6.46 mmol) in 12 ml ethanol, 4M hydrochloric acid in dioxane (6 ml, 24 mmol) was added. The mixture was heated to reflux overnight. Diethyl ether and water were added and organic layer was washed with water and brine, dried, filtered and concentrated. Purification of the crude residue by flash chromatography (0%-100%, hexane - ethyl acetate) afforded the desired product as a solid (0.966 g, 43% yield).
LRMS (m/z): 275 (M+1)⁺.

### b) 2-[5-(Difluoromethoxy)-3-(trifluoromethyl)-1H-pyrazol-1-yl]-5-nitropyridine

Sodium 2-chloro-2,2-difluoroacetate (1.06 g, 6.95 mmol) and potassium carbonate (0.962 g, 6.95 mmol) were added to a solution of 1-(5-nitropyridin-2-yl)-3-(trifluoromethyl)-1*H*-pyrazol-5-ol (Preparation 23a, 0.960 g, 2.80 mmol) in 10 ml *N*,*N-*dimethylformamide and 2 ml water. The mixture was stirred at 100°C for 5h and then it was partitioned between ethyl acetate and water, organic layer was washed with water and brine, dried, filtered and concentrated. Purification of the crude residue by flash chromatography (0 to 100%, hexane - diethyl ether) afforded the title compound as a yellow oil (0.510 g, 54% yield).
LRMS (m/z): 325 (M+1)⁺.

### c) 6-[5-(Difluoromethoxy)-3-(trifluoromethyl)-1H-pyrazol-1-yl]pyridin-3-amine

2-[5-(difluoromethoxy)-3-(trifluoromethyl)-1*H*-pyrazol-1-yl]-5-nitropyridine (Preparation 23b, 0.510 g, 1.57 mmol), iron (0.701 g, 12.55 mmol) and ammonium chloride (0.072 g, 1.35 mmol) were dissolved in 7 ml ethanol and 2.5 ml water. The mixture was stirred at 100°C for 5h and then the suspension was filtered through celite and solvent was removed in vacuo. The crude was partitioned between ethyl acetate and water, organic layer was washed with brine, dried, filtered and concentrated. Purification of the crude residue by flash chromatography (0% to 100%, hexane - diethyl ether) afforded the title compound (0.300 g, 65% yield).
LRMS (m/z): 295 (M+1)⁺.

### d) 2-Bromo-6-[5-(difluoromethoxy)-3-(trifluoromethyl)-1H-pyrazol-1-yl]pyridin-3-amine

Obtained (84% yield) as an orange solid from 6-[5-(difluoromethoxy)-3-(trifluoromethyl)-1*H*-pyrazol-1-yl]pyridin-3-amine (Preparation 23c) following the experimental procedure as described in Preparation 4b, using acetronitrile as a solvent and followed by flash chromatography (0% to 100%, hexane - ethyl acetate).
LRMS (m/z): 373, 375 (M+1)⁺.

### PREPARATION 24

### [5-[1-Methyl-3-(trifluoromethyl)-1H-pyrazol-5-yl]-1-(phenylsulfonyl)-1H-pyrrolo[3,2-b]pyridin-2-yl]boronic acid

Obtained (95% yield) as a yellow solid from 5-[1-methyl-3-(trifluoromethyl)-1*H*-pyrazol-5-yl]-1-(phenylsulfonyl)-1*H*-pyrrolo[3,2-*b*]pyridine (Preparation 22b) following the experimental procedure as described in Preparation 2.
LRMS (m/z): 451 (M+1)⁺.

### PREPARATION 25

### (2-Fluoro-6-methylphenyl)boronic acid

2-Bromo-1-fluoro-3-methylbenzene (300 mg, 1.59 mmol) was dissolved in diethyl ether (3 ml) and triisopropyl borate (6.2 ml, 26.7 mmol) was added. The mixture was cooled at -78°C under argon atmosphere. *tert*-Butyllithium solution (1.7M in pentane, 2.2 ml, 3.74 mmol) was added drop-wise. Then temperature was increased at -10°C and the reaction was stirred for 30 min. Hydrochloric acid (5N, 2ml) was added and the reaction was stirred at room temperature for 30 min more. The mixture was extracted twice with diisopropyl ether, organic layers were basified with sodium hydroxide 2N and the aqueous phase was acidified with hydrochloric acid to pH 1. The aqueous was extracted twice with diethyl ether, washed with brine, dried over sodium sulphate and evaporated under reduced pressure to obtain a title compound as a white solid (117 mg, 43% yield).
LRMS (m/z): 155 (M+1)⁺.

### PREPARATION 26

### (2-Chloro-6-methylphenyl)boronic acid

Obtained (31% yield) as an oil from 2-bromo-1-chloro-3-methylbenzene following the experimental procedure as described in Preparation 25.
LRMS (m/z): 171 (M+1)⁺.

### PREPARATION 27

### 2-Iodo-5-(1-methyl-3-(trifluoromethyl)-1H-pyrazol-5-yl)-1H-pyrrolo[3,2-b]pyridine

To a solution of 5-(1-methyl-3-(trifluoromethyl)-1*H*-pyrazol-5-yl)-1-(phenylsulfonyl)-1*H-*pyrrolo[3,2-b]pyridine (Preparation 22a, 1.86 g, 4.50 mmol) was dissolved in 30 ml tetrahydrofuran. At -78°C butyllithium solution (1.6 M in hexane, 6.5 ml, 10.40 mmol) was added under argon conditions and the reaction was stirred at -78°C for 1.25 h. Iodine (2.5 g, 9.85 mmol) in 5 ml tetrahydrofuran was added dropwise. After stirring at -78°C for 3h, the reaction was warmed to room temperature slowly and stirred at this temperature overnight. Aqueous saturated solution of ammonium chloride was added and product was extracted with ethyl acetate, organic layer was washed twice with a saturated solution of thiosulphate. The organic layers were dried (MgSO₄), filtered and concentrated. The crude was re-dissolved in 66 ml ethanol and 14ml sodium hydroxide 8N was added. The reaction was stirred 1.5 h. The solvent was concentrated under reduced pressure and extracted with ethyl acetate. The organic was washed with water, dried over sodium sulphate and evaporated under reduced pressure. Purification of the crude residue by normal phase chromatography (0% to 100%, hexane - diethyl ether) afforded the desired product as a solid (0.967 g, 50% yield).
LRMS (m/z): 393 (M+1)⁺.

### PREPARATION 28

### 2-((2-Chloro-6-fluorophenyl)ethynyl)-6-(5-methyl-3-(trifluoromethyl)-1H-1,2,4-triazol-1-yl)pyridin-3-amine

2-Bromo-6-(5-methyl-3-(trifluoromethyl)-1*H*-1,2,4-triazol-1-yl)pyridin-3-amine (Preparation 60c) was treated with 1-chloro-2-ethynyl-3-fluorobenzene (Preparation 5b), copper (I) iodide, bis(triphenylphosphine)palladium (II) chloride, triethylamine and tetrahydrofuran as a solvent according to the method described in Preparation 5a using termal conditions. The crude was purified by flash chromatography (0% to 100%, hexane-dichloromethane) to obtain the final compound (35% yield).
LRMS (m/z): 396 (M+1)⁺.

### PREPARATION 29

### 2-Iodo-5-(6-methoxy-4-methylpyridin-3-yl)-1H-pyrrolo[2,3-b]pyridine

### a) 5-Bromo-1-(phenylsulfonyl)-1H-pyrrolo[2,3-b]pyridine

5-Bromo-1*H*-pyrrolo[2,3-*b*]pyridine was treated with sodium hydride, benzenesulfonyl chloride and *N*,*N*-dimethylformamide as a solvent according to the method described in Preparation 21d obtaining the title compound (84% yield).
LRMS (m/z): 337, 339 (M+1)⁺.

### b) 5-(6-Methoxy-4-methylpyridin-3-yl)-1-(phenylsulfonyl)-1H-pyrrolo[2,3-b]pyridine

5-Bromo-1-(phenylsulfonyl)-1*H*-pyrrolo[2,3-*b*]pyridine (Preparation 29a) was treated with (6-methoxy-4-methylpyridin-3-yl)boronic acid (Preparation 2), cesium carbonate, 1,1'-bis(diphenytphosphino)ferrocene-palladium(II) dichloride dichloromethane complex according to the method described in Preparation 3a to give the title compound (83% yield).
LRMS (m/z): 380 (M+1)⁺.

### c) 2-Iodo-5-(6-methoxy-4-methylpyridin-3-yl)-1H-pyrrolo[2,3-b]pyridine 5-(6-Methoxy-4-methylpyridin-3-yl)-1-(phenylsulfonyl)-1H-pyrrolo[2,3-b]pyridine

(Preparation 29b) was treated with 2,2,6,6-tetramethylpiperidine, butyllithium solution and iodine according to the method described in Preparation 22b to give the desired compound (68% yield).
LRMS (m/z): 506 (M+1)⁺.

### PREPARATION 30

### 5-(1-Methyl-3-(trifluoromethyl)-1H-pyrazol-5-yl)-2-(4,4,5,5-tetramethyl-1,3,2-dioxaborolan-2-yl)-1H-pyrrolo[3,2-b]pyridine

2-Iodo-5-(1-methyl-3-(trifluoromethyl)-1*H*-pyrazol-5-yl)-1*H*-pyrrolo[3,2-b]pyridine (Preparation 27, 100 mg, 0.26 mmol) and 4,4,4',4',5,5,5',5'-octamethyl-2,2'-bi(1,3,2-dioxaborolane) (97 mg, 0.38 mmol) were dissolved in 5 ml dimethylethane. Potassium carbonate (62 mg, 0.63 mmol) was added and the microwaves vessel was sealed. The mixture was submitted to three vaccum-argon cycles and then tris(dibenzylideneacetone)dipalladium(0) (11 mg, 0.01 mmol) and tricyclohexylphosphine (14 mg, 0.05 mmol) were added and submitted the reaction mixture to three vaccum cycles. The reaction was heated at 120ºC for 50 min under microwaves conditions.
The mixture was poured into water and a solution saturated ammonium chloride and extracted twice with ethyl acetate. The organics were combined, dried over sodium sulphate, filtered and evaporated under reduced pressure to obtain 150 mg as a brown oil. The product was used without further purification.
LRMS (m/z): 393 (M+1)⁺.

### PREPARATION 31

### 5-(1-Methyl-3-(trifluoromethyl)-1H-pyrazol-5-yl)-2-(1,4-dioxaspiro[4.5]decan-8-yl)-1H-pyrrolo[3,2-b]pyridine

2-Iodo-5-(1-methyl-3-(trifluoromethyl)-1*H*-pyrazol-5-yl)-1-(phenylsulfonyl)-1*H-*pyrrolo[3,2-b]pyridine (Preparation 22b, 150 mg, 0.28 mmol) was treated with 4,4,5,5-tetramethyl-2-(1,4-dioxaspiro[4.5]dec-7-en-8-yl)-1,3,2-dioxaborolane, cesium carbonate and 1,1'-bis(diphenylphosphino)ferrocene-palladium(II) dichloride dichloromethane complex according to the method described in Preparation 3a to obtain (55% yield) the title compound.
LRMS (m/z): 545 (M+1)⁺.

### PREPARATION 32

### 5-(1-Methyl-3-(trifluoromethyl)-1H-pyrazol-5-yl)-2-(1,4-dioxaspiro[4.5]dec-7-en-8-yl)-1H-pyrrolo[3,2-b]pyridine

5-(1-Methyl-3-(trifluoromethyl)-1*H*-pyrazol-5-yl)-2-(1,4-dioxaspiro[4.5]dec-7-en-8-yl)-1*H-*pyrrolo[3,2-b]pyridine (Example 31, 48 mg, 0.12 mmol) was dissolved in 3 ml ethyl acetate. The mixture was submitted to three vacuum-argon cycles and palladium on carbon (10%, 6 mg, 0.06 mmol) was added. The reaction mixture was hydrogenated for 2h.
The mixture was degassed, filtered through a plug of celite and concentrated under reduced pressure to obtain 55 mg (100%) of the title compound.
LRMS (m/z): 407 (M+1)⁺.

### PREPARATION 33

### 4-(5-(1-Methyl-3-(trifluoromethyl)-1H-pyrazol-5-yl)-1H-pyrrolo[3,2-b]pyridin-2-yl)cyclohexanone

5-(1-Methyl-3-(trifluoromethyl)-1*H*-pyrazol-5-yl)-2-(1,4-dioxaspiro[4.5]dec-7-en-8-yl)-1*H-*pyrrolo[3,2-*b*]pyridine (Preparation 32, 55 mg, 0.14 mmol) was dissolved in 1 ml acetone and 1 ml water. *p*-Toluenesulfonic acid monohydrate (25 mg, 0.13 mmol) was added and the reaction was heated at 80ºC for 5h.
The organic solvent was evaporated and the aqueous residue was basified with sodium bicarbonate, extracted with ethyl acetate, dried over sodium sulphate, filtered and evaporated under reduced pressure to give 35 mg (66% yield) of the title compound as a brown solid.
LRMS (m/z): 363 (M+1)⁺.

### PREPARATION 34

### 2-(3,5-Difluoro-4-iodophenoxy)ethanol

3,5-Difluoro-4-iodophenol was treated with 2-bromoethanol and potassium carbonate according to the method described in Preparation 11 to give the title compound (94% yield).
LRMS (m/z): Non ionizable
¹H NMR (300 MHz, CHLOROFORM-*d*) d ppm 3.95 - 4.00 (m, 2 H), 4.04 - 4.08 (m, 2 H), 6.50 - 6.58 (m, 2 H).

### PREPARATION 35

### 3-(Cyclohexylethynyl)-5-(1-methyl-3-(trifluoromethyl)-1H-pyrazol-5-yl)pyrazin-2-amine

3-Bromo-5-(1-methyl-3-(trifluoromethyl)-1H-pyrazol-5-yl)pyrazin-2-amine (Preparation 6b) was treated with triethylamine, copper (I) iodide, bis(triphenylphosphine)palladium (II) chloride (0.055 g, 0.078 mmol), ethynylcyclohexane and tetrahydrofuran as a solvent following the method described in Preparation 5a to give the title compound (61% yield).
LRMS (m/z): 350 (M+1)⁺.

### PREPARATION 36

### 2-(4-Methyl-3-(4,4,5,5-tetramethyl-1,3,2-dioxaborolan-2-yl)phenyl)oxazole

2-(3-lodo-4-methylphenyl)oxazole (1.5 g, 5.26 mmol) was treated with 4,4,4',4',5,5,5',5'-octamethyl-2,2'-bi(1,3,2-dioxaborolane), [1,1'-bis(diphenylphosphino)ferrocene]-dichloro-palladium(II), complex with dichloromethane and potassium acetate according to the method described in Preparation 3a. The reaction was heated at 100ºC for 48h followed by purification by flash chromatography (0% to 20%, hexane-ethyl acetate) to obtain (78% yield) the title compound.
LRMS (m/z): 286 (M+1)⁺.

### PREPARATION 37

**1-((3-Amino-6-(1-methyl-3-(trifluoromethyl)-1*H*-pyrazol-5-yl)pyridin-2-yl)ethynyl)cyclohexanol**

2-Bromo-6-(1-methyl-3-(trifluoromethyl)-1*H*-pyrazal-5-yl)pyridin-3-amine (Preparation 19b, 128 mg, 0.4 mmol) was treated with triethylamine , copper (I) iodide, bis(triphenylphosphine)palladium (II) chloride, 1-ethynylcyclohexanal and tetrahydrofuran as a solvent following the method described in Preparation 5a to give the title compound (76% yiled).
LRMS (m/z): 365 (M+1)⁺.

### PREPARATION 38

### 2-((2,6-Difluorophenyl)ethynyl)-6-(5-methyl-3-(trifluoromethyl)-1H-pyrazol-1-yl)pyridin-3-amine

2-Bromo-6-(5-methyl-3-(trifluoromethyl)-1H-pyrazol-1-yl)pyridin-3-amine (Preparation 20b) was treated with with triethylamine, copper (I) iodide, bis(triphenylphosphine)palladium (II) chloride, 2-ethynyl-1,3-difluorobenzene (Preparation 16b) and tetrahydrofuran as a solvent following the method described in Preparation 5a to give (47% yield) the title compound.
LRMS (m/z): 379 (M+1)⁺.

### PREPARATION 39

### tert-Butyl 4-((3-amino-6-(5-methyl-3-(trifluoromethyl)-1H-pyrazol-1-yl)pyridin-2-yl)ethynyl)piperidine-1-carboxylate

2-Bromo-6-(5-methyl-3-(trifluoromethyl)-1*H*-pyrazol-1-yl)pyridin-3-amine (Preparation 20b, 300 mg, 0.93 mmol) was treated with *tert*-butyl 4-ethynylpiperidine-1-carboxylate, triethylamine, copper (I) iodide, bis(triphenylphosphine)palladium (II) chloride and tetrahydrofuran as a solvent following the method described in Preparation 5a to give (60% yield) the title compound.
LRMS (m/z): 450 (M+1)⁺.

### PREPARATION 40

### 4-((3-Methyl-4-(2-(4,4,5,5-tetramethyl-1,3,2-dioxaborolan-2-yl)-1H-pyrrolo[2,3-b]pyridin-5-yl)phenyl)sulfonyl)morpholine

### a) 4-((4-(2-Chloro-1H-pyrrolo[2,3-b]pyridin-5-yl)-3-methylphenyl)sulfonyl)-morpholine

5-Bromo-2-chloro-1*H*-pyrrolo[2,3-*b*]pyridine (Preparation 1c) was treated with (2-methyl-4-(morpholinosulfonyl)phenyl)boronic acid, cesium carbonate, 1,1'-bis(diphenylphosphino)ferrocene-palladium(II) dichloride dichloromethane complex, dioxane and water according to the method described in Preparation 3a to give the title compound (51% yield).
LRMS (m/z): 392 (M+1)⁺.

### b) 4-((3-Methyl-4-(2-(4,4,5,5-tetramethyl-1,3,2-dioxaborolan-2-yl)-1H-pyrrolo[2,3-b]pyridin-5-yl)phenyl)sulfonyl)morpholine

4-((4-(2-Chloro-1*H*-pyrrolo[2,3-b]pyridin-5-yl)-3-methylphenyl)sulfonyl)morpholine (Preparation 40a) was treated with 4,4,4',4',5,5,5',5'-octamethyl-2,2'-bi(1,3,2-dioxaborolane, tricyclohexylphosphine), potassium acetate, tris(dibenzylideneacetone)dipalladium(0) and 1,2-dimethoxyethane as a solvent. Vial was capped and it was subjected to three cycles of evacuation-backfilling with argon and reaction was submitted to microwaves conditions heating at 120ºC for 2 h according to the method described in Preparation 3b to give the title compound (100% yield). The product was used without further purification.
LRMS (m/z): 484 (M+1)⁺.

### PREPARATION 41

### N,N,3-trimethyl-4-(2-(4,4,5,5-tetramethyl-1,3,2-dioxaborolan-2-yl)-1H-pyrrolo[2,3-b]pyridin-5-yl)benzenesulfonamide

### a) 4-(2-Chloro-1H-pyrrolo[2,3-b]pyridin-5-yl)-N,N,3-trimethylbenzenesulfonamide

5-Bromo-2-chloro-1*H*-pyrrolo[2,3-*b*]pyridine (Preparation 1c) was treated with (4-(*N,N-*dimethylsulfamoyl)-2-methylphenyl)boronic acid, cesium carbonate, 1,1'-bis(diphenylphosphino)ferrocene-palladium(II) dichloride dichloromethane complex, dioxane and water as a solvents according to the method described in Preparation 3a. The crude was purified by flash chromatography using SP1 Purification System (0% to 10%, dichloromethane-methanol) to give 435 mg (55% yield) of the title compound as a solid.
LRMS (m/z): 350 (M+1)⁺.

### b) N,N,3-Trimethyl-4-(2-(4,4,5,5-tetramethyl-1,3,2-dioxaborolan-2-yl)-1H-pyrrolo-[2,3-b]pyridin-5-yl)benzenesulfonamide

4-(2-Chloro-1H-pyrrolo[2,3-b]pyridin-5-yl)-N,N,3-trimethylbenzenesulfonamide (Preparation 41a) was treated with 4,4,4',4',5,5,5',5'-octamethyl-2,2'-bi(1,3,2-dioxaborolane, tricyclohexylphosphine), potassium acetate, tris(dibenzylideneacetone)-dipalladium(0) and 1,2-dimethoxyethane as a solvent. Vial was capped and it was subjected to three cycles of evacuation-backfilling with argon and reaction was submitted to microwaves conditions heating at 120ºC for 1 h according to the method described in Preparation 3b to give 674 mg (100% yield).
The product was used without further purification.
LRMS (m/z): 442 (M+1)⁺.

### PREPARATION 42

### 3-Bromo-5-(5-methyl-3-(trifluoromethyl)-1H-pyrazol-1-yl)pyridin-2-amine

### a) 5-(5-Methyl-3-(trifluoromethyl)-1H-pyrazol-1-yl)pyridin-2-amine

An oven dried resealable Schlenk tube was charged with 5-bromopyridin-2-amine (400 mg, 2.24 mmol), 5-methyl-3-(trifluoromethyl)-1*H*-pyrazole (340 mg, 2.24 mmol), potassium phosphate (972 mg, 4.49 mmol) and 7 ml dioxane were added. The Schlenk tube was subjected to three cycles of evacuation-backfilling with argon, and copper(I) iodide (65 mg, 0.34 mmol) and *N*,*N'*-dimethylcyclohexane-1,2-diamine (55 µl, 0.34 mmol) were added. After three further cycles of evacuation-backfilling with argon, the Schlenk tube was capped and placed in an oil bath at 130 ºC overnight. The mixture was cooled and was filtered through a plug of Celite. The filtered was concentrated under reduced pressure and the brown crude was purified by flash chromatography (0% to 50%, hexane-ethyl acetate) to give 476 mg (88% yield) of the title compound.
LRMS (m/z): 343 (M+1)⁺.

### b) 3-Bromo-5-(5-methyl-3-(trifluoromethyl)-1H-pyrazol-1-yl)pyridin-2-amine

5-(5-Methyl-3-(trifluoromethyl)-1*H*-pyrazol-1-yl)pyridin-2-amine (Preparation 42a) was treated with *N*-Bromosuccinimide and acetonitrile as a solvent according to the method described in Preparation 4b to obtain (90% yield) the title compound as a solid.
LRMS (m/z): 321, 323 (M+1)⁺.

### PREPARATION 43

### 3-((2-Chloro-6-fluorophenyl)ethynyl)-5-(5-methyl-3-(trifluoromethyl)-1H-pyrazol-1-yl)pyridin-2-amine

3-Bromo-5-(5-methyl-3-(trifluoromethyl)-1 *H*-pyrazol-1-yl)pyridin-2-amine (Preparation 42b) was treated with 1-chloro-2-ethynyl-3-fluorobenzene, triethylamine, copper (I) iodide, bis(triphenylphosphine)palladium (II) chloride and dimethylformamide as a solvent. The reaction was heating at 120ºC for 30 min under microwaves conditions following the method described in Preparation 5a. The crude was purified by flash chromatography (0% to 70%, hexane-ethyl acetate) to give (60% yield) the title compound as a brown solid.
LRMS (m/z): 395 (M+1)⁺.

### PREPARATION 44

### N-(3-((2-Chloro-6-fluorophenyl)ethynyl)-5-(5-methyl-3-(trifluoromethyl)-1H-pyrazol-1-yl)pyridin-2-yl)-2,2,2-trifluoroacetamide

3-((2-Chloro-6-fluorophenyl)ethynyl)-5-(5-methyl-3-(trifluoromethyl)-1*H*-pyrazol-1-yl)pyridin-2-amine (Preparation 43, 282 mg, 0.5 mmol) was dissolved in 13 ml dioxane. Trifluoroacetic anhydride (141 µl, 1 mmol) was added and the reaction was stirred at room temperature for 1 h. The mixture was evaporated to dryness and the residue was re-dissolved in dichloromethane. A brown solid was formed and was filtered to give 506 mg (100% yield) of the title compound.
LRMS (m/z): 491 (M+1)⁺.

### PREPARATION 45

### 2-Chloro-5-(4,4,5,5-tetramethyl-1,3,2-dioxaborolan-2-yl)-1H-pyrrolo[2,3-b]pyridine

5-Bromo-2-chloro-1*H*-pyrrolo[2,3-b]pyridine (Preparation 1c) was treated with 4,4,4',4',5,5,5',5'-octamethyl-2,2'-bi(1,3,2-dioxaborolane), 1,1'-bis(diphenylphosphino)-ferrocene]dichloropalladium(II), complex with dichloromethane and potassium acetate. The reaction was heated at 100ºC overnight according to the method described in Preparation 3a followed by purification by flash chromatography 0% to 10%, dichloromethane-methanol) to obtain (79% yield) the title compound.
LRMS (m/z): 279 (M+1)⁺.

### PREPARATION 46

### 5-Chloro-6-(2-chloro-1H-pyrrolo[2,3-b]pyridin-5-yl)-2-methylbenzo[d]oxazole

2-Chloro-5-(4,4,5,5-tetramethyl-1,3,2-dioxaborolan-2-yl)-1*H*-pyrrolo[2,3-*b*]pyridine (Preparation 45) was treated with 6-bromo-5-chloro-2-methylbenzo[*d*]oxazole1,1'-bis(diphenylphosphino)ferrocene]dichloropalladium(II), complex with dichloromethane, cesium carbonate and dioxane as a solvent according to the method described in Preparation 3a. The crude was purified by flash chromatography (0% to 10%, dichloromethane-methanol) to give (33% yield) the title compound as a yellow solid.
LRMS (m/z): 318 (M+1)⁺.

### PREPARATION 47

### 5-Chloro-2-methyl-6-(2-(4,4,5,5-tetramethyl-1,3,2-dioxaborolan-2-yl)-1H-pyrrolo[2,3-b]pyridin-5-yl)benzo[d]oxazole

5-Chloro-6-(2-chloro-1*H*-pyrrolo[2,3-*b*]pyridin-5-yl)-2-methylbenzo[*d*]oxazole (Preparation 46), 4,4,4',4',5,5,5',5'-octamethyl-2,2'-bi(1,3,2-dioxaborolane), tricyclohexylphosphine, potassium acetate, tris(dibenzylideneacetone)dipalladium(0) and 1,2-dimethoxyethane as a solvent according to the method described in Preparation 3b (100% yield).
LRMS (m/z): 410 (M+1)⁺.

### PREPARATION 48

### 4-(2-Iodo-1-(phenylsulfonyl)-1H-pyrrolo[3,2-b]pyridin-5-yl)-N,N,3-trimethylbenzenesulfonamide

### a) N,N,3-trimethyl-4-(1-(phenylsulfonyl)-1H-pyrrolo[3,2-b]pyridin-5-yl)benzenesulfonamide

5-Chloro-1-(phenylsulfonyl)-1H-pyrrolo[3,2-b]pyridine (Preparation 21d) was treated with (4-(*N*,*N*-dimethylsulfamoyl)-2-methylphenyl)boronic acid, potassium carbonate, [1,1'-bis(diphenylphosphino)ferrocene]dichloropalladium(II), complex with dichloromethane, dioxane and water as a solvents according to the method described in method 3a. The crude was purified by flash chromatography using SP1 Purification System (50% to 100%, hexane-diethyl ether) to give the title compound (55% yield) as a brown oil.
LRMS (m/z): 456 (M+1)⁺.

### b) 4-(2-Iodo-1-(phenylsulfonyl)-1H-pyrrolo[3,2-b]pyridin-5-yl)-N,N,3-trimethyl-benzenesulfonamide

*N*,*N*,3-trimethyl-4-(1-(phenylsulfonyl)-1*H*-pyrrolo[3,2-*b*]pyridin-5-yl)benzenesulfonamide (Preparation 48a) was treated with butyllithium solution (1.6M in hexane), iodine and tetrahydrofuran as a solvent according to the method described in Preparation 22b. The crude was purified by flash chromatography using Isolera Purification System (0% to 100%, hexane- (diethyl ether/ethanol 1%)) to obtain the title compound (35% yield) as a yellow oil.
LRMS (m/z): 582 (M+1)⁺.

### PREPARATION 49

### 3-Bromo-5-(2-methyl-4-(morpholinosulfonyl)phenyl)pyrazin-2-amine

### a) 5-(2-Methyl-4-(morpholinosulfonyl)phenyl)pyrazin-2-amine

5-Bromopyrazin-2-amine was treated with (2-methyl-4-(morpholinosulfonyl)-phenyl)boronic acid, cesium carbonate, [1,1'-bis(diphenylphosphino)ferrocene]-dichloropalladium(II), complex with dichloromethane and dioxane as a solvent according to the method described in Preparation 3a. The crude was purified by flash chromatography (0% to 100%, hexane-ethyl acetate) to give the title compound (74% yield).
LRMS (m/z): 335 (M+1)⁺.

### b) 3-Bromo-5-(2-methyl-4-(morpholinosulfonyl)phenyl)pyrazin-2-amine

5-(2-Methyl-4-(morpholinosulfonyl)phenyl)pyrazin-2-amine (Preparation 49a) was treated with *N*-bromosuccinimide and DMSO as a solvent according to the method described in Preparation 4b. The crude of the reaction was precipitated with water. A brown solid was formed and was dried in the oven overnight to obtain the title compound (50% yield).
LRMS (m/z): 413, 415 (M+1)⁺.

### PREPARATION 50

### 3-((2-Chloro-6-fluorophenyl)ethynyl)-5-(2-methyl-4-(morpholinosulfonyl)phenyl)pyrazin-2-amine

3-Bromo-5-(2-methyl-4-(morpholinosulfonyl)phenyl)pyrazin-2-amine (Preparation 49b) was treated with 1-chloro-2-ethynyl-3-fluorobenzene (Preparation 5b), triethylamine, copper (I) iodide, bis(triphenylphosphine)palladium(II)dichloride and tetrahydrofuran as a solvent. The reaction was heated at 90ºC for 2h according to the method described in Preparation 5a. The crude was purifed by flash chromatography using SP1 Purification System (0% to 100%, hexane-ethyl acetate) to give the title compound (43% yield) as a solid.
LRMS (m/z): 487 (M+1)⁺.

### PREPARATION 51

### 5-Chloro-1H-pyrrolo[2,3-c]pyridine

### a) (E)-2-(2-Chloro-5-nitropyridin-4-yl)-N,N-dimethylethenamine

In a sealed tube was dissolved 2-chloro-4-methyl-5-nitropyridine (1.5 g, 8.69 mmol) in dimethylformamide. *N,N*-Dimethylformamide dimethyl acetal (2.54 ml, 19.15 mmol) was added and the reaction was heated at 90ºC overnight. The mixture was allowed to cool and was co-evaporated with toluene to obtain the title compound (100% yield) as a brown solid.
LRMS (m/z): 228 (M+1)⁺.

### b) 5-Chloro-1H-pyrrolo[2,3-c]pyridine

(*E*)-2-(2-Chloro-5-nitropyridin-4-yl)-*N*,*N*-dimethylethenamine (Preparation 51 a, 2 g, 8.79 mmol)) was dissolved in 90 ml ethanol. Hydrogen hydrochloride (35%, 807 ml, 2000 mmol) and iron (powder, 1.77 g, 32 mmol) were added and the reaction was heated at 85ºC for 3 h. The mixture was allowed to cool and was filtered through a plug of celite, washing several times with ethyl acetate.The combined filtered were washed with water and the aqueous layer was basified with ammonia. The organics were combined, dried over sodium sulphate, filtered and evaporated under reduced pressure. The crude was purified by flash chromatography using SP1 Purification System (0% to 50%, hexane-ethyl acetate) to give 570 mg (43% yield) of the title compound as a brown solid.
LRMS (m/z): 153 (M+1)⁺.

### PREPARATION 52

### 2-Iodo-5-(6-methoxy-4-methylpyridin-3-yl)-1-(phenylsulfonyl)-1H-pyrrolo[2,3-c]pyridine

### a) 5-Chloro-1-(phenylsulfonyl)-1H-pyrrolo[2,3-c]pyridine

5-Chloro-1*H*-pyrrolo[2,3-*c*]pyridine (Preparation 51 b) was treated with benzenesulfonyl chloride, sodium hydride 60% and dimethylformamide as a solvent according to the method described in Preparation 21d to obtain the title compound (65% yield) as a yellow solid.
LRMS (m/z): 293 (M+1)⁺.

### b) 5-(6-Methoxy-4-methylpyridin-3-yl)-1-(phenylsulfonyl)-1H-pyrrolo[2,3-c]pyridine

5-Chloro-1-(phenylsulfonyl)-1*H*-pyrrolo[2,3-*c*]pyridine (Preparation 52a) was treated with (6-methoxy-4-methylpyridin-3-yl)boronic acid, 2M cesium carbonate, [1,1'-bis(diphenylphosphino)ferrocene]dichloropalladium(II) complex with dichloromethane and dioxane as a solvent according to the method described in Preparation 3a. The crude was purified by flash chromatography using SP1 Purification System (0% to 100%, hexane-diethyl ether) to give the title compound (72% yield) as a yellow solid.
LRMS (m/z): 380 (M+1)⁺.

### c) 2-Iodo-5-(6-methoxy-4-methylpyridin-3-yl)-1-(phenylsulfonyl)-1H-pyrrolo[2,3-c]pyridine

5-(6-Methoxy-4-methylpyridin-3-yl)-1-(phenylsulfonyl)-1*H*-pyrrolo[2,3-*c*]pyridine (Preparation 52b) was treated with butyllithium solution (1.6 M in hexane), iodine and tetrahydrofuran as a solvent according to the method described in Preparation 22b to obtain the title compound (50% yield) as a solid.
LRMS (m/z): 506 (M+1)⁺.

### PREPARATION 53

### 4-(5-Amino-6-bromopyrazin-2-yl)-3-methylbenzenesulfonamide

### a) 4-(5-Aminopyrazin-2-yl)-3-methylbenzenesulfonamide

5-Bromopyrazin-2-amine was treated with (2-methyl-4-sulfamoylphenyl)boronic acid, cesium carbonate, [1,1'-bis(diphenylphosphino)ferrocene]dichloropalladium(II), complex with dichloromethane and dioxane as a solvent according to the method described in Preparation 3a. The crude was purified by flash chromatography (0% to 100%, hexane-ethyl acetate) to give the title compound (67% yield).
LRMS (m/z): 265 (M+1)⁺.

### b) 4-(5-Amino-6-bromopyrazin-2-yl)-3-methylbenzenesulfonamide

4-(5-Aminopyrazin-2-yl)-3-methylbenzenesulfonamide (Preparation 53a, 180 mg, 0.68 mmol)) was dissolved in 3 ml DMSO and 0.2 ml water. The mixture was cooled at 0ºC and *N*-Bromosuccinimide (121 mg, 0.68 mmol) was added. The reaction was stirred at 0ºC for 1h. The reaction mixture was poured into water and a solid was formed. This solid was filtered and dried in the oven to give 208 mg (89% yield) of the title compound as a yellow solid.
LRMS (m/z): 343, 345 (M+1)⁺.

### PREPARATION 54

### 4-(5-Amino-6-((2-chloro-6-fluorophenyl)ethynyl)pyrazin-2-yl)-3-methylbenzenesulfonamide

4-(5-Amino-6-bromopyrazin-2-yl)-3-methylbenzenesulfonamide (Preparation 53b) was treated with 1-chloro-2-ethynyl-3-fluorobenzene (Preparation 5b), copper (I) iodide, bis(triphenylphosphine)palladium (II) chloride, triethylamine and tetrahydrofuran as a solvent according to the method described in Preparation 5a to obtain the title compound (16% yield).
LRMS (m/z): 417 (M+1)⁺.

### PREPARATION 55

### (2-Methyl-4-(N-methylsulfamoyl)phenyl)boronic acid

### a) 4-Bromo-N,3-dimethylbenzenesulfonamide

4-Bromo-3-methylbenzene-1-sulfonyl chloride (3 g, 14.13 mmol) was dissolved in 110 ml dichloromethane. Triethylamine (1.90 ml, 13.63 mmol) was added and the reaction was cooled at 0ºC. Methylamine solution (2M in THF, 1.9 ml, 13.63 mmol) was added drop-wise and the reaction was stirred overnight. The mixture was partitioned between dichloromethane and water and the organic phase was washed with hydrogen hydrochloride 1 N, brine, dried over sodium sulphate, filtered and evaporated under reduced pressure to give 2.75 g (89% yield) of the title compound as an oil.
LRMS (m/z): 264 (M+1)⁺.

### b) (2-Methyl-4-(N-methylsulfamoyl)phenyl)boronic acid

4-Bromo-*N*,3-dimethylbenzenesulfonamide (Preparation 55a) was treated with 4,4,4',4',5,5,5',5'-octamethyl-2,2'-bi(1,3,2-dioxaborolane), 1,1'-bis(diphenylphosphino)-ferrocene]dichloropalladium(II), complex with dichloromethane, potassium acetate and dioxane as a solvent. The reaction was heated at 100ºC overnight according to the method described in Preparation 3a under standard conditions. The crude was purified by flash chromatography (0% to 100%, hexane-dichloromethane) to obtain the title compound (18% yield) as a boronic acid.
LRMS (m/z): 230 (M+1)⁺.

### PREPARATION 56

### 4-(5-Amino-6-bromopyrazin-2-yl)-N,3-dimethylbenzenesulfonamide

### a) 4-(5-Aminopyrazin-2-yl)-N,3-dimethylbenzenesulfonamide

In a Schlenk vessel 5-bromopyrazin-2-amine was treated with (2-methyl-4-(N-methylsulfamoyl)phenyl)boronic acid (Preparation 55b), cesium carbonate, [1,1'-bis(diphenylphosphino)ferrocene]dichloropalladium(II) complex with dichloromethane and dioxane as a solvent according to the method described in Preparation 3a and heating at 100ºC 3 h under standard conditions. The crude was purified by flash chromatography (0% to 100%, hexane-ethyl acetate) to give the title compound (91% yield).
LRMS (m/z): 279 (M+1)⁺.

### b) 4-(5-Amino-6-bromopyrazin-2-yl)-N,3-dimethylbenzenesulfonamide

4-(5-Aminopyrazin-2-yl)-*N*,3-dimethylbenzenesulfonamide (Preparation 56a) was treated with *N*-Bromosuccinimide, DMSO and water as a solvents according to the method described in Preparation 53b. The crude was purified by flash chromatography using lsolera Purification System (0%-50%, dichloromethane-(dichloromethane-methanol 5%)) to give the title compound (20% yield).
LRMS (m/z): 357, 359 (M+1)⁺.

### PREPARATION 57

### 4-(5-Amino-6-((2-chloro-6-fluorophenyl)ethynyl)pyrazin-2-yl)-N,3-dimethylbenzenesulfonamide

4-(5-Amino-6-bromopyrazin-2-yl)-*N*,3-dimethylbenzenesulfonamide (Preparation 56b) was treated with 1-chloro-2-ethynyl-3-fluorobenzene (Preparation 5b), copper (I) iodide, bis(triphenylphosphine)palladium (II) chloride, triethylamine and tetrahydrofuran as a solvent according to the method described in Preparation 5a to obtain the title compound (61% yield).
LRMS (m/z): 431 (M+1)⁺.

### PREPARATION 58

### 2-Benzyl-5-(1-methyl-3-(trifluoromethyl)-1H-pyrazol-5-yl)-1-(phenylsulfonyl)-1H-pyrrolo[3,2-b]pyridine

(5-(1-Methyl-3-(trifluoromethyl)-1H-pyrazol-5-yl)-1-(phenylsulfonyl)-1H-pyrrolo[3,2-b]pyridin-2-yl)boronic acid (Preparation 24, 200 mg, 0.28 mmol) was dissolved in 1 ml tetrahydrofuran. Under argon conditions (bromomethyl)benzene (43 µl, 0.36 mmol), potassium carbonate (128 mg, 0.92 mmol) and tetrakis(triphenylphosphine)palladium(0) (13 mg, 0.01 mmol) were added and the reaction was heated at 80ºC for 6h. The reaction mixture was partitioned between ethyl acetate and water. The organic layer was dried over sodium sulphate, filtered and evaporated under reduced pressure. The crude was purified by flash chromatography (0% to 40%, hexane-diethyl ether) to obtain 61 mg (44 % yield) of the title compound as a white solid.
LRMS (m/z): 497 (M+1)⁺.

### PREPARATION 59

### 3-((2,6-Difluorophenyl)ethynyl)-5-(1-methyl-3-(trifluoromethyl)-1H-pyrazol-5-yl)pyrazin-2-amine

3-Bromo-5-(1-methyl-3-(trifluoromethyl)-1*H*-pyrazol-5-yl)pyrazin-2-amine (Preparation 6) was treated with 2-ethynyl-1,3-difluorobenzene (Preparation 16b), copper (I) iodide, bis(triphenylphosphine)palladium (II) chloride, triethylamine and tetrahydrofuran as a solvent according to the method described in Preparation 5a. The crude was purified by flash chromatography (0% to 20%, hexane-ethyl acetate) to obtain the title compound (20% yield).
LRMS (m/z): 380 (M+1)⁺.

### PREPARATION 60

### 2-Bromo-6-(5-methyl-3-(trifluoromethyl)-1H-1,2,4-triazol-1-yl)pyridin-3-amine

### a) 2-(5-Methyl-3-(trifluoromethyl)-1H-1,2,4-triazol-1-yl)-5-nitropyridine

5-Methyl-3-(trifluoromethyl)-1H-1,2,4-triazole (400 mg, 2.65 mmol) was dissolved in 10 ml tetrahydrofuran and potassium carbonate (731 mg, 5.29 mmol) was added. The reaction was heated at 40ºC for 30 min and then 2-chloro-5-nitropyridine (335 mg, 2.11 mmol) was added. The reaction was heated at 40ºC overnight. The mixture was diluted in diethyl ether and washed with water. The organic layer was dried over sodium sulphate, filtered and evaporated under reduced pressure. The crude was purified by flash chromatography using SP1 Purification System (0% to 100%, hexane-diethyl ether) to obtain 315 mg (57% yield) of the final compound. Purity 100%.
LRMS (m/z): 274 (M+1)⁺.

### b) 6-(5-Methyl-3-(trifluoromethyl)-1H-1,2,4-triazol-1-yl)pyridin-3-amine

2-(5-Methyl-3-(trifluoromethyl)-1*H*-1,2,4-triazol-1-yl)-5-nitropyridine (Preparation 60a, 315 mg, 1.15 mmol) was dissolved in 10 ml ethanol. Under argon atmosphere palladium on carbon (10%, 3 mg, 0.03 mmol) was added and the reaction mixture was hydrogenated for 6 h. The catalyst was filtered and the filtered was concentrated under reduced pressure to give 270 mg (87% yield) of the title compound as a oil.
LRMS (m/z): 244 (M+1)⁺.

### c) 2-Bromo-6-(5-methyl-3-(trifluoromethyl)-1H-1,2,4-triazol-1-yl)pyridin-3-amine

6-(5-Methyl-3-(trifluoromethyl)-1*H*-1,2,4-triazol-1-yl)pyridin-3-amine (Preparation 60b, 270 mg, 1.11 mmol) was dissolved in 10 ml acetonitrile. The mixture was cooled at 0°C and *N*-bromosuccinimide (210 mg, 1.18 mmol) was added. The reaction mixture was stirred at 0ºC for 30 min. The mixture was poured into water and a precipitate was formed. This solid was filtered and washed with water then was dried in the oven for 3 h to obtain 310 mg (68% yield) of the desired compound.
LRMS (m/z): 322, 324 (M+1)⁺.

### (Preparation 61

### 5-bromo-2-(2-chloro-6-fluorophenyl)-1H-pyrrolo[2,3-b]pyridine

### a)5-bromo-3-[(2-chloro-6-fluorophenyl)ethynyl]pyridin-2-amine

Obtained (74% yield) from 5-bromo-3-iodopyridin-2-amine and 1-chloro-2-ethynyl-3-fluorobenzene (Preparation 5) following the experimental procedure as described in Preparation 5a, using thermal conditions (110ºC, 3h) instead of microwave irradiation and followed by flash chromatography purification (0-100%, hexane - diethyl ether) afforded the title compound as a yellow solid
LRMS (m/z): 325,327 (M+1)⁺.

### b)N-{5-bromo-3-[(2-chloro-6-fluorophenyl)ethynyl]pyridin-2-yl}-2,2,2-trifluoroacetamide

Obtained (96% yield) from 5-bromo-3-[(2-chloro-6-fluorophenyl)ethynyl]pyridin-2-amine (preparation 61 a) and trifluoroacetic anhydride following the experimental procedure as described in Preparation 44 to give the title compound as a brown solid.
LRMS (m/z): 421,423 (M+1)⁺.

### c)5-bromo-2-(2-chloro-6-fluorophenyl)-1H-pyrrolo[2,3-b]pyridine

N-{5-bromo-3-[(2-chloro-6-fluorophenyl)ethynyl]pyridin-2-yl}-2,2,2-trifluoroacetamide (preparation 61b, 295 mg, 0.59 mmol) was dissolved in 3 ml dimethylformamide. Triethylamine (82 µl, 0.60 mmol) was added and the reaction was heated at 100ºC for 3 days in a sealed tube. The solvent was evaporated to dryness and the crude was dissolved in AcOEt, the organic phase was washed with water, dried (MgSO4 and evaporated and the crude was purifed by flash chromatography (0% to 100%, hexane-diethyl ether) to give the title compound as a yellow solid (46% yield)
LRMS (m/z): 325,327 (M+1)⁺.

### Preparation 62

### (4,6-dimethoxypyridin-3-yl)boronic acid

### a) 5-bromo-2,4-dimethoxypyridine

A mixture of 2,4 dimethoxypyridine (0.5 g, 3.59 mmol) and N-bromosuccinimide (0.64 g, 3.6 mmol) in anhydrous CH₃CN (10 ml) was stirred in dark for 16 h at 75 °C. After cooling to room temperature the mixture was evaporated to dryness and the crude was dissolved in AcOEt, the organic phase was washed with water, dried (MgSO4) and evaporated. Purification by flash chromatography (0% to 100%, hexane-diethyl ether) afforded the title compound as a white solid (66% yield).
LRMS (m/z): 218, 220 (M+1)⁺.

### b) (4,6-dimethoxypyridin-3-yl)boronic acid

5-bromo-2,4-dimethoxypyridine (preparation 62a, 0.52mg, 2.38 mmol) was treated with 4,4,4',4',5,5,5',5'-octamethyl-2,2'-bi(1,3,2-dioxaborolane) (0.908 mg, 3.58 mmol),1,1' bis(diphenylphosphino)ferrocene]dichloropalladium(II) complex with dichloromethane (195 mg, 0.24 mmol), potassium acetate (0.702 mg, 7.15 mmol) and dioxane (8 mL) as a solvent. The reaction was heated at 110ºC overnight according to the method described in Preparation 3a under standard conditions. The crude was purified by flash chromatography (0% to 100%, hexane - diethyl ether) to give the title compound 55% yield) to give the title compound as a uncoloured oil (55% yield).
LRMS (m/z): 184 (M+1)⁺.

### Preparation 63

### 4-methyl-5-(4,4,5,5-tetramethyl-1,3,2-dioxaborolan-2-yl)-2-(trifluoromethyl)pyridine

### a) 5-bromo-4-methyl-2-(trifluoromethyl)pyridine

To a suspension of 5-bromo-2-iodo-4-methylpyridine(1g, 3.36 mmol) in NMP (6mL) with an inert atmosphere of argon, KF (0.585 g, 10.07 mmol), Cul (1.92 g, 10.08 mmol) and CF₃SiMe₃ (2.49 mL, 15.07 mmol) were added. The resulting solution was stirred overnight at 60ºC. After cooling, the mixture was poured into 12% aqueous ammonia, and then extracted with Et₂O. The organic solutions were combined, dried over MgSO₄, filtered and concentrated. Purification of the crude residue by normal phase chromatography (0% to 100%, hexane - diethyl ether) afforded the desired product (23% yield) as a yellow oil.

### b)4-methyl-5-(4,4,5,5-tetramethyl-1,3,2-dioxaborolan-2-yl)-2-(trifluoromethyl)pyridine

5-bromo-4-methyl-2-(trifluoromethyl)pyridine (Preparation 63a, 0.27mg, 1.13 mmol) was treated with 4,4,4',4',5,5,5',5'-octamethyl-2,2'-bi(1,3,2-dioxaborolane) (0.57 mg, 2.27 mmol),1,1' bis(diphenylphosphino)ferrocene]dichloropalladium(II) complex with dichloromethane (56 mg, 0.07 mmol), potassium acetate (0.33 mg, 3.4 mmol) and dioxane (3 mL) as a solvent. The reaction was heated at 110ºC overnight according to the method described in Preparation 3a under standard conditions. The crude was purified by flash chromatography (10%, dichloromethane-methanol) to give the title compound (0.325 g, 93% yield) as a brown solid.
LRMS (m/z): 288 (M+1)⁺.

### Preparation 64

### 5-chloro-2-(2-chloro-6-fluorophenyl)-1-(phenylsulfonyl)-1H-pyrrolo[2,3-c]pyridine

### a) 6-chloro-4-[(2-chloro-6-fluorophenyl)ethynyl]pyridin-3-amine

Obtained (51% yield) from 6-chloro-4-iodopyridin-3-amine and 1-Chloro-2-ethynyl-3-fluorobenzene (preparation 5), following the experimental procedure as described in Preparation 5a, using thermal conditions (110ºC, 2h) instead of microwave irradiation and followed by flash chromatography purification (0% to 100%, hexane - diethyl ether) as a yellow solid.
LRMS (m/z): 282 (M+1)⁺.

### b) 5-chloro-2-(2-chloro-6-fluorophenyl)-1H-pyrrolo[2,3-c]pyridine

Obtained (24% yield) as a brown oil from 6-chloro-4-[(2-chloro-6-fluorophenyl)ethynyl]pyridin-3-amine (preparation 64a) following the experimental procedure as described in Preparation 14c and followed by flash chromatography purification (0% to 100%, hexane - diethyl ether).
LRMS (m/z): 281 (M+1)⁺.

### c)5-chloro-2-(2-chloro-6-fluorophenyl)-1-(phenylsulfonyl)-1H-pyrrolo[2,3-c]pyridine

Obtained (42% yield) as a white solid from 5-chloro-2-(2-chloro-6-fluorophenyl)-1H-pyrrolo[2,3-c]pyridine (preparation 64b) following the experimental procedure as described in Preparation 21 d and and followed by flash chromatography purification (0% to 100%, hexane - diethyl ether).
LRMS (m/z): 421 (M+1)⁺.

### EXAMPLE 1

### 2-(2-Chloro-6-fluorophenyl)-5-(6-methoxy-4-methylpyridin-3-yl)-1H-pyrrolo[2,3-b]pyridine

A microwave tube was charged with 5-(6-methoxy-4-methylpyridin-3-yl)-2-(4,4,5,5-tetramethyl-1,3,2-dioxaborolan-2-yl)-1H-pyrrolo[2,3-b]pyridine (Preparation 3b, 0.5 g, 0.9 mmol), 1-chloro-3-fluoro-2-iodobenzene (0.278 g, 1.08 mmol), cesium carbonate (0.442 g, 1.36 mmol), dioxane (6 ml) and water (1.2 ml). The mixture was degassed with argon and 1,1'-bis(diphenylphosphino)ferrocene-palladium(II) dichloride dichloromethane complex (0.037 g, 0.05 mmol) was added. After capping the vial, it was subjected to three further cycles of evacuation-backfilling with argon and heated at 80ºC. After 1h, saturated aqueous ammonium chloride solution was added and product was extracted with ethyl acetate. Organic layer was dried (Na₂SO₄) and evaporated. Purification of the residue by flash chromatography (0% to 10%, dichloromethane-methanol) to give the title compound (0.087 g, 26% yield).
LRMS (m/z): 368 (M+1)⁺.
¹H NMR (300 MHz, CHLOROFORM-*d*) d ppm 2.28 (s, 3 H) 3.99 (s, 3 H) 6.72 (s, 1 H) 6.92 (s, 1 H) 7.13 - 7.24 (m, 1 H) 7.31 - 7.43 (m, 2 H) 7.91 (s, 1 H) 8.06 (s, 1 H) 8.24 (d, *J*=2.35 Hz, 1 H) 9.89 (s, 1 H).

### EXAMPLE 2

### 6-(2-Chloro-6-fluorophenyl)-2-(6-methoxy-4-methylpyridin-3-yl)-5H-pyrrolo[2,3-b]pyrazine

### a) 3-[(2-Chloro-6-fluorophenyl)ethynyl]-5-(6-methoxy-4-methylpyridin-3-yl)pyrazin -2-amine

Obtained (60% yield) from 7-bromo-2-(6-methoxy-4-methylpyridin-3-yl)-5H-pyrrolo[2,3-b]pyrazin-6-amine (Preparation 4b) and 1-ahlaro-2-ethynyl-3-fluorabenzene (Preparation 5b) following the experimental procedure as described in Preparation 5a followed by precipitation in 2-propanol.
LRMS (m/z): 369 (M+1)⁺.

### b) N-[3-[(2-Chloro-6-fluorophenyl)ethynyl]-5-(6-methoxy-4-methylpyridin-3-yl) pyrazin-2-yl]-2,2,2-trifluoroacetamide

3-[(2-Chloro-6-fluorophenyl)ethynyl]-5-(6-methoxy-4-methylpyridin-3-yl)pyrazin-2-amine (Example 2a, 0.15 mg, 0.407 mmol) was dissolved in 10 ml 1,4-dioxane (extra dry) and trifluoroacetic anhydride (0.115 ml, 0.813 mmol) was added and the mixture was stirred at room temperature for 2h. Reaction mixture was partitioned between water and ethyl acetate and organic layer was washed with brine, dried (Na₂SO₄), filtered and concentrated to afford the desired product (0.18 g, 95% yield) as a white solid.
LRMS (m/z): 465 (M+1)⁺.

### c) 6-(2-Chloro-6-fluorophenyl)-2-(6-methoxy-4-methylpyridin-3-yl)-5H-pyrrolo[2,3-b]pyrazine

*N*-[3-[(2-Chloro-6-fluorophenyl)ethynyl]-5-(6-methoxy-4-methylpyridin-3-yl) pyrazin-2-yl]-2,2,2-trifluoroacetamide (Example 2b, 0.18 g, 0.387 mmol) was dissolved in *N*,*N-*dimethylformamide (2 ml), triethylamine (0.2 ml, 1.435 mmol) was added and the mixture was stirred at 100ºC overnight. Reaction mixture was partitioned between water and ethyl acetate, organic layer was washed with brine, dried (Na₂SO₄), filtered and concentrated. Purification of the crude residue by flash chromatography (10% to 70% heptane - ethyl acetate) afforded the title compound (0.032 g, 22%) as a white solid.
LRMS (m/z): 369 (M+1)⁺.
¹H NMR (400 MHz, CHLOROFORM-*d*) d ppm 2.44 (s, 3 H), 4.00 (s, 3 H), 6.74 (s, 1 H), 7.13 - 7.16 (m, 1 H), 7.19 - 7.25 (m, 1 H), 7.37 - 7.44 (m, 2 H), 8.24 (s, 1 H), 8.37 (s, 1 H), 9.59 (s, 1 H).

### EXAMPLE 3

### 6-(2-Chloro-6-fluorophenyl)-2-[1-methyl-3-(trifluoromethyl)-1H-pyrazol-5-yl]-5H-pyrrolo[2,3-b]pyrazine

### a) 3-[(2-Chloro-6-fluorophenyl)ethynyl]-5-[1-methyl-3-(trifluoromethyl)-1H-pyrazol-5-yl]pyrazin-2-amine

Obtained (100% yield) from 3-bromo-5-[1-methyl-3-(trifluoromethyl)-1*H*-pyrazol-5-yl]pyrazin-2-amine (Preparation 6b) and 1-ch)oro-2-ethyny)-3-f!uorobenzene (Preparation 5b) following the experimental procedure as described in Preparation 5a.
LRMS (m/z): 396 (M+1)⁺.

### b) N-{3-[(2-Chloro-6-fluorophenyl)ethynyl]-5-[l-methyl-3-(trifluoromethyl)-1H-pyrazol-5-yl]pyrazin-2-yl}-2,2,2-trifluoroacetamide

Obtained as a brown solid (62% yield) from 3-[(2-chloro-6-fluorophenyl)ethynyl]-5-[1-methyl-3-(trifluoromethyl)-1*H*-pyrazol-5-yl]pyrazin-2-amine (Example 3a) following the experimental procedure as described in Example 2b.
LRMS (m/z): 492 (M+1)⁺.

### c) 6-(2-Chloro-6-fluorophenyl)-2-[1-methyl-3-(trifluoromethyl)-1H-pyrazol-5-yl]-5H-pyrrolo[2,3-b]pyrazine

Obtained (35% yield) from *N*-{3-[(2-chloro-6-fluorophenyl)ethynyl]-5-[1-methyl-3-(trifluoromethyl)-1*H*-pyrazol-5-yl]pyrazin-2-yl}-2,2,2-trifluoroacetamide (Example 3b) following the experimental procedure as described in Example 2c, followed by reverse phase purification using SP1 Purification System.
LRMS (m/z): 396 (M+1)⁺.
¹H NMR (300 MHz, CHLOROFORM-*d*) d ppm 4.33 (s, 3 H), 6.89 (s, 1 H), 7.17 (s, 1 H), 7.20 - 7.24 (m, 1 H), 7.41 - 7.48 (m, 2 H), 8.58 (s, 1 H), 9.70 (s, 1 H).

### EXAMPLE 4

### 2-(2,6-Difluorophenyl)-5-(6-methoxy-4-methylpyridin-3-yl)-1H-pyrrolo[2,3-b]pyridine

Obtained (23% yield) from 5-(6-methoxy-4-methylpyridin-3-yl)-2-(4,4,5,5-tetramethyl-1,3,2-dioxaborolan-2-yl)-1*H*-pyrrolo[2,3-*b*]pyridine (preparation 3b) and 1,3-difluoro-2-iodobenzene following the experimental procedure as described in Example 1, followed by purification by flash chromatography (30% to 50% heptane - ethyl acetate).
LRMS (m/z): 352 (M+1)⁺.
¹H NMR (400 MHz, DMSO-*d*₆) d ppm 2.26 (s, 3 H), 3.88 (s, 3 H), 6.83 (s, 1 H), 7.27 - 7.35 (m, 2 H), 7.50 - 7.61 (m, 1 H), 8.01 (d, *J*=2.02 Hz, 1 H), 8.05 (s, 1 H), 8.25 (d, *J*=2.27 Hz, 1 H), 12.12 (s, 1 H).

### EXAMPLE 5

### 2-Cyclohexyl-5-(6-methoxy-4-methylpyridin-3-yl)-1H-pyrrolo[2,3-b]pyridine

### a) 5-(Cyclohexylethynyl)-6'-methoxy-4'-methyl-3,3'-bipyridin-6-amine

In a microwave vial 5-bromo-6'-methoxy-4'-methyl-3,3'-bipyridin-6-amine (Preparation 7b, 0.100 g, 0.340 mmol) was added in 3 ml *N,N*-dimethylformamide (dry) and triethylamine (0.284 ml, 2.040 mmol). Argon was bubbled through the solution for 10 min and then copper (I) iodide (0.0026 g, 0.014 mmol), tetrakis(triphenylphosphine) palladium (0) (0.0079 g, 0.007 mmol) and ethynylcyclohexane (0.037 g, 0.340 mmol) were added. The mixture was heated in the microwave at 120ºC for 30 min. The reaction mixture was diluted with dichloromethane and washed with water and brine, dried (Na₂SO₄) and solvent was evaporated. The crude product was purified by flash chromatography (0% to 50% heptane - ethyl acetate) to give the title compound (0.070 g, 48%)
LRMS (m/z): 322 (M+1)⁺.

### b) N-[5-(Cyclohexylethynyl)-6'-methoxy-4'-methyl-3,3'-bipyridin-6-yl]-2,2,2-trifluoroacetamide

Obtained (100% yield) from 5-(cyclohexylethynyl)-6'-methoxy-4'-methyl-3,3'-bipyridin-6-amine (Example 5a) and following the experimental procedure as described in Example 2b.
LRMS (m/z): 418 (M+1)⁺.

### c) 2-Cyclohexyl-5-(6-methoxy-4-methylpyridin-3-yl)-1H-pyrrolo[2,3-b]pyridine

Obtained (53% yield) from *N*-[5-(cyclohexylethynyl)-6'-methoxy-4'-methyl-3,3'-bipyridin-6-yl]-2,2,2-trifluoroacetamide (Example 5b) following the experimental procedure as described in Example 2c, followed by reverse phase purification using SP1 Purification System.
LRMS (m/z): 322 (M+1)⁺.
Partial ¹H NMR (400 MHz, CHLOROFORM-*d*) d ppm 1.40 - 1.61 (m, 4 H), 1.84 - 1.94 (m, 2 H), 2.08 - 2.17 (m, 2 H), 2.25 (s, 3 H), 3.97 (s, 3 H), 6.22 (s, 1 H), 6.69 (s, 1 H), 7.72 (s, 1 H), 8.02 (s, 1 H), 8.13 (s, 1 H).

### EXAMPLE 6

### 5-(6-Methoxy-4-methylpyridin-3-yl)-2-(4-methylpyridin-3-yl)-1H-pyrrolo[2,3-b]pyridine

5-(6-Methoxy-4-methytpyridin-3-yl)-2-(4,4,5,5-tetramethyl-1,3,2-dioxaborolan-2-yl)-1*H-*pyrrolo[2,3-b]pyridine (Preparation 3b) was treated with 3-iodo-4-methylpyridine, cesium carbonate, dioxane and water as a solvent according to the method described in Example 1 to give the title compound (24% yield).
LRMS (m/z): 331 (M+1)⁺.
¹H NMR (400 MHz, DMSO-*d*₆) d ppm 2.26 (s, 3 H), 2.46 (s, 3 H), 3.88 (s, 3 H), 6.72 (s, 1 H), 6.83 (s, 1 H), 7.41 (d, *J*=5.05 Hz, 1 H), 7.98 (d, *J*=2.27 Hz, 1 H), 8.05 (s, 1 H), 8.22 (d, *J*=1.77 Hz, 1 H), 8.47 (d, *J*=4.80 Hz, 1 H), 8.73 (s, 1 H), 12.17 (s, 1 H).

### EXAMPLE 7

### 6-(2-Chloro-6-fluorophenyl)-2-[1-ethyl-3-(trifluoromethyl)-1H-pyrazol-5-yl]-5H-pyrrolo[2,3-b]pyrazine

### a) 3-[(2-chloro-6-fluorophenyl)ethynyl]-5-[1-ethyl-3-(trifluoromethyl)-1H-pyrazol-5-yl]pyrazin-2-amine

Obtained (85% yield) from 3-bromo-5-[1-ethyl-3-(trifluoromethyl)-1*H*-pyrazol-5-yl]pyrazin-2-amine (Preparation 9b) and 1-chloro-2-ethynyl-3-fluorobenzene (Preparation 5b) following the experimental procedure as described in Example 5a, followed by flash chromatography (10% to 70% heptane - ethyl acetate in heptane) using SP1 Purification System.
LRMS (m/z): 410 (M+1)⁺.

### b) N-{3-[(2-chloro-6-fluorophenyl)ethynyl]-5-[1-ethyl-3-(trifluoromethyl)-1H-pyrazol-5-yl]pyrazin-2-yl}-2,2,2-trifluoroacetamide

Obtained (93% yield) from 3-[(2-chloro-6-fluorophenyl)ethynyl]-5-[1-ethyl-3-(trifluoromethyl)-1*H*-pyrazol-5-yl]pyrazin-2-amine (Example 7a) and following the experimental procedure as described in Example 2b.
LRMS (m/z): 506 (M+1)⁺.

### c) 6-(2-chloro-6-fluorophenyl)-2-[1-ethyl-3-(trifluoromethyl)-1H-pyrazol-5-yl]-5H-pyrrolo[2,3-b]pyrazine

Obtained (36% yield) from *N*-{3-[(2-chloro-6-fluorophenyl)ethynyl]-5-[1-ethyl-3-(trifluoromethyl)-1H-pyrazol-5-yl]pyrazin-2-yl}-2,2,2-trifluoroacetamide (Example 7b) following the experimental procedure as described in Example 2c, followed by flash chromatography (10% to 70% heptane - ethyl acetate) and then reverse phase purification using SP1 Purification System.
LRMS (m/z): 410 (M+1)⁺.

### EXAMPLE 8

### 4-[6-(2-chloro-6-fluorophenyl)-5H-pyrrolo[2,3-b]pyrazin-2-yl]-N,N,3-trimethylbenzenesulfonamide

### a) 4-{5-amino-6-[(2-chloro-6-fluorophenyl)ethynyl]pyrazin-2-yl}-N,N,3-trimethyl benzenesulfonamide

Obtained (52% yield) from 5-bromo-6'-methoxy-4'-methyl-3,3'-bipyridin-6-amine (preparation 10b) and 1-chloro-2-ethynyl-3-fluorobenzene (preparation 5b) following the experimental procedure as described in Example 5a, followed by flash chromatography (10% to 70%, heptane - ethyl acetate).
LRMS (m/z): 445 (M+1)⁺.

### b) N-(3-[(2-chloro-6-fluorophenyl)ethynyl]-5-{4-[(dimethylamino)sulfonyl]-2-methylphenyl}pyrazin-2-yl)-2,2,2-trifluoroacetamide

Obtained (15% yield) as a solid from 4-{5-amina-6-[(2-chloro-6-fluorophenyl)ethynyl]pyrazin-2-yl}-*N*,*N*,3-trimethyl benzenesulfonamide (Example 8a) and following the experimental procedure as described in Example 2b.
LRMS (m/z): 541 (M+1)⁺.

### c) 4-[6-(2-chloro-6-fluorophenyl)-5H-pyrrolo[2,3-b]pyrazin-2-yl]-N,N,3-trimethyl benzenesulfonamide

Obtained (8% yield) from *N*-(3-[(2-chloro-6-fluorophenyl)ethynyl]-5-{4-[(dimethylamino) sulfonyl]-2-methylphenyl}pyrazin-2-yl)-2,2,2-trifluoroacetamide (Example 8b) following the experimental procedure as described in Example 2c, followed by flash chromatography (10% to 70%, heptane - ethyl acetate) and then reverse phase purification using SP1 Purification System.
LRMS (m/z): 445 (M+1)⁺.
¹H NMR (400 MHz, CHLOROFORM-*d*) d ppm 2.53 (s, 3 H), 2.76 (s, 6 H), 7.15 - 7.17 (m, 1 H), 7.20 - 7.25 (m, 1 H), 7.40 - 7.44 (m, 2 H), 7.64 - 7.68 (m, 1 H), 7.71 - 7.75 (m, 1 H), 7.76 (s, 1 H), 8.42 (s, 1 H).

### EXAMPLE 9

### 3-{3,5-difluoro-4-[5-(6-methoxy-4-methylpyridin-3-yl)-1H-pyrrolo[2,3-b]pyridin-2-yl]phenoxy}propan-1-ol

Obtained (60% yield) from 5-(6-methoxy-4-methylpyridin-3-yl)-2-(4,4,5,5-tetramethyl-1,3,2-diaxaborolan-2-yl)-1*H*-pyrrolo[2,3-*b*]pyridine (Preparation 3b) and 3-(3,5-difluoro-4-iodophenoxy)propan-1-ol (Preparation 11) following the experimental procedure as described in Example 1.
LRMS (m/z): 426 (M+1)⁺.
¹H NMR (300 MHz, DMSO-*d*₆) d ppm 1.82 - 1.94 (m, 2 H), 2.21 - 2.27 (m, 3 H), 3.56 (q, *J*=5.87 Hz, 2 H), 3.88 (s, 3 H), 4.14 (t, *J*=6.46 Hz, 2 H), 4.62 (t, *J*=5.28 Hz, 1 H), 6.67 (s, 1 H), 6.82 (s, 1 H), 6.94 (d, *J*=10.57 Hz, 2 H), 7.96 (d, *J*=1.76 Hz, 1 H), 8.03 (s, 1 H), 8.20 (d, *J*=2.35 Hz, 1 H), 11.99 (s, 1 H).

### EXAMPLE 10

### 6-Cyclohexyl-2-(1-methyl-3-(trifluoromethyl)-1H-pyrazol-5-yl)-5H-pyrrolo[2,3-b]pyrazine

Potassium *tert*-butoxide (75 mg, 0.67 mmol) was suspended in 2 ml N-methylpyrrolidone. 3-(Cyctohexytethynyl)-5-(1-methyl-3-(trifluoromethyl)-1*H*-pyrazol-5-yl)pyrazin-2-amine (Preparation 35) dissolved in 2.5 ml *N*-methylpyrrolidone was added and the reaction was stirred at room temperature overnight. The mixture was partitioned between ethyl acetate and water and the organic layer was washed with water, dried over sodium sulphate, filtered and evaporated to dryness. The crude was purified by reverse phase to give 50 mg (44% yield) of the title compound as a solid.
LRMS (m/z): 350 (M+1)⁺.
¹H NMR (300 MHz, DMSO-*d*₆) d ppm 1.34 - 1.62 (m, 6 H), 1.76 - 1.88 (m, 2 H), 1.98 - 2.11 (m, *J*=11.74 Hz, 2 H), 2.74 - 2.89 (m, 1 H), 4.21 (s, 3 H), 5.76 (s, 1 H), 6.45 (s, 1 H), 7.30 (s, 1 H), 8.62 (s, 1 H).

### EXAMPLE 11

### 2-(2-Chloro-6-fluorophenyl)-5-(6-methoxy-4-methylpyridin-3-yl)-1H-pyrrolo[3,2-b]pyridine

Obtained (63% yield) from 5-bromo-2-(2-chloro-6-fluorophenyl)-1*H*-pyrrolo[3,2-*b*]pyridine (Preparation 14c) and (6-methoxy-4-methylpyridin-3-yl)boronic acid following the experimental procedure as described in Preparation 3a and purifying by flash chromatography (0%-10% dichloromethane-methanol).
LRMS (m/z): 368 (M+1)⁺.
¹H NMR (300 MHz, DMSO-*d*₆) d ppm 2.37 (s, 3 H), 3.88 (s, 3 H), 6.79 (d, *J*=4.70 Hz, 2 H), 7.31 (d, *J*=8.80 Hz, 1 H), 7.40 - 7.48 (m, 1 H), 7.54 - 7.58 (m, 2 H), 7.88 (d, *J*=8.22 Hz, 1 H),8.17(s, 1 H), 11.81 (s, 1 H).

### EXAMPLES 12

### 2-(2-Chloro-6-fluorophenyl)-5-[1-methyl-3-(trifluoromethyl)-1H-pyrazol-5-yl]-1H-pyrrolo[2,3-b]pyridine

Obtained (24% yield) from 5-[1-methyl-3-(trifluoromethyl)-1*H*-pyrazol-5-yl]-2-(4,4,5,5-tetramethyl-1,3,2-dioxaborolan-2-yl)-1*H*-pyrrolo[2,3-*b*]pyridine (Preparation 12b) and 1-chloro-3-fluoro-2-iodobenzene following the experimental procedure as described in Example 1, followed by purification by flash chromatography (0%-100%, hexane - ethyl acetate).
LRMS (m/z): 395 (M+1)⁺.
¹H NMR (300 MHz, DMSO-*d*₆) d ppm 3.97 (s, 3 H), 6.74 (d, *J*=1.76 Hz, 1 H), 6.98 (s, 1 H), 7.39 - 7.47 (m, 1 H), 7.51 - 7.62 (m, 2 H), 8.27 (d, *J*=1.76 Hz, 1 H), 8.46 (d, *J*=1.76Hz, 1 H), 12.33 (s, 1 H).

### EXAMPLE 13

### 5-(6-Chloro-2,2-difluoro-1,3-benzodioxol-5-yl)-2-(2-chloro-6-fluorophenyl)-1H-pyrrolo[2,3-b]pyridine

### a) 5-(6-Chloro-2,2-difluoro-1,3-benzodioxol-5-yl)-3-[(2-chloro-6-fluorophenyl) ethynyl]pyridin-2-amine

Obtained (49% yield) from 3-bromo-5-(6-chloro-2,2-difluoro-1,3-benzodioxol-5-yl)pyridin-2-amine (Preparation 13b) and 1-chloro-2-ethynyl-3-fluorobenzene (Preparation 5b) following the experimental procedure as described in Example 5a, followed by flash chromatography (0-100% ethyl acetate in hexane).
LRMS (m/z):437, 439 (M+1)⁺.

### b) N-{5-(6-Chloro-2,2-difluoro-1,3-benzodioxol-5-yl)-3-[(2-chloro-6-fluorophenyl) ethynyl]pyridin-2-yl}-2,2,2-trifluoroacetamide

Obtained (69% yield) from 5-(6-chloro-2,2-difluoro-1,3-benzodioxol-5-yl)-3-[(2-chloro-6-fluorophenyl)ethynyl]pyridin-2-amine (Example 13a) and following the experimental procedure as described in Example 2b.
LRMS (m/z):533(M+1)⁺.

### c) 5-(6-Chloro-2,2-difluoro-1,3-benzodioxol-5-yl)-2-(2-chloro-6-fluorophenyl)-1H-pyrrolo[2,3-b]pyridine

Obtained (47% yield) from *N*-{5-(6-chloro-2,2-difluoro-1,3-benzodioxol-5-yl)-3-[(2-chloro-6-fluorophenyl)ethynyl]pyridin-2-yl}-2,2,2-trifluoroacetamide (Example 13b) following the experimental procedure as described in Example 2c.
LRMS (m/z): 437, 439 (M+1)⁺.
¹H NMR (300 Hz, DMSO-*d*₆) d ppm 6.71 (d, J=1.76 Hz, 1 H), 7.38 - 7.47 (m, 1 H), 7.51 - 7.63 (m, 2 H), 7.68 (s, 1 H), 7.86 (s, 1 H), 8.07 (d, J=2.35 Hz, 1 H), 8.29 (d, J=1.76 Hz, 1H), 12.21 (s, 1 H).

### EXAMPLE 14

### 2-(2-Chloro-6-fluorophenyl)-5-[1-methyl-3-(trifluoromethyl)-1H-pyrazol-5-yl]-1H-pyrrolo[3,2-b]pyridine

Obtained (35% yield) from 5-bromo-2-(2-chloro-6-fluorophenyl)-1H-pyrrolo[3,2-*b*]pyridine (Preparation 14) and 1-methyl-3-(trifluoromethyl)-1*H*-pyrazol-5-ylboronic acid following the experimental procedure as described in Preparation 3a, using sodium carbonate as base and purifying by flash chromatography (0% to 100%, hexane - ethyl acetate).
LRMS (m/z): 395 (M+1)⁺.
¹H NMR (300 Hz, DMSO-*d*₆) d ppm 3.45 (s, 3 H), 6.04 (s, 1 H), 6.41 (s, 1 H), 6.56 - 6.66 (m, 1 H), 6.68 - 6.80 (m, 2 H), 6.84 (d, *J*=8.22 Hz, 1 H), 7.11 (d, *J*=9.39 Hz, 1H), 11.16 (s, 1 H).

### EXAMPLE 15

### 6-Cyclohexyl-2-(6-methoxy-4-methylpyridin-3-yl)-5H-pyrrolo[2,3-b]pyrazine

### a) 3-(Cyclohexylethynyl)-5-(6-methoxy-4-methylpyridin-3-yl)pyrazin-2-amine

Obtained (42% yield) from 3-bromo-5-(6-methoxy-4-methylpyridin-3-yl)pyrazin-2-amine (Preparation 4b) and ethynylcyclohexane following the experimental procedure as described in Example 5a, using thermal conditions instead of microwave irradiation and followed by flash chromatography purification (0%-100% hexane - ethyl acetate).
LRMS (m/z):323(M+1)⁺.

### b) 6-Cyclohexyl-2-(6-methoxy-4-methylpyridin-3-yl)-5H-pyrrolo[2,3-b]pyrazine

Obtained (57% yield) from 3-(cyclohexylethynyl)-5-(6-methoxy-4-methylpyridin-3-yl)pyrazin-2-amine (Preparation 15a) following the experimental procedure as described in Preparation 14c.
LRMS (m/z):323 (M+1)⁺.
¹H NMR (300 MHz, CHLOROFORM-*d*) d ppm 1.28 - 1.58 (m, 6 H), 1.87 - 1.98 (m, 2 H), 2.11 - 2.21 (m, 2 H), 2.38 (s, 3 H), 2.78 - 2.91 (m, 1 H), 3.98 (s, 3 H), 6.45 (s, 1 H), 6.71 (s, 1 H), 8.19 (s, 1 H), 8.22 (s, 1 H), 8.93 (s, 1 H).

### EXAMPLE 16

### 2-Cyclohexyl-5-[1-methyl-3-(trifluoromethyl)-1H-pyrazol-5-yl]-1H-pyrrolo[3,2-b]pyridine

Obtained (15% yield) as a solid from 5-bromo-2-cyclohexyl-1*H*-pyrrolo[3,2-*b*]pyridine (Preparation 15b) and 1-methyl-3-(trifluoromethyl)-1*H*-pyrazol-5-ylboronic acid following the experimental procedure as described in Preparation 3a, using sodium carbonate as base and purifying by flash chromatography (0%-100%, hexane - diethyl ether) and reverse phase chromatography using SP1 Purification System.
LRMS (m/z): 349(M+1)⁺.
¹H NMR (300 MHz, CHLOROFORM-*d*) d ppm 1.25 - 1.54 (m, 6 H), 1.85 - 1.95 (m, J=12.33 Hz, 2 H), 2.07 - 2.19 (m, J=11.74 Hz, 2 H), 2.71 - 2.87 (m, 1 H), 4.27 (s, 3 H), 6.49 (s, 1 H), 6.75 (s, 1 H), 7.32 (d, *J*=8.22 Hz, 1 H), 7.65 (d, *J*=8.22 Hz, 1 H), 8.09 (s, 1 H).

### EXAMPLES 17 2-Cyclohexyl-5-(6-methoxy-4-methylpyridin-3-yl)-1H-pyrrolo[3,2-b]pyridine

Obtained (13% yield) as a solid from 5-bromo-2-cyclohexyl-1*H*-pyrrolo[3,2-*b*]pyridine (Preparation 15b) and 6-methoxy-4-methylpyridin-3-ylboronic acid following the experimental procedure as described in Preparation 3a, followed by flash chromatography (0% to 100%, hexane - ethyl acetate) and reverse phase chromatography using SP1 Purification System.
LRMS (m/z):322(M+1)⁺.
¹H NMR (300 Hz, DMSO-*d*₆) d ppm 1.35 - 1.59 (m, 6 H), 1.77 - 1.87 (m, 2 H), 1.99 - 2.09 (m, *J*=11.74 Hz, 2 H), 2.32 (s, 3 H), 2.70 - 2.81 (m, 1 H), 3.87 (s, 3 H), 6.28 (s, 1 H), 6.75 (s, 1 H), 7.13 (d, *J*=8.80 Hz, 1 H),7.69 (d, *J*=8.22 Hz, 1 H), 8.10 (s, 1 H), 11.22 (s, 1 H).

### EXAMPLE 18

### 2-(2-Chloro-6-fluorophenyl)-5-(1,3-dimethyl-1H-pyrazol-5-yl)-1H-pyrrolo[3,2-b]pyridine

Obtained (41% yield) from 5-bromo-2-(2-chloro-6-fluorophenyl)-1*H*-pyrrolo[3,2-*b*]pyridine (Preparation 14c) and 1,3-dimethyl-1H-pyrazol-5-ylboronic acid following the experimental procedure as described in Preparation 3a, using sodium carbonate as base and purifying by flash chromatography (0% to100%, hexane - ethyl acetate).
LRMS (m/z): 341 (M+1)⁺.
¹H NMR (300 Hz, DMSO-*d*₆) d ppm 2.19 (s, 3 H), 4.12 (s, 3 H), 6.49 (s, 1 H), 6.83 (s, 1 H), 7.41 - 7.49 (m, 1 H), 7.52 (d, *J*=8.22 Hz, 1 H), 7.55 - 7.63 (m, 2 H), 7.88 (d, *J*=9.39 Hz, 1 H), 11.87 (s, 1 H).

### EXAMPLE 19

### 2-(4-Methyl-3-(2-propyl-1H-pyrrolo[3,2-b]pyridin-5-yl)phenyl)oxazole

5-Bromo-2-propyl-1H-pyrrolo[3,2-b]pyridine (Preparation 18b, 66 mg, 0.28 mmol) was treated with 2-(4-methyl-3-(4,4,5,5-tetramethyl-1,3,2-dioxaborolan-2-yl)phenyl)oxazole (Preparation 36, 110 mg, 0.39 mmol), 1,1'-bis(diphenylphosphino)ferrocene]dichloropalladium(II), complex with dichloromethane (11.5 mg, 0.01 mmol) and cesium carbonate (414 mg, 0.83 mmol) according to the method described in Preparation 3a. The crude was purified by flash chromatography (0% to 100%, hexane-ethyl acetate) using SP1 Purification System to obtain 33 mg (37% yield) of the final compound as a beige solid.
LRMS (m/z): 318 (M+1)⁺.
¹H NMR (300 Hz, DMSO-*d*₆) d ppm 0.97 (t, *J*=7.34 Hz, 3 H), 1.66 - 1.84 (m, 2 H), 2.40 (s, 3 H), 2.77 (t, *J*=7.63 Hz, 2 H), 6.34 (s, 1 H), 7.20 (d, *J*=8.22 Hz, 1 H), 7.37 (s, 1 H), 7.46 (d, *J*=7.63 Hz, 1 H), 7.73 (d, *J*=8.22 Hz, 1 H), 7.84 - 7.93 (m, 1 H), 7.99 (s, 1 H), 8.21 (s, 1 H), 11.27 (s, 1 H).

### EXAMPLE 20

### 2-(2-Chloro-6-fluorophenyl)-5-[1-ethyl-3-(trifluoromethyl)-1H-pyrazol-5-yl]-1H-pyrrolo[3,2-b]pyridine

Obtained (24% yield) as a white solid from 5-bromo-2-(2-chloro-6-fluorophenyl)-1*H-*pyrrolo[3,2-*b*]pyridine (Preparation 14c) and [1-ethyl-3-(trifluoromethyl)-1*H*-pyrazol-5-yl]boronic acid (Preparation 8b) following the experimental procedure as described in Preparation 3a, using sodium carbonate as base and purifying by flash chromatography (0% to100%, hexane - ethyl acetate).
LRMS (m/z): 409 (M+1)⁺.
¹H NMR (300 MHz, DMSO-*d*₆) d ppm 1.41 (t, *J*=7.34 Hz, 3 H), 4.76 (q, *J*=7.04 Hz, 2 H), 6.87 (s, 1 H), 7.23 (s, 1 H), 7.41 - 7.51 (m, 1 H), 7.53 - 7.63 (m, 2 H), 7.67 (d, *J*=8.80 Hz, 1 H), 7.96 (d, *J*=8.80 Hz, 1 H), 12.01 (s, 1 H).

### EXAMPLE 21

### 2-(2,6-Difluorophenyl)-5-[1-methyl-3-(trifluoromethyl)-1H-pyrazol-5-yl]-1H-pyrrolo[3,2-b]pyridine

Obtained (15% yield) as a solid from 5-bromo-2-(2,6-difluorophenyl)-1*H*-pyrrolo[3,2-b]pyridine (Preparation 17b) and 1-methyl-3-(trifluoromethyl)-1*H*-pyrazol-5-ylboronic acid following the experimental procedure as described in Preparation 3a, using sodium carbonate as base and purifying by flash chromatography (0% to 100%, hexane- diethyl ether).
LRMS (m/z): 379 (M+1)⁺.
¹H NMR (300 MHz, DMSO-*d*₆) d ppm 4.29 (s, 3 H), 7.02 (s, 1 H), 7.26 (s, 1 H), 7.31 - 7.40 (m, 2 H), 7.52 - 7.62 (m, 1 H), 7.69 (d, *J*=8.80 Hz, 1 H), 7.98 (d, *J*=9.39 Hz, 1 H), 11.90 (s, 1 H).

### EXAMPLE 22

### 5-[1-Methyl-3-(trifluoromethyl)-1H-pyrazol-5-yl]-2-propyl-1H-pyrrolo[3,2-b]pyridine

Obtained (44% yield) as a white solid from 5-bromo-2-propyl-1*H*-pyrrolo[3,2-*b*]pyridine (Preparation 18b) and 1-methyl-3-(trifluoromethyl)-1*H*-pyrazol-5-ylboronic acid following the experimental procedure as described in Preparation 3a, using sodium carbonate as base and purifying by flash chromatography (0% to 100%, hexane - diethyl ether).
LRMS (m/z): 309 (M+1)⁺.
¹H NMR (300 MHz, DMSO-*d*₆) d ppm 0.96 (t, *J*=7.34 Hz, 3 H), 1.65 - 1.82 (m, 2 H), 2.77 (t, *J*=7.63 Hz, 2 H), 4.24 (s, 3 H), 6.39 (s, 1 H), 7.15 (s, 1 H), 7.50 (d, *J*=8.22 Hz, 1 H), 7.76 (d, J=7.63 Hz, 1 H), 11.42 (s, 1 H).

### EXAMPLE 23

### 5-[5-Methyl-3-(trifluoromethyl)-1H-pyrazol-1-yl]-2-propyl-1H-pyrrolo[3,2-b]pyridine

An oven dried resealable Schlenk tube was charged with 5-bromo-2-propyl-1*H-*pyrrolo[3,2-*b*]pyridine (Preparation 18b, 0.1 g, 0.42 mmol), 5-methyl-3-(trifluoromethyl)-1H-pyrazole (0.063 g, 0.42 mmol), potassium phosphate (0.177 g, 0.83 mmol), copper (I) iodide (0.012 g, 0.06 mmol) and dioxane (1.5 ml). The Schlenk tube was subjected to three cycles of evacuation-backfilling with argon, and *N1,N2*-dimethylcyclohexane-1,2-diamine (0.010 ml, 0.06 mmol) was added. After three further cycles of evacuation-backfilling with argon, the Schlenk tube was capped and placed in an oil bath at 130 ºC overnight. The mixture was cooled, filtered on celite and the solvent was removed in vacuo. The residue was purified by flash chromatography (0-100% diethyl ether in hexane) to give the title compound (0.086 g, 65%) as a solid.
LRMS (m/z): 309 (M+1)⁺.
¹H NMR (300 MHz, CHLOROFORM-*d*) d ppm 1.05 (t, *J*=7.34 Hz, 3 H), 1.70 - 1.91 (m, 2 H), 2.63 (s, 3 H), 2.81 (t, *J*=7.63 Hz, 2 H), 6.45 (s, 1 H), 7.27 (s, 1 H), 7.49 (d, *J*=8.22 Hz, 1 H), 7.69 (d, *J*=7.63 Hz, 1 H), 8.13 (s, 1 H).

### EXAMPLE 24

### 2-Cyclopentyl-5-[1-methyl-3-(trifluoromethyl)-1H-pyrazol-5-yl]-1H-pyrrolo[3,2-b]pyridine

### a) 2-(Cyclopentylethynyl)-6-[1-methyl-3-(trifluoromethyl)-1H-pyrazol-5-yl]pyridin-3-amine

Obtained (30% yield) from 2-bromo-6-[1-methyl-3-(trifluoromethyl)-1*H*-pyrazol-5-yl]pyridin-3-amine (Preparation 19b) and ethynylcyclopentane following the experimental procedure as described in Example 5a, using thermal conditions instead of microwave irradiation and tetrahydrofuran as solvent. Crude residue was purified by flash chromatography (0% to 100%, hexane - diethyl ether).
LRMS (m/z): 335 (M+1)⁺.

### b) 2-Cyclopentyl-5-[1-methyl-3-(trifluoromethyl)-1H-pyrazol-5-yl]-1H-pyrrolo[3,2-b]pyridine

Obtained (38%) from 2-(cyclopentylethynyl)-6-[1-methyl-3-(trifluoromethyl)-1*H*-pyrazol-5-yl]pyridin-3-amine (Example 24a) following the experimental procedure as described in Preparation 14c.
LRMS (m/z): 335 (M+1)⁺.
¹H NMR (300 MHz, DMSO-*d*₆) d ppm 1.64 - 1.83 (m, 6 H), 2.02 - 2.15 (m, 2 H), 3.16 - 3.29 (m, 1 H), 4.24 (s, 3 H), 6.40 (s, 1 H), 7.14 (s, 1 H), 7.50 (d, *J*=8.80 Hz, 1 H), 7.75 (d, *J*=8.22 Hz, 1 H), 11.41 (s, 1 H).

### EXAMPLE 25

### 2-Cyclohexyl-5-[5-methyl-3-(trifluoromethyl)-1H-pyrazol-1-yl]-1H-pyrrolo[3,2-b]pyridine

### a) 2-(Cyclohexylethynyl)-6-[5-methyl-3-(trifluoromethyl)-1H-pyrazol-1-yl]pyridin-3-amine

Obtained (60% yield) from 2-bromo-6-[5-methyl-3-(trifluoromethyl)-1*H*-pyrazol-1-yl]pyridin-3-amine (Preparation 20) and ethynylcyclohexane following the experimental procedure as described in Example 5a, using thermal conditions instead of microwave irradiation and tetrahydrofuran as solvent. Crude residue was purified by flash chromatography (0% to 100%, hexane - diethyl ether).
LRMS (m/z): 349 (M+1)⁺.

### b) 2-Cyclohexyl-5-[5-methyl-3-(trifluoromethyl)-1H-pyrazol-1-yl]-1H-pyrrolo[3,2-b]pyridine

Obtained (61% yield) as a white solid from 2-(cyclohexylethynyl)-6-[5-methyl-3-(trifluoromethyl)-1*H*-pyrazol-1-yl]pyridin-3-amine (Example 25a) following the experimental procedure as described in Preparation 14c.
LRMS (m/z): 349 (M+1)⁺.
¹H NMR (300 MHz, CHLOROFORM-*d*) d ppm 1.24 - 1.53 (m, 6 H), 1.84 - 1.95 (m, *J*=11.74 Hz, 2 H), 2.03 - 2.19 (m, *J*=11.15 Hz, 2 H), 2.63 (s, 3 H), 2.72 - 2.86 (m, 1 H), 6.44 (s, 2 H), 7.49 (m, 1 H), 7.70 (d, *J*=7.63 Hz, 1 H), 8.13 (s, 1 H),

### EXAMPLE 26

### 2-(2-Chlorophenyl)-5-[1-methyl-3-(trifluoromethyl)-1H-pyrazol-5-yl]-1H-pyrrolo[3,2-b]pyridine

Obtained (36% yield) as a white solid from 2-iodo-5-[1-methyl-3-(trifluoromethyl)-1*H-*pyrazol-5-yl]-1-(phenylsulfonyl)-1*H*-pyrrolo[3,2-*b*]pyridine (Preparation 22b) and (2-chlorophenyl)boronic acid following the experimental procedure as described in Preparation 3a, followed by treatment with 8N sodium hydroxide for 1h at room temperature. Then ethyl acetate was added and organic layer was washed with water and brine, dried (MgSO₄) filtered and concentrated. Purification of the crude residue by normal phase chromatography (0% to 100%, hexane - diethyl ether) afforded the title compound.
LRMS (m/z): 377 (M+1)⁺.
¹H NMR (300 MHz, DMSO-*d*₆) d ppm 4.29 (s, 3 H), 7.06 (s, 1 H), 7.24 (s, 1 H), 7.39 - 7.60 (m, 2 H), 7.67 (d, *J*=8.22 Hz, 2 H), 7.79 (d, *J*=9.39 Hz, 1 H), 7.93 (d, *J*=9.39 Hz, 1 H), 11.97 (br.s, 1 H)

### EXAMPLE 27

### 1-(5-(1-Methyl-3-(trifluoromethyl)-1H-pyrazol-5-yl)-1H-pyrrolo[3,2-b]pyridin-2-yl)cyclohexanol

1-((3-Amino-6-(1-methyl-3-(trifluoromethyl)-1H-pyrazol-5-yl)pyridin-2-yl)ethynyl)-cyclohexanol (Preparation 37) was treated with potassium *tert*-butoxide and NMP as a solvent according to the method described in Example 10. The crude was precipitate with water to give (40% yield) the title compound as a beige solid.
LRMS (m/z): 365 (M+1)⁺.
¹H NMR (300 MHz, CHLOROFORM-*d*) d ppm 1.69 - 1.80 (m, 5 H), 1.92 - 2.02 (m, 5 H), 4.24 - 4.29 (m, 3 H), 6.56 (d, *J*=2.93 Hz, 1 H), 6.77 (s, 1 H), 7.36 (d, *J*=8.22 Hz, 1 H), 7.71 (d, *J*=8.22 Hz, 1 H), 8.72 (s, 1 H).

### EXAMPLE 28

### 5-(1-Methyl-3-(trifluoromethyl)-1H-pyrazol-5-yl)-2-(4-methylpyridin-3-yl)-1H-pyrrolo[3,2-b]pyridine

Obtained (28% yield) as a brown solid from 2-iodo-5-[1-methyl-3-(trifluoromethyl)-1*H-*pyrazo)-5-yl]-1-(phenylsulfonyl)-1*H*-pyrrolo[3,2-*b*]pyridine (Preparation 22b) and 4-methyl-3-(4,4,5,5-tetramethyl-1,3,2-dioxaborolan-2-yl)pyridine following the experimental procedure as described in Preparation 3a, followed by treatment with 8N sodium hydroxide for 1h at room temperature. Then ethyl acetate was added and organic layer was washed with water and brine, dried (MgSO₄), filtered and concentrated. Purification of the crude residue by normal phase chromatography (0% to 10%, dichloromethane-methanol) afforded the title compound.
LRMS (m/z): 358 (M+1)⁺.
¹H NMR (300 MHz, DMSO-*d*₆) d ppm 2.53 (s, 3 H), 4.30 (s, 3 H), 6.96 (s, 1 H), 7.24 (s, 1 H), 7.43 (d, J=4.70 Hz, 1 H), 7.67 (d, J=8.22 Hz, 1 H), 7.93 (d, J=8.22 Hz, 1 H), 8.50 (d, J=4.70 Hz, 1 H), 8.76 (s, 1 H), 11.97 (s, 1 H).

### EXAMPLE 29

### 2-(2,6-Difluorophenyl)-5-(5-methyl-3-(trifluoromethyl)-1H-pyrazol-1-yl)-1H-pyrrolo[3,2-b]pyridine

2-((2,6-difluorophenyl)ethynyl)-6-(5-methyl-3-(trifluoromethyl)-1*H*-pyrazol-1-yl)pyridin-3-amine (Preparation 38) was treated with potassium *tert*-butoxide and *N-*methylpyrrolidone as a solvent according to the method described in Example 10. The crude was purified by flash chromatography using SP1 Purification System (0% to 50%, hexane-diethyl ether) to give (40% yield) the title compound as a yellow solid.
LRMS (m/z): 379 (M+1)⁺.
¹H NMR (300 MHz, DMSO-*d*₆) d ppm 2.62 (s, 3 H), 6.80 (s, 1 H), 6.99 (s, 1 H), 7.29 - 7.42 (m, 2 H), 7.52 - 7.65 (m, *J*=8.51, 8.51 Hz, 2 H), 8.08 (d, *J*=7.63 Hz, 1 H), 12.01 (s, 1 H).

### EXAMPLE 30

### 2-Cyclohexyl-5-[5-(difluoromethoxy)-3-(trifluoromethyl)-1H-pyrazol-1-yl]-1H-pyrrolo[3,2-b]pyridine

### a) 2-(Cyclohexylethynyl)-6-[5-(difluoromethoxy)-3-(trifluoromethyl)-1H-pyrazol-1-yl]pyridin-3-amine

Obtained (67% yield) from 2-bromo-6-[5-(difluoromethoxy)-3-(trifluoromethyl)-1*H-*pyrazol-1-yl]pyridin-3-amine (Preparation 23d) and ethynylcyclohexane following the experimental procedure as described in Preparation 5a, followed by flash chromatography (0% to 100%, hexane - diethyl ether).
LRMS (m/z): 401 (M+1)⁺.

### b) 2-Cyclohexyl-5-[5-(difluoromethoxy)-3-(trifluoromethyl)-1H-pyrazol-1-yl]-1H-pyrrolo[3,2-b]pyridine

Obtained (53% yield) as a solid from 2-(cyclohexylethynyl)-6-[5-(difluoromethoxy)-3-(trifluoromethyl)-1*H*-pyrazol-1-yl]pyridin-3-amine (Example 30a) following the experimental procedure as described in Preparation 14c, obtaining the desired product by precipitation in water.
LRMS (m/z): 401 (M+1)⁺.
¹H NMR (300 MHz, CHLOROFORM-*d*) d ppm 1.23 - 1.53 (m, 6 H), 1.83 - 1.93 (m, *J*=12.33 Hz, 2 H), 2.06 - 2.17 (m, *J*=11.74 Hz, 2 H), 2.70 - 2.88 (m, 1 H), 6.41 (s, 1 H), 6.45 - 6.51 (m, *J*=2.35 Hz, 1 H), 7.38 (d, *J*=8.80 Hz, 1 H), 7.72 (d, *J*=7.63 Hz, 1 H), 8.16(s, 1 H).

### EXAMPLE 31

### 2-(1,4-Dioxaspiro[4.5]dec-7-en-8-yl)-5-[1-methyl-3-(trifluoromethyl)-1H-pyrazol-5-yl]-1H-pyrrolo[3,2-b]pyridine

5-(1-Methyl-3-(trifluoromethyl)-1*H*-pyrazol-5-yl)-1-(phenylsulfonyl)-2-(1,4-dioxaspiro[4.5]dec-7-en-8-yl)-1*H*-pyrrolo[3,2-*b*]pyridine (Preparation 31) was treated with sodium hydroxide (8N, 92 ml, 736 mmol) and the reaction was stirred at room temperature for 2 h. The mixture was diluted with ethyl acetate and the organic layer was washed with water and saturated sodium bicarbonate solution. Then it was dried over sodium sulphate, filtered and evaporated under reduced pressure to obtain (79% yield) the title compound as a yellow solid.
LRMS (m/z): 405 (M+1)⁺.
¹H NMR (300 MHz, CHLOROFORM-*d*) d ppm 1.98 (m, 2 H), 2.55 (m, 2 H), 2.77 (m, 2 H), 4.05 (s, 4 H), 4.28 (s, 3 H), 6.08 - 6.24 (m, 1 H), 6.71 (s, 1 H), 6.77 (s, 1 H), 7.36 (d, *J*=8.80 Hz, 1 H), 7.66 (d, *J*=8.80 Hz, 1 H), 8.26 (br.s, 1 H)

### EXAMPLE 32

### tert-Butyl 4-(5-(5-methyl-3-(trifluoromethyl)-1H-pyrazol-1-yl)-1H-pyrrolo[3,2-b]pyridin-2-yl)piperidine-1-carboxylate

*tert*-Butyl 4-((3-amino-6-(5-methyl-3-(trifluoromethyl)-1*H*-pyrazol-1-yl)pyridin-2-yl)-ethynyl)piperidine-1-carboxylate (Preparation 39) was treated with potassium *tert-*butoxide and *N*-methylpyrrolidone as a solvent according to the method described in Example 10. The crude was purified by flash chromatography using SP1 Purification System (0% to 70%, hexane-diethyl ether) to give (50% yield) the title compound as a yellow solid.
LRMS (m/z): 450(M+1)⁺.
¹H NMR (300 MHz, CHLOROFORM-*d*) d ppm 1.63 - 1.82 (m, 2 H), 2.05 - 2.12 (m, 2 H), 2.64 (s, 3 H), 2.84 - 3.01 (m, 4 H), 4.07 - 4.18 (m, 1 H), 6.47 (d, *J*=5.87 Hz, 2 H), 7.54 (d, *J*=8.22 Hz, 1 H), 7.73 (d, *J*=8.80 Hz, 1 H), 8.19 (s, 1 H).

### EXAMPLE 33

### 2-{5-[1-Methyl-3-(trifluoromethyl)-1H-pyrazol-5-yl]-1H-pyrrolo[3,2-b]pyridin-2-yl}benzonitrile

Obtained (28% yield) as a white solid from 2-iodo-5-[1-methyl-3-(trifluoromethyl)-1*H-*pyrazol-5-yl]-1-(phenylsulfonyl)-1*H*-pyrrolo[3,2-*b*]pyridine (Preparation 22b) and 2-cyanophenylboronic acid following the experimental procedure as described in Example 26.
LRMS (m/z): 368 (M+1)⁺.
¹H NMR (300 MHz, DMSO-*d*₆) d ppm 4.31 (s, 3 H), 7.28 (s, 2 H), 7.56 - 7.83 (m, 2 H), 7.81 - 8.17 (m, 4 H), 12.23 (br.s, 1 H)

### EXAMPLE 34

### 4-(5-(1-Methyl-3-(trifluoromethyl)-1H-pyrazol-5-yl)-1H-pyrrolo[3,2-b]pyridin-2-yl)cyclohexanol

4-(5-(1ethyl-3-(trifluoromethyl)-1*H*-pyrazol-5-yl)-1*H*-pyrrolo[3,2-*b*]pyridin-2-yl)cyclohexanone (Preparation 33, 35 mg, 0.10 mmol) was dissolved in 1 ml methanol. Sodium borohydride (7 mg, 0.19 mmol) was added and the reaction was stirred at room temperature for 30 min. The solvent was evaporated and the residue was partitioned between ethyl acetate and water. The organic layer was dried over sodium sulphate, filtered and concentrated to dryness. The crude was purified using SP1 Purification System (0% to 5%, dichloromethane-methanol) to give 12 mg (34% yield) of the title compound as a beige solid.
LRMS (m/z): 365 (M+1)⁺.
¹H NMR (300 MHz, DMSO-*d*₆) d ppm 1.22 - 1.39 (m, 2 H), 1.46 - 1.62 (m, 2 H), 1.90 - 2.10 (m, 4 H), 2.42 - 2.46 (m, 1 H), 2.55 - 2.59 (m, 1 H), 4.23 (s, 3 H), 6.36 (s, 1 H), 7.13 (s, 1 H), 7.49 (d, J=8.80 Hz, 2 H), 7.75 (d, J=7.63 Hz, 1 H).

### EXAMPLE 35

### 2-(2-Fluorophenyl)-5-[1-methyl-3-(trifluoromethyl)-1H-pyrazol-5-yl]-1H-pyrrolo[3,-b]pyridine

An oven dried resealable Schlenk tube was charged with [5-[1-methyl-3-(trifluoromethyl)-1*H*-pyrazol-5-yl]-1-(phenylsulfonyl)-1*H*-pyrrolo[3,2-*b*]pyridin-2-yl]boronic acid (Preparation 24, 0.030 g, 0.07 mmol), 1-fluoro-2-iodobenzene (0.022 g, 0.10 mmol), potassium phosphate (0.028 g, 0.13 mmol) and dioxane (2 ml). The Schlenk tube was subjected to three cycles of evacuation-backfilling with argon, and tris(dibenzylideneacetone)dipalladium(0) (0.002 g, 0.002 mmol) and 2-dicyclohexylphosphino-2',6'-dimethoxybiphenyl (0.002 g, 0.005 mmol) were added. After three further cycles of evacuation-backfilling with argon, the Schlenk tube was capped and placed in an oil bath at 110 ºC overnight. The mixture was cooled, the slurry was filtered through celite and the solvent was removed in vacuo. Ethanol (2 ml) and 8N sodium hydroxide solution (0.5 ml) were and the mixture was stirred at room temperature for 1h. The crude was partitioned between ethyl acetate and water, organic layer was washed with brine, dried (MgSO₄), filtered and concentrated. The residue was purified by flash chromatography (0% to 100%, hexane - diethyl ether) to give the title compound (0.006 g, 24%) as a white solid.
LRMS (m/z): 361 (M+1)⁺.
¹H NMR (300 MHz, CHLOROFORM-*d*) d ppm 4.32 (s, 3 H), 6.81 (s, 1 H), 7.16 (s, 1 H), 7.18 - 7.49 (m, 4 H), 7.71 - 7.94 (m, 2 H), 9.09 (br.s, 1 H)

### EXAMPLE 36

### (2-{5-[1-Methyl-3-(trifluoromethyl)-1H-pyrazol-5-yl]-1H-pyrrolo[3,2-b]pyridin-2-yl}phenyl)methanol

Obtained (47% yield) from 2-iodo-5-[1-methyl-3-(trifluoromethyl)-1*H*-pyrazol-5-yl]-1-(phenylsulfonyl)-1*H*-pyrrolo[3,2-*b*]pyridine (Preparation 22b) and 2-(hydroxymethyl)phenylboronic acid following the experimental procedure as described in Example 26.
LRMS (m/z): 373 (M+1)⁺.
¹H NMR (300 MHz, CHLOROFORM-*d*) d ppm 4.32 (s, 3 H), 4.80 (s, 2 H), 5.30 (s, 1 H), 6.80 (s, 1 H), 6.99 (s, 1 H), 7.37 - 7.46 (m, 4 H), 7.48 - 7.55 (m, 1 H), 7.77 - 7.87 (m, *J*=12.33, 7.63 Hz, 2 H).

### EXAMPLE 37

### 2-(3,5-Difluoro-4-(5-(1-methyl-3-(trifluoromethyl)-1H-pyrazol-5-yl)-1H-pyrrolo[3,2-b]pyridin-2-yl)phenoxy)ethanol

5-(1-Methyl-3-(trifluoromethyl)-1*H*-pyrazol-5-yl)-2-(4,4,5,5-tetramethyl-1,3,2-dioxaborolan-2-yl)-1*H*-pyrrolo[3,2-*b*]pyridine (Preparation 30) were treated with 2-(3,5-difluoro-4-iodophenoxy)ethanol (Preparation 34), cesium carbonate and 1,1'-bis(diphenylphosphino)ferrocene-palladium(II) dichloride dichloromethane complex according to the method described in Preparation 3a. The crude was purified by reverse phase using SP1 Purification System to give (3 % yield) the title compound as a white solid.
LRMS (m/z): 439 (M+1)⁺.
¹H NMR (300 MHz, CHLOROFORM-*d*) d ppm 3.95 - 4.28 (m, 4 H), 4.32 (s, 3 H), 6.53 - 6.97 (m, 3 H), 7.32 - 7.63 (m, 2 H), 7.86 - 8.00 (m, 1 H), 9.02 (br. s., 1 H).

### EXAMPLE 38

### 3-(3,5-Difluoro-4-(5-(1-methyl-3-(trifluoromethyl)-1H-pyrazol-5-yl)-1H-pyrrolo[3,2-b]pyridin-2-yl)phenoxy)propan-1-ol

5-(1-Methyl-3-(trifluoromethyl)-1*H*-pyrazol-5-yl)-2-(4,4,5,5-tetramethyl-1,3,2-dioxaborolan-2-yl)-1*H*-pyrrolo[3,2-*b*]pyridine (Preparation 30) were treated with 3-(3,5-difluoro-4-iodophenoxy)propan-1-ol (Preparation 11), cesium carbonate and 1,1'-bis(diphenylphosphino)ferrocene-palladium(II) dichloride dichloromethane complex according to the method described in Preparation 3a. The crude was purified using SP1 Purification System (0% to 10%, dichloromethane-methanol) to give (11 % yield) the title compound as a brown solid.
LRMS (m/z): 453 (M+1)⁺.
¹H NMR (300 MHz, DMSO-*d*₆) d ppm 3.50 - 3.63 (m, 2 H), 4.16 (t, *J*=6.46 Hz, 2 H), 4.28 (s, 3 H), 4.60 (t, *J*=5.28 Hz, 2 H), 6.90 (s, 1 H), 6.93 - 7.06 (m, 2 H), 7.22 (s, 1 H), 7.65 (d, *J*=8.22 Hz, 1 H), 7.94 (d, *J*=8.22 Hz, 1 H), 11.74 (br. s., 1 H).

### EXAMPLE 39

### 4-((4-(2-(2-Chloro-6-fluorophenyl)-1H-pyrrolo[2,3-b]pyridin-5-yl)-3-methylphenyl)sulfonyl)morpholine

4-((3-Methyl-4-(2-(4,4,5,5-tetramethyl-1,3,2-dioxaborolan-2-yl)-1*H*-pyrrolo[2,3-*b*]pyridin-5-yl)phenyl)sulfonyl)morpholine (Preparation 40b) was treated with 1-chloro-3-fluoro-2-iodobenzene, cesium carbonate and 1,1'-bis(diphenylphosphino)ferrocene-palladium(II) dichloride dichloromethane complex according to the method described in Preparation 3a. The crude was purified using SP1 Purification System (0% to 10%, dichloromethane-methanol) to give (25 % yield) the title compound as a yellow solid.
LRMS (m/z): 486 (M+1)⁺.
¹H NMR (300 MHz, DMSO-*d*₆) d ppm 2.42 (s, 3 H), 2.90 - 2.97 (m, 4 H), 3.63 - 3.71 (m, 4 H), 6.70 (d, *J*=1.76 Hz, 1 H), 7.39 - 7.47 (m, 1 H), 7.52 - 7.62 (m, 3 H), 7.63 - 7.74 (m, 2 H), 8.09 (d, *J*=2.35 Hz, 1 H), 8.30 (d, *J*=2.35 Hz, 1 H), 12.17 (s, 1 H).

### EXAMPLE 40

### 2-(2-Methylphenyl)-5-[1-methyl-3-(trifluoromethyl)-1H-pyrazol-5-yl]-1H-pyrrolo[3,2-b]pyridine

Obtained (37% yield) from 2-iodo-5-[1-methyl-3-(trifluoromethyl)-1*H*-pyrazol-5-yl]-1-(phenylsulfonyl)-1*H*-pyrrolo[3,2-*b*]pyridine (Preparation 22b) and *o*-tolylboronic acid following the experimental procedure as described in Example 26, followed by reverse phase chromatography using SP1 Purification System.
LRMS (m/z): 357 (M+1)⁺.
¹H NMR (300 MHz, DMSO-*d*₆) d ppm 2.50 (s, 3H), 4.29 (s, 3 H), 6.82 (s, 1 H), 7.20 (s, 1 H), 7.33 - 7.41 (m, 4 H), 7.58 (d, *J*=3.52 Hz, 1 H), 7.62 (d, *J*=8.22 Hz, 1 H), 7.88 (d, *J*=7.63 Hz, 1 H), 11.78 (s, 1 H).

### EXAMPLE 41

### 2-(1,4-Dioxaspiro[4.5]dec-8-yl)-5-[1-methyl-3-(trifluoromethyl)-1H-pyrazol-5-yl]-1 H-pyrrolo[3,2-b]pyridine

Obtained (79% yield) as a white solid from 2-(1,4-dioxaspiro[4.5]dec-7-en-8-yl)-5-[1-methyl-3-(trifluoromethyl)-1*H*-pyrazol-5-yl]-1*H*-pyrrolo[3,2-*b*]pyridine (Example 31) following the experimental procedure as described in Example 46, using ethyl acetate as solvent and followed by purification by flash chromatography (0-100% diethyl ether in hexane).
LRMS (m/z): 407 (M+1)⁺.
¹H NMR (300 MHz, DMSO-*d*₆) d ppm 1.53 - 1.87 (m, 6 H), 1.93 - 2.12 (m, 2 H), 2.80 - 2.94 (m, 1 H), 3.90 (s, 4 H), 4.23 (s, 3 H), 6.38 (s, 1 H), 7.13 (s, 1 H), 7.50 (d, *J*=8.22 Hz, 1 H), 7.75 (d, *J*=8.22 Hz, 1 H), 11.44 (br.s, 1 H)

### EXAMPLE 42

### 2-(3,6-Dihydro-2H-pyran-4-yl)-5-[1-methyl-3-(trifluoromethyl)-1H-pyrazol-5-yl]-1H-pyrrolo[3,2-b]pyridine

Obtained (44% yield) as a white solid from 2-iodo-5-[1-methyl-3-(trifluoromethyl)-1*H-*pyrazol-5-yl]-1-(phenylsulfonyl)-1*H*-pyrrolo[3,2-*b*]pyridine (Preparation 22b) and 2-(3,6-dihydro-2H-pyran-4-yl)-4,4,5,5-tetramethyl-1,3,2-dioxaborolane following the experimental procedure as described in Example 26.
LRMS (m/z): 349 (M+1)⁺.
¹H NMR (300 MHz, CHLOROFORM-*d*) d ppm 2.63 (br. s., 2 H), 3.99 (t, J=5.28 Hz, 2 H), 4.29 (s, 3 H), 4.40 (t, *J*=2.93 Hz, 2 H), 6.24 (s, 1 H), 6.72 (s, 1 H), 6.78 (s, 1 H), 7.38 (d, *J*=8.22 Hz, 1 H), 7.68 (d, *J*=8.22 Hz, 1 H), 8.30 (br.s, 1 H).

### EXAMPLE 43

### 4-{5-[1-Methyl-3-(trifluoromethyl)-1H-pyrazol-5-yl]-1H-pyrrolo[3,2-b]pyridin-2-yl}cyclohexanone

A solution of 2-(1,4-dioxaspiro[4.5]dec-8-yl)-5-[1-methyl-3-(trifluoromethyl)-1*H*-pyrazol-5-yl]-1*H*-pyrrolo[3,2-*b*]pyridine (Example 41, 0.163 g, 0.40 mmol) and *p*-toluenesulfonic acid (0.076 g, 0.40 mmol) in a mixture of acetone/water 1:1 (6 ml) was heated at 80°C for 90 min. Acetone was removed in vacuo and sodium bicarbonate was added until pH 8. The mixture was extracted with ethyl acetate, organic layer was dried (MgSO4), filtered and concentrated. Purification of the crude residue by flash chromatography afforded the title compound as a yellow solid (0.059 g, 40%).
LRMS (m/z): 363 (M+1)⁺.
¹H NMR (300 MHz, DMSO-*d*₆) d ppm 1.85 - 2.11 (m, 2 H), 2.21 - 2.41 (m, 4 H), 2.44 - 2.80 (m, 5 H), 4.24 (s, 1 H), 6.47 (s, 1 H), 7.15 (s, 1 H), 7.53 (d, *J*=8.22 Hz, 1 H), 7.79 (d, *J*=7.63 Hz, 1 H), 11.51 (br.s, 1 H)

### EXAMPLE 44

### 4-(2-(2-Chloro-6-fluorophenyl)-1H-pyrrolo[2,3-b]pyridin-5-yl)-N,N,3-trimethylbenzenesulfonamide

*N,N*,3-Trimethyl-4-(2-(4,4,5,5-tetramethyl-1,3,2-dioxaborolan-2-yl)-1*H*-pyrrolo[2,3-*b*]pyridin-5-yl)benzenesulfonamide (Preparation 41b) was treated with 1-chloro-3-fluoro-2-iodobenzene, cesium carbonate and 1,1'-bis(diphenylphosphino)ferrocene-palladium(II) dichloride dichloromethane complex, dioxane and water as a solvents according to the method described in Preparation 3a. The crude was purified using SP1 Purification System (0% to 10%, dichloromethane-methanol) to give (18 % yield) the title compound as a yellow solid.
LRMS (m/z): 444 (M+1)⁺.
¹H NMR (300 Hz, DMSO-*d*₆) d ppm 2.41 (s, 3 H), 2.68 (s, 6 H), 6.69 (d, *J*=2.35 Hz, 1 H), 7.38 - 7.46 (m, 1 H), 7.50 - 7.61 (m, 3 H), 7.63 - 7.69 (m, 1 H), 7.71 - 7.74 (m, 1 H), 8.07 (d, *J*=2.35 Hz, 1 H), 8.29 (d, *J*=1.76 Hz, 1 H), 12.16 (s, 1 H).

### EXAMPLE 45

### 2-(2-Methoxyphenyl)-5-[1-methyl-3-(trifluoromethyl)-1H-pyrazol-5-yl]-1H-pyrrolo[3,2-b]pyridine

Obtained (50% yield) from 2-iodo-5-[1-methyl-3-(trifluoromethyl)-1*H*-pyrazol-5-yl]-1-(phenylsulfonyl)-1*H*-pyrrolo[3,2-*b*]pyridine (Preparation 22b) and 2-methoxyphenylboronic acid following the experimental procedure as described in Example 26.
LRMS (m/z): 373 (M+1)⁺.
¹H NMR (300 MHz, CHLOROFORM-*d*) d ppm 4.07 (s, 3 H), 4.31 (s, 3 H), 6.79 (s, 1 H), 7.01 - 7.19 (m, 3 H), 7.31 - 7.47 (m, 2 H), 7.76 (d, *J*=9.39 Hz, 1 H), 7.91 (d, *J*=6.46 Hz, 1 H), 9.90 (br.s., 1 H)

### EXAMPLE 46

### 5-[1-Methyl-3-(trifluoromethyl)-1H-pyrazol-5-yl]-2-(tetrahydro-2H-pyran-4-yl)-1H-pyrrolo[3,2-b]pyridine

Palladium on carbon (10%, 0.005 g, 0.05 mmol) was added to a solution of 2-(3,6-dihydro-2*H*-pyran-4-yl)-5-[1-methyl-3-(trifluoromethyl)-1*H*-pyrazol-5-yl]-1*H*-pyrrolo[3,2-b]pyridine (Example 42, 0.025 g, 0.07 mmol) in ethanol (3 ml) and the mixture was stirred under hydrogen at room temperature overnight. The suspension was filtered through celite and solvent was removed in vacuo to give 0.022g (87% yield) of the title compound as a white solid.
LRMS (m/z): 351 (M+1)⁺.
¹H NMR (300 MHz, CHLOROFORM-*d*) d ppm 1.81 - 2.09 (m, 4 H), 2.93 - 3.16 (m, 1 H), 3.50 - 3.67 (m, 2 H), 4.06 - 4.18 (m, 2 H), 4.27 (s, 3 H), 6.47 - 6.61 (m, 1 H), 6.76 (s, 1 H), 7.31 - 7.42 (m, 1 H), 7.67 (d, *J*=9.39 Hz, 1 H), 8.11 (br. s., 1 H).

### EXAMPLE 47

### N,N,3-Trimethyl-4-(5-(1-methyl-3-(trifluoromethyl)-1H-pyrazol-5-yl)-1H-pyrrolo[3,2-b]pyridin-2-yl)benzenesulfonamide

2-Iodo-5-(1-methyl-3-(trifluoromethyl)-1*H*-pyrazol-5-yl)-1-(methylsulfonyl)-1H-pyrrolo[3,2-*b*]pyridine (Preparation 22b) was treated with (4-(*N,N-*dimethylsulfamoyl)-2-methylphenyl)boronic acid, 2M cesium carbonate and 1,1'-bis(diphenylphosphino)ferrocene-palladium(II) dichloride dichloromethane complex. The reaction was heated at 100ºC overnight according to the method described in Preparation 3a. The crude was purified by flash chrmotagraphy (0% to 70%, hexane-ehtl acetate) using SP1 Purification System to obtain (32% yield) the title compound.
LRMS (m/z): 464 (M+1)⁺.
¹H NMR (300 MHz, CHLOROFORM-*d*) d ppm 2.64 (s, 3 H), 2.80 (s, 6 H), 4.33 (s, 3 H), 6.83 (s, 1 H), 6.97 (s, 1 H), 7.49 (d, *J*=8.22 Hz, 1 H), 7.64 - 7.73 (m, 2 H), 7.76 (s, 1 H), 7.84 (d, *J*=8.22 Hz, 1 H), 8.58 (s, 1 H).

### EXAMPLE 48

### 5-Fluoro-2-{5-[1-methyl-3-(trifluoromethyl)-1H-pyrazol-5-yl]-1H-pyrrolo[3,2-b]pyridin-2-yl}benzonitrile

Obtained (19% yield) from 2-iodo-5-[1-methyl-3-(trifluoromethyl)-1*H*-pyrazol-5-yl]-1-(phenylsulfonyl)-1*H*-pyrrolo[3,2-*b*]pyridine (Preparation 22b) and 2-cyano-4-fluorophenylboronic acid following the experimental procedure as described in Example 35.
LRMS (m/z): 386 (M+1)⁺.
¹H NMR (400 MHz, DMSO-*d*₆) d ppm 4.30 (s, 3 H), 7.24 (s, 1 H), 7.28 (s, 1 H), 7.72 (d, *J*=8.21 Hz, 1 H), 7.79 - 7.89 (m, 1 H), 7.93 - 8.04 (m, 2 H), 8.10 (dd, *J*=8.60, 2.74 Hz, 1 H)

### EXAMPLE 49

### 2-(4,4-Difluorocyclohexyl)-5-[1-methyl-3-(trifluoromethyl)-1H-pyrazol-5-yl]-1H-pyrrolo[3,2-b]pyridine

To a suspension of 4-{5-[1-methyl-3-(trifluoromethyl)-1*H*-pyrazol-5-yl]-1*H*-pyrrolo[3,2-*b*]pyridin-2-yl}cyclohexanone (Example 43, 0.047 g, 0.13 mmol) in dichloromethane (1.5 ml) diethylaminosulfur trifluoride (DAST) (0.051 ml, 0.39 mmol) was added dropwise under argon and the mixture was stirred at room temperature overnight. The mixture was added dropwise onto ice-water and stirred for 20 min. The mixture was then neutralized with aqueous sodium bicarbonate, extracted with dichloromethane and organic layer was concentrated. Purification of the crude residue by flash chromatography (30-70% diethyl ether in hexane) afforded the desired product as a yellow solid (0.009 g, 16%).
LRMS (m/z): 385 (M+1)⁺.
¹H NMR (400 MHz, DMSO-*d*₆) d ppm 1.80 (s, 2 H), 1.89 - 2.24 (m, 6 H), 3.00 (s, 1 H), 4.24 (s, 3 H), 6.44 (s, 1 H), 7.16 (s, 1 H), 7.53 (d, *J*=8.21 Hz, 1 H), 7.78 (d, *J*=8.21 Hz, 1 H), 11.53 (br.s., 1 H).

### EXAMPLE 50

### 5-[1-Methyl-3-(trifluoromethyl)-1H-pyrazol-5-yl]-2-phenyl-1H-pyrrolo[3,2-b]pyridine

Obtained (39%) as a white solid from 2-iodo-5-[1-methyl-3-(trifluoromethyl)-1*H*-pyrazol-5-yl]-1-(phenylsulfonyl)-1*H*-pyrrolo[3,2-*b*]pyridine (Preparation 22b) and phenylboronic acid following the experimental procedure as described in Example 35, with toluene as solvent.
LRMS (m/z): 343 (M+1)⁺.
¹H NMR (400 MHz, DMSO-*d*₆) d ppm 4.41 (s, 3 H), 7.21 (dd, *J*=11.33, 2.93 Hz, 2 H), 7.35 - 7.47 (m, 1 H), 7.53 (dd, *J*=7.42, 4.69 Hz, 2 H), 7.62 (dd, *J*=8.40, 3.71 Hz, 1 H), 7.89 (dd, *J*=8.40, 2.93 Hz, 1 H), 7.94 - 8.06 (m, 2 H), 12.06 (br. s., 1 H)

### EXAMPLE_51

### 2-(4-Butoxy-2-methylphenyl)-5-[1-methyl-3-(trifluoromethyl)-1H-pyrazol-5-yl]-1H-pyrrolo[3,2-b]pyridine

Obtained (47% yield) as a white solid from 2-iodo-5-[1-methyl-3-(trifluoromethyl)-1*H-*pyrazol-5-yl]-1-(phenylsulfonyl)-1*H*-pyrrolo[3,2-*b*]pyridine (Preparation 22b) and 4-butoxy-2-methylphenylboronic acid following the experimental procedure as described in Example 26.
LRMS (m/z): 429 (M+1)⁺.
¹H NMR (400 MHz, CHLOROFORM-*d*) d ppm 1.00 (t, J=7.42 Hz, 3 H), 1.47 - 1.55 (m, 2 H), 1.75 - 1.88 (m, 2 H), 2.51 (s, 3 H), 4.02 (t, *J*=6.64 Hz, 2 H), 4.31 (s, 3 H), 6.79 (s, 2 H), 6.83 - 6.91 (m, 2 H), 7.41 (t, *J*=8.99 Hz, 2 H), 7.74 (d, *J*=8.21 Hz, 1 H), 8.27 (s, 1 H).

### EXAMPLE 52

### 2-Isobutyl-5-[1-methyl-3-(trifluoromethyl)-1H-pyrazol-5-yl]-1H-pyrrolo[3,2-b]pyridine

### a) 2-(4-Methylpent-1-yn-1-yl)-6-[1-methyl-3-(trifluoromethyl)-1H-pyrazol-5-yl]pyridin-3-amine

Obtained (81% yield) as a solid from 2-bromo-6-[1-methyl-3-(trifluoromethyl)-1*H-*pyrazol-5-yl]pyridin-3-amine (preparation 19b) and 4-methylpent-1-yne following the experimental procedure as described in preparation 5a, using thermal conditions instead of microwave irradiation and tetrahydrofuran as solvent. Crude residue was purified by flash chromatography (0% to 100%, hexane - ethyl acetate).
LRMS (m/z): 323 (M+1)⁺.

### b) 2-Isobutyl-5-[1-methyl-3-(trifluoromethyl)-1H-pyrazol-5-yl]-1H-pyrrolo[3,2-b]pyridine

Obtained (38% yield) from 2-(4-methylpent-1-yn-1-yl)-6-[1-methyl-3-(trifluoromethyl)-1*H*-pyrazol-5-yl]pyridin-3-amine (Example 52a) following the experimental procedure as described in Preparation 14c.
LRMS (m/z): 323 (M+1)⁺.
¹H NMR (400 MHz, CHLOROFORM-*d*) d ppm 1.02 (d, J=6.64 Hz, 6 H), 1.94 - 2.14 (m, 1 H), 2.71 (d, *J*=6.64 Hz, 2 H), 4.27 (s, 3 H), 6.49 (s, 1 H), 6.76 (s, 1 H), 7.32 (d, *J*=8.60 Hz, 1 H), 7.64 (d, *J*=7.42 Hz, 1 H), 8.06 (s, 1 H).

### EXAMPLE 53

### 2-(4-Fluoro-2-methylphenyl)-5-[1-methyl-3-(trifluoromethyl)-1H-pyrazol-5-y)]-1H-pyrrolo[3,2-b]pyridine

Obtained (43% yield) as a white solid from 2-iodo-5-[1-methyl-3-(trifluoromethyl)-1*H-*pyrazol-5-yl]-1-(phenylsulfonyl)-1*H*-pyrrolo[3,2-*b*]pyridine (Preparation 22b) and 4-fluoro-2-methylphenylboronic acid following the experimental procedure as described in Example 26.
LRMS (m/z): 375 (M+1)⁺.
¹H NMR (300 MHz, CHLOROFORM-*d*) d ppm 2.45 (s, 3H), 4.31 (s, 3 H), 6.74 - 6.87 (m, 2 H), 6.95 - 7.17 (m, 2 H), 7.39 - 7.51 (m, 2 H), 7.76 (d, *J*=7.63 Hz, 1 H), 8.29 (br. s., 1 H).

### EXAMPLE 54

### 2-(5-Fluoro-2-methylphenyl)-5-[1-methyl-3-(trifluoromethyl)-1H-pyrazol-5-yl]-1H-pyrrolo[3,2-b]pyridine

Obtained (60% yield) as a white solid from 2-iodo-5-[1-methyl-3-(trifluoromethyl)-1*H-*pyrazol-5-yl]-1-(phenylsulfonyl)-1*H*-pyrrolo[3,2-*b*]pyridine (Preparation 22b) and 5-fluoro-2-methylphenylboronic acid following the experimental procedure as described in Example 26.
LRMS (m/z): 375 (M+1)⁺.
¹H NMR (300 MHz, DMSO-*d*₆) d ppm 2.50 (s, 3 H), 4.29 (s, 3 H), 6.91 (s, 1 H), 7.12 - 7.29 (m, 2 H), 7.35 - 7.53 (m, 2 H), 7.65 (d, J=8.80 Hz, 1 H), 7.91 (d, J=8.80 Hz, 1 H), 11.84 (br. s., 1 H)

### EXAMPLE 55

### 2-(2,3-Difluorophenyl)-5-[1-methyl-3-(trifluoromethyl)-1H-pyrazol-5-yl]-1H-pyrrolo[3,2-b]pyridine

Obtained (48% yield) as a white solid from 2-iodo-5-[1-methyl-3-(trifluoromethyl)-1*H-*pyrazol-5-yl]-1-(phenylsulfonyl)-1*H*-pyrrolo[3,2-*b*]pyridine (Preparation 22b) and 2,3-difluorophenylboronic acid following the experimental procedure as described in Example 26.
LRMS (m/z): 379 (M+1)⁺.
¹H NMR (300 MHz, DMSO-*d*₆) d ppm 4.26 - 4.34 (m, 3 H), 7.17 (d, J=3.52 Hz, 1 H), 7.24 (s, 1 H), 7.34 - 7.58 (m, 2 H), 7.65 - 7.73 (m, 1 H), 7.74 - 7.87 (m, 1 H), 7.97 (d, *J*=7.63 Hz, 1 H), 12.07 (br. s., 1 H).

### EXAMPLES 56

### 2-Fluoro-6-{5-[1-methyl-3-(trifluoromethyl)-1H-pyrazol-5-yl]-1H-pyrrolo[3,2-b]pyridin-2-yl]benzonitrile

Obtained (45% yield) as a white solid from 2-iodo-5-[1-methyl-3-(trifluoromethyl)-1*H-*pyrazol-5-yl]-1-(phenylsulfonyl)-1*H*-pyrrolo[3,2-*b*]pyridine (Preparation 22b) and 2-fluoro-6-(4,4,5,5-tetramethyl-1,3,2-dioxaborolan-2-yl)benzonitrile following the experimental procedure as described in Example 26.
LRMS (m/z): 386 (M+1)⁺.
¹H NMR (300 MHz, DMSO-*d*₆) d ppm 4.31 (s, 3 H), 7.29 (s, 1 H), 7.34 (s, 1 H), 7.56 - 7.68 (m,1 H), 7.68 - 7.90 (m, 2 H), 7.91 - 8.08 (m, 2 H), 12.31 (br. s., 1 H).

### EXAMPLE 57

### 2-(5-Chloro-2-methylphenyl)-5-[1-methyl-3-(trifluoromethyl)-1H-pyrazol-5-yl]-1H-pyrrolo[3,2-b]pyridine

Obtained (19% yield) as a white solid from 2-iodo-5-[1-methyl-3-(trifluoromethyl)-1*H-*pyrazol-5-yl]-1-(phenylsulfonyl)-1*H*-pyrrolo[3,2-*b*]pyridine (Preparation 22b) and 5-chloro-2-methylphenylboronic acid following the experimental procedure as described in Example 26.
LRMS (m/z): 391 (M+1)⁺.
¹H NMR (300 MHz, DMSO-*d*₆) d ppm 2.50 (s, 3 H), 4.29 (s, 3 H), 6.92 (s, 1 H), 7.24 (s, 1 H), 7.43 (s, 2 H), 7.59 - 7.73 (m, 2 H), 7.91 (d, *J*=8.80 Hz, 1 H), 11.92 (br. s., 1 H).

### EXAMPLE 58

### 2-(3-Fluoropyridin-4-yl)-5-(1-methyl-3-(trifluoromethyl)-1H-pyrazol-5-yl)-1H-pyrrolo[3,2-b]pyridine

Obtained (24% yield) as a yelow solid from 2-iodo-5-[1-methyl-3-(trifluoromethyl)-1*H-*pyrazol-5-yl]-1-(phenylsulfonyl)-1*H*-pyrrolo[3,2-*b*]pyridine (Preparation 22b) and 3-fluoro-4-(4,4,5,5-tetramethyl-1,3,2-dioxaborolan-2-yl)pyridine following the experimental procedure as described in Example 26.
LRMS (m/z): 362(M+1)⁺.
¹H NMR (300 MHz, DMSO-*d*₆) d ppm 4.30 (s, 3 H), 7.29 (s, 1 H), 7.38 (s, 1 H), 7.75 (d, *J*=8.80 Hz, 1 H), 7.96 - 8.09 (m, 2 H), 8.60 (d, *J*=4.11 Hz, 1 H), 8.77 (d, *J*=2.93 Hz, 1 H), 12.25 (br. s., 1 H).

### EXAMPLE 59

### 2-(2-Chloro-6-fluorophenyl)-5-(5-methyl-3-(trifluoromethyl)-1H-pyrazol-1-yl)-1H-pyrrolo[2,3-b]pyridine

*N*-(3-((2-chloro-6-fluorophenyl)ethynyl)-5-(5-methyl-3-(trifluoromethyl)-1*H*-pyrazol-1-yl)pyridin-2-yl)-2,2,2-trifluoroacetamide (Preparation 44, 506 mg, 0.72 mmol) was dissolved in 9 ml dimethylformamide. Triethylamine (604 µl, 4.33 mmol) was added and the reaction was heated at 120ºC for 3 h. The solvent was evaporated to dryness and the crude was purifed directly by flash chromatography (0% to 50%, hexane-ehtyl acetate) to give 102 mg (35% yield) of the title compound as a white solid.
LRMS (m/z): 395 (M+1)⁺.
¹H NMR (300 MHz, DMSO-*d*₆) d ppm 2.32 - 2.37 (m, 3 H), 6.76 (d, J=1.76 Hz, 1 H), 6.80 (s, 1 H), 7.39 - 7.49 (m, 1 H), 7.51 - 7.64 (m, 2 H), 8.26 (d, *J*=2.93 Hz, 1 H), 8.42 (d, *J*=2.35 Hz, 1 H), 12.43 (s, 1 H).

### EXAMPLE 60

### 4-(2-(2-Chlorophenyl)-1H-pyrrolo[3,2-b]pyridin-5-yl)-N,N,3-trimethylbenzenesulfonamide

4-(2-Iodo-1-(phenylsulfonyl)-1*H*-pyrrolo[3,2-*b*]pyridin-5-yl)-*N,N*,3-trimethylbenzene-sulfonamide (Preparation 48b) was treated with (2-chlorophenyl)boronic acid, 2M cesium carbonate, [1,1'-bis(diphenylphosphino)ferrocene]dichloropalladium(II), complex with dichloromethane and dioxane as a solvent according to the method described in Preparation 3a. The reaction was heated at 100ºC overnight. The crude was purified by flash chromatography (0% to 20%, dichloromethane-methanol) to give the title compound (50% yield) as a beige solid.
LRMS (m/z): 426 (M+1)⁺.
¹H NMR (300 MHz, DMSO-*d*₆) d ppm 2.47 (s, 3 H), 2.67 (s, 6 H), 7.01 (s, 1 H), 7.37 (d, *J*=8.80 Hz, 1 H), 7.45 - 7.57 (m, 3 H), 7.64 - 7.73 (m, 3 H), 7.76 - 7.82 (m, 1 H), 7.92 (d, *J*=8.80 Hz, 1 H), 11.87 (s, 1 H).

### EXAMPLE 61

### 4-((4-(6-(2-Chloro-6-fluorophenyl)-5H-pyrrolo[2,3-b]pyrazin-2-yl)-3-methylphenyl)sulfonyl)morpholine

3-((2-Chloro-6-fluorophenyl)ethynyl)-5-(2-methyl-4-(morpholinosulfonyl)phenyl)pyrazin-2-amine (Preparation 50) was treated with potassium *tert*-butoxide and N-methylpyrrolidone as a solvent according to the method described in Example 10. The crude was purified by reverse phase using SP1 Purification System to give (27% yield) of the title compound as a solid.
LRMS (m/z): 487 (M+1)⁺.
¹H NMR (300 MHz, DMSO-*d*₆) d ppm 2.90 - 2.97 (m, 4 H), 3.31 (s, 3 H), 3.64 - 3.70 (m, 4 H), 6.92 (s, 1 H), 7.44 - 7.52 (m, 1 H), 7.55 - 7.65 (m, 2 H), 7.69 (d, *J*=7.63 Hz, 1 H), 7.74 (s, 1 H), 7.79 (d, *J*=8.22 Hz, 1 H), 8.55 (s, 1 H), 12.63 (s, 1H).

### EXAMPLE 62

### 2-(3-Fluoro-2-methylphenyl)-5-(1-methyl-3-(trifluoromethyl)-1H-pyrazol-5-yl)-1H-pyrrolo[3,2-b]pyridine

Obtained (68% yield) as a yelow solid from 2-iodo-5-[1-methyl-3-(trifluoromethyl)-1*H-*pyrazol-5-yl]-1-(phenylsulfonyl)-1*H*-pyrrolo[3,2-*b*]pyridine (Preparation 22b) and (3-fluoro-2-methylphenyl)boronic acid following the experimental procedure as described in Example 26.
LRMS (m/z): 375 (M+1)⁺.
¹H NMR (300 MHz, DMSO-*d*₆) d ppm 2.39 (d, *J*=2.35 Hz, 3 H), 4.29 (s, 3 H), 6.87 (s, 1 H), 7.23 (s, 1 H), 7.27 - 7.33 (m, 1 H), 7.39 - 7.47 (m, 2 H), 7.66 (d, *J*=8.80 Hz, 1 H), 7.91 (d, *J*=9.39 Hz, 1 H), 11.89 (s, 1 H).

### EXAMPLE 63

### 2-(2-Chlorophenyl)-5-(6-methoxy-4-methylpyridin-3-yl)-1H-pyrrolo[2,3-c]pyridine

2-Iodo-5-(6-methoxy-4-methylpyridin-3-yl)-1-(phenylsulfonyl)-1*H*-pyrrolo[2,3-*c*]pyridine (Preparation 52c) was treated with (2-chlorophenyl)boronic acid, cesium carbonate, [1,1'-bis(diphenylphosphino)ferrocene]dichloropalladium(II), complex with dichloromethane and dioxane as a solvent according to the method of Preparation 3a to give the title compound (30% yield) as a white solid.
LRMS (m/z): 350(M+1)⁺.
¹H NMR (300 MHz, DMSO-*d*₆) d ppm 2.34 (s, 3 H), 3.88 (s, 3 H), 6.77 (s, 1 H), 6.93 (s, 1 H), 7.43 - 7.58 (m, 2 H), 7.61 - 7.73 (m, 2 H), 7.72 - 7.83 (m, 1 H), 8.16 (s, 1 H), 8.86 (s, 1 H), 12.05 (br. s., 1 H).

### EXAMPLE 64

### 2-(2-Fluoro-6-methylphenyl)-5-(1-methyl-3-(trifluoromethyl)-1H-pyrazol-5-yl)-1H-pyrrolo[3,2-b]pyridine

In a microwave vessel 2-Iodo-5-[1-methyl-3-(trifluoromethyl)-1*H*-pyrazol-5-yl]-1-(phenylsulfonyl)-1*H*-pyrrolo[3,2-*b*]pyridine (Preparation 22b, 100 mg, 0.19 mmol)) was dissolved in 1ml toluene. (2-Fluoro-6-methylphenyl)boronic acid (Preparation 25, 48 mg, 0.28 mmol) and potassium phosphate (120 mg, 0.57 mmol) were added. The mixture was submitted to three vacuum-argon cycles. Tris(dibenzylideneacetone)dipalladium(0) (5 mg, 0.01 mmol) and 2-dicyclohexylphosphino-2',6'-dimethoxybiphenyl (5 mg, 0.01 mmol) were added and was submitted to three vacuum-argon cycles again. The vessel was sealed and heated at 120ºC for 30 min. under microwaves conditions. The reaction mixture was filtered and washed with ethyl acetate. The residue was evaporated under reduced pressure and the crude was dissolved in 4ml ethanol and 0.5 ml sodium hydroxide 8N. The mixture was stirred at room temperature for 30 min and was evaporated under reduced pressure. The residue was re-dissolved in ethyl acetate and washed with water. The organic phase was dried over sodium sulphate and concentrated. The residue was purified by flash chromatography (0%-65% hexane-diehtyl ether) to give the title compound (29 mg, 40%) as a white solid.
LRMS (m/z): 375 (M+1)⁺.
¹H NMR (300 MHz, DMSO-*d*₆) d ppm 2.34 (s, 3 H), 4.29 (s, 3 H), 6.78 (s, 1 H), 7.15 - 7.31 (m, 3 H), 7.36 - 7.50 (m, *J*=5.87 Hz, 1 H), 7.65 (s, 1 H), 7.90 (s, 1 H), 11.83 (s, 1 H).

### EXAMPLE 65

### 4-(6-(2-Chloro-6-fluorophenyl)-5H-pyrrolo[2,3-b]pyrazin-2-yl)-3-methylbenzenesulfonamide

4-(5-Amino-6-((2-chloro-6-fluorophenyl)ethynyl)pyrazin-2-yl)-3-methylbenzene-sulfonamide (Preparation 54) was treated with potassium *tert*-butoxide and *N-*methylpyrrolidone as a solvent according to the method described in Example 10. The crude was precipitated with a dichloromethane obtained a solid that was dried in the oven to give the title compound (36% yield) as a beige solid.
LRMS (m/z): 417 (M+1)⁺.
¹H NMR (300 MHz, DMSO-*d*₆) d ppm 2.47 (s, 3 H), 6.92 (s, 1 H), 7.43 (s, 1 H), 7.47 - 7.57 (m, 1 H), 7.58 - 7.60 (m, 1 H), 7.61 - 7.66 (m, 1 H), 7.69 (dd, 1 H), 7.75 - 7.80 (m, 1 H), 7.82 (s, 1 H), 8.51 (s, 1 H).

### EXAMPLE 66

### 2-(2-Chloro-4-fluorophenyl)-5-(1-methyl-3-(trifluoromethyl)-1H-pyrazol-5-yl)-1H-pyrrolo[3,2-b]pyridine

Obtained (19% yield) as a solid from 2-iodo-5-[1-methyl-3-(trifluoromethyl)-1*H*-pyrazol-5-yl]-1-(phenylsulfonyl)-1*H*-pyrrolo[3,2-*b*]pyridine (Preparation 22b) and (2-chloro-4-fluorophenyl)boronic acid following the experimental procedure as described in Example 26.
LRMS (m/z): 395 (M+1)⁺.
¹H NMR (300 MHz, DMSO-*d*₆) d ppm 4.29 (s, 3 H), 7.02 (s, 1 H), 7.24 (s, 1 H), 7.40 - 7.49 (m, 1 H), 7.64 - 7.72 (m, 2 H), 7.80 - 7.87 (m, *J*=8.80, 6.46 Hz, 1 H), 7.93 (d, *J*=7.63 Hz, 1 H), 11.98 (s, 1 H).

### EXAMPLE 67

### 6-Cyclopentyl-2-(6-methoxy-4-methylpyridin-3-yl)-5H-pyrrolo[2,3-b]pyrazine

### a) 3-(Cyclopentylethynyl)-5-(6-methoxy-4-methylpyridin-3-yl)pyrazin-2-amine

In a Schlenk vessel 3-bromo-5-(6-methoxy-4-methylpyridin-3-yl)pyrazin-2-amine (Preparation 4b, 120 mg, 0.41 mmol) was dissolved in 0.39 ml tetrahydrofuran and 2.3 ml triethylamine. Copper (I) iodide (7.7 mg, 0.04 mmol) was added and the mixture was submitted to three vacuum-argon cycles. Then bis(triphenylphosphine)palladium(II) dichloride (14 mg, 0.02 mmol) was added and the was further submitted to three vacuum-argon cycles more. The reaction mixture was heated at 90ºC for 3h. The reaction was cooled at room temperature, filtered through celite and washed with ethyl acetate and dichloromethane. The filtered was concentrated under reduced pressure and purifed using Isolera purification system (0%-50%, hexane-ehtyl acetate) to give 111 mg (87% yield) of the title compound.
LRMS (m/z): 309 (M+1)⁺.

### b) 6-Cyclopentyl-2-(6-methoxy-4-methylpyridin-3-yl)-5H-pyrrolo[2,3-b]pyrazine

Obtained (39% yield) from 3-(cyclopentylethynyl)-5-(6-methoxy-4-methylpyridin-3-yl)pyrazin-2-amine (Example 67a) following the experimental procedure as described in Preparation 14c. The crude was purified using Isolera purification system (0%-55%, hexane-ethyl acetate) to obtain the final producte as a solid.
LRMS (m/z): 309 (M+1)⁺.
¹H NMR (300 MHz, DMSO-*d*₆) d ppm 1.62 - 1.83 (m, 6 H), 2.06 - 2.15 (m, 2 H), 2.33 (s, 3 H), 3.19 - 3.29 (m, 1 H), 3.89 (s, 3 H), 6.41 (s, 1 H), 6.81 (s, 1 H), 8.17 (s, 1 H), 8.25 (s, 1 H), 11.99 (s, 1 H).

### EXAMPLE 68

### 6-(2,6-Difluorophenyl)-2-(6-methoxy-4-methylpyridin-3-yl)-5H-pyrrolo[2,3-b]pyrazine

### a) 3-((2,6-Difluorophenyl)ethynyl)-5-(6-methoxy-4-methylpyridin-3-yl)pyrazin-2-amine

Obtained (69% yield) from 3-bromo-5-(6-methoxy-4-methylpyridin-3-yl)pyrazin-2-amine (Preparation 4b) and 2-ethynyl-1,3-difluorobenzene following the experimental procedure as described in Example 67a.
LRMS (m/z): 353 (M+1)⁺.

### b) 6-(2,6-Difluorophenyl)-2-(6-methoxy-4-methylpyridin-3-yl)-5H-pyrrolo[2,3-b]pyrazine

Obtained (22% yield) from 3-((2,6-difluorophenyl)ethynyl)-5-(6-methoxy-4-methylpyridin-3-yl)pyrazin-2-amine (Example 68a) following the experimental procedure as described in Preparation 14c. The crude was precipitate with dichloromethane and washed with hexane. The solid was dried in the oven to give the title compound.
LRMS (m/z): 353 (M+1)⁺.
¹H NMR (300 MHz, DMSO-*d*₆) d ppm 2.39 (s, 3 H), 3.90 (s, 3 H), 6.84 (s, 1 H), 6.97 (s, 1 H), 7.35 (t, *J*=8.22 Hz, 2 H), 7.53 - 7.70 (m, 1 H), 8.25 (s, 1 H), 8.48 (s, 1 H), 12.54 (s, 1 H).

### EXAMPLE 69

### 4-(6-(2-Chloro-6-fluorophenyl)-5H-pyrrolo[2,3-b]pyrazin-2-yl)-N,3-dimethylbenzenesulfonamide

4-(5-Amino-6-((2-chloro-6-fluorophenyl)ethynyl)pyrazin-2-yl)-*N*,3-dimethylbenzene-sulfonamide (Preparation 57) was treated with potassium *tert*-butoxide and *N-*methylpyrrolidone as a solvent according to the method described in Example 10. The crude was purified by flash chromatography using Isolera Purification System (0% to 35%, dichloromethane - (dichloromethane-methanol 5%)) to obtain the title compound (9% yield) as a solid.
LRMS (m/z): 431 (M+1)⁺.
¹H NMR (300 MHz, DMSO-*d*₆) d ppm 2.47 (s, 3 H), 3.32 (s, 3 H), 6.90 (s, 1 H), 7.42 - 7.56 (m, 2 H), 7.57 - 7.67 (m, 2 H), 7.73 (s, 1 H), 7.77 (s, 1 H), 8.52 (s, 1 H), 12.62 (s, 1 H).

### EXAMPLE 70

### 2-(2-Chloro-6-methylphenyl)-5-(1-methyl-3-(trifluoromethyl)-1H-pyrazol-5-yl)-1H-pyrrolo[3,2-b]pyridine

Obtained (3% yield) as a solid from 2-iodo-5-[1-methyl-3-(trifluoromethyl)-1*H*-pyrazol-5-yl]-1-(phenylsulfonyl)-1*H*-pyrrolo[3,2-*b*]pyridine (Preparation 22b) and (2-chloro-6-methylphenyl)boronic acid following the experimental procedure as described in Example 64. The residue was filtered, washed with ethyl acetate and concentrated under reduces pressure. The crude was re-dissolved in 4ml ethanol and 0.5 ml sodium hydroxide 8N was added and stirred at room temperature for 50 min. The mixture was evaporated under reduced pressure and partitioned between ethyl acetate and water. The organic layer was dried over sodium sulphate, filtered and evaporated. The crude was purified by flash chromatography (0%-55%, hexane-ethyl acetate) to obtain the title compound.
LRMS (m/z): 391 (M+1)⁺.
¹H NMR (300 MHz, CHLOROFORM-*d*) d ppm 2.24 - 2.35 (m, 3 H), 4.34 (s, 3 H), 6.77 (d, *J*=2.93 Hz, 1 H), 6.82 (s, 1 H), 7.30 - 7.41 (m, 3 H), 7.46 (d, *J*=8.80 Hz, 1 H), 7.80 (d, *J*=9.39 Hz, 1 H), 8.29 (s, 1 H).

### EXAMPLE 71

### 2-(2-Chloro-5-fluorophenyl)-5-(1-methyl-3-(trifluoromethyl)-1H-pyrazol-5-yl)-1H-pyrrolo[3,2-b]pyridine

Obtained (3% yield) as a solid from 2-iodo-5-[1-methyl-3-(trifluoromethyl)-1*H*-pyrazol-5-yl]-1-(phenylsulfonyl)-1*H*-pyrrolo[3,2-*b*]pyridine (Preparation 22b) and (2-chloro-5-fluorophenyl)boronic acid acid following the experimental procedure as described in Example 26.
LRMS (m/z): 395 (M+1)⁺.
¹H NMR (300 MHz, CHLOROFORM-*d*) d ppm 4.32 (s, 3 H), 6.82 (s, 1 H), 7.05 - 7.15 (m, 2 H), 7.40 - 7.54 (m, 3 H), 7.82 (d, *J*=7.63 Hz, 1 H), 9.02 (s, 1 H).

### EXAMPLE 72

### 2-Benzyl-5-(1-methyl-3-(trifluoromethyl)-1H-pyrazol-5-yl)-1H-pyrrolo[3,2-b]pyridine

2-Benzyl-5-(1-methyl-3-(trifluoromethyl)-1*H*-pyrazol-5-yl)-1-(phenylsulfonyl)-1H-pyrrolo[3,2-*b*]pyridine (Preparation 58) was treated with sodium hydroxide 8N and ethanol as a solvent. The reaction was stirred at room temperature for 1 h according to the method described in Example 31. The crude was purified by flash chromatography using the Isolera Purification System (0% to 60%, hexane-diethyl ether) to obtain the final product (70% yield) as a white solid.
LRMS (m/z): 357 (M+1)⁺.
¹H NMR (300 MHz, CHLOROFORM-*d*) d ppm 4.22 (s, 2 H), 4.27 (s, 3 H), 6.60 (s, 1 H), 6.76 (s, 1 H,) 7.28 - 7.41 (m, 6 H), 7.59 (d, *J*=7.63 Hz, 1 H), 7.97 (s, 1 H).

### EXAMPLE 73

### 5-Chloro-6-(2-(2-chloro-6-fluorophenyl)-1H-pyrrolo[2,3-b]pyridin-5-yl)-2-methylbenzo[d]oxazole

5-Chloro-2-methyl-6-(2-(4,4,5,5-tetramethyl-1,3,2-dioxaborolan-2-yl)-1*H*-pyrrolo[2,3-*b*]pyridin-5-yl)benzo[*d*]oxazole (Preparation 47) was treated with 1-chloro-3-fluoro-2-iodobenzene, cesium carbonate, 1,1'-bis(diphenylphosphino)ferrocene-palladium(II) dichloride dichloromethane complex, dioxane and water as a solvents according to the method described in Preparation 3a. The crude was purified using SP1 Purification System (0% to 100%, hexane-ethyl acetate) to give (12 % yield) the title compound as a solid.
LRMS (m/z): 412 (M+1)⁺.
¹H NMR (300 MHz, DMSO-*d*₆) d ppm 2.66 (s, 3 H), 6.71 (d, *J*=2.35 Hz, 1 H), 7.38 - 7.48 (m, 1 H), 7.50 - 7.63 (m, 2 H), 7.88 (s, 1 H), 7.95 (s, 1 H), 8.10 (d, *J*=2.35 Hz, 1 H), 8.32 (d, *J*=2.35 Hz, 1 H), 12.19 (s, 1 H).

### EXAMPLE 74

### 2-(2-Chloro-3-fluorophenyl)-5-(1-methyl-3-(trifluoromethyl)-1H-pyrazol-5-yl)-1H-pyrrolo[3,2-b]pyridine

Obtained (11% yield) as a solid from 2-iodo-5-[1-methyl-3-(trifluoromethyl)-1*H*-pyrazol-5-yl]-1-(phenylsulfonyl)-1*H*-pyrrolo[3,2-*b*]pyridine (Preparation 22b) and (2-chloro-3-fluorophenyl)boronic acid following the experimental procedure as described in Example 26.
LRMS (m/z): 395 (M+1)⁺.
¹H NMR (300 MHz, CHLOROFORM-*d*) d ppm 4.33 (s, 3 H), 6.81 - 6.84 (m, 1 H), 7.13 (d, J=2.35 Hz, 1 H), 7.35 - 7.45 (m, 2 H), 7.45 - 7.57 (m, 2 H). 7.83 (d, J=7.63 Hz, 1 H), 8.95 (s, 1 H).

### EXAMPLE 75

### 2-(2-Chlorophenyl)-5-(6-methoxy-4-methylpyridin-3-yl)-1H-pyrrolo[2,3-b]pyridine

2-Iodo-5-(6-methoxy-4-methylpyridin-3-yl)-1*H*-pyrrolo[2,3-*b*]pyridine (Preparation 29c) was treated with (2-chlorophenyl)boronic acid, cesium carbonate, 1,1'-bis(diphenylphosphino)ferrocene-palladium(II) dichloride dichloromethane complex, dioxane as a solvents according to the method described in Preparation 3a. The crude was purified by flash chromatography using SP1 Purification System (0% to 100%, hexane-diehtyl ether) to obtain the title compound (44% yield) as a yellow solid.
LRMS (m/z): 350(M+1)⁺.
¹H NMR (300 MHz, DMSO-*d*₆) d ppm 2.26 (s, 3 H), 3.88 (s, 3 H), 6.83 (s, 1 H), 6.90 (s, 1 H), 7.38 - 7.55 (m, 2 H), 7.64 (d, *J*=7.63 Hz, 1 H), 7.78 (d, *J*=9.39 Hz, 1 H), 7.99 (s, 1 H), 8.05 (s, 1 H), 8.23 (s, 1 H), 12.17 (br. s., 1 H).

### EXAMPLE 76

### 2-(2-Fluorophenyl)-5-(6-methoxy-4-methylpyridin-3-yl)-1H-pyrrolo[2,3-b]pyridine

2-Iodo-5-(6-methoxy-4-methylpyridin-3-yl)-1*H*-pyrrolo[2,3-*b*]pyridine (Preparation 29c) was treated with (2-fluorophenyl)boronic acid, cesium carbonate, 1,1'-bis(diphenylphosphino)ferrocene-palladium(II) dichloride dichloromethane complex, dioxane as a solvents. The reaction was heating at 110ºC for 3.5 h according to the method described in Preparation 3a under standard conditions. The crude was purified by flash chromatography using SP1 Purification System (0% to 2%, dichloromethane-methanol) to obtain the title compound (58% yield) as a white solid.
LRMS (m/z): 334(M+1)⁺.
¹H NMR (300 MHz, DMSO-*d*₆) d ppm 2.26 (s, 3 H), 3.88 (s, 3 H), 6.83 (s, 1 H), 6.95 (d, *J*=2.35 Hz, 1 H), 7.28 - 7.56 (m, 3 H), 7.92 - 8.10 (m, 3 H), 8.19 - 8.29 (m, *J*=2.35 Hz, 1 H), 12.26(br.s., 1 H).

### EXAMPLE 77

### 2-(2-Chloro-6-fluorophenyl)-5-(5-methyl-3-(trifluoromethyl)-1H-1,2,4-triazol-1-yl)-1H-pyrrolo[3,2-b]pyridine

2-((2-Chloro-6-fluorophenyl)ethynyl)-6-(5-methyl-3-(trifluoromethyl)-1*H*-1,2,4-triazol-1-yl)pyridin-3-amine (Preparation 28) was treated with potassium *tert*-butoxide and *N-*methylpyrrolidone as a solvent according to the method described in Example 10. The crude was purified by flash chromatography using Isolera Purification System (0% to 30%, dichloromethane - (dichloromethane-methanol 2%)) to obtain the title compound (11% yield) as a solid.
LRMS (m/z): 396 (M+1)⁺.
¹H NMR (300 MHz, CHLOROFORM-*d*) d ppm 2.89 (s, 3 H), 7.05 - 7.17 (m, 2 H), 7.25 - 7.36 (m, 2 H), 7.65 (d, *J*=8.80 Hz, 1 H), 7.84 (d, *J*=8.80 Hz, 1 H), 8.83 (s, 1 H).

### EXAMPLE 78

### 3,5-Dimethyl-4-(5-(1-methyl-3-(trifluoromethyl)-1H-pyrazol-5-yl)-1H-pyrrolo[3,2-b]pyridin-2-yl)isoxazole

Obtained (54% yield) as a solid from 2-iodo-5-[1-methyl-3-(trifluoromethyl)-1*H*-pyrazol-5-yl]-1-(phenylsulfonyl)-1*H*-pyrrolo[3,2-*b*]pyridine (Preparation 22b) and (3,5-dimethylisoxazol-4-yl)boronic acid following the experimental procedure as described in Example 26.
LRMS (m/z): 362(M+1)⁺.
¹H NMR (300 MHz, CHLOROFORM-*d*) d ppm 2.47 (s, 3 H), 2.62 (s, 3 H), 4.32 (s, 3 H), 6.80 (d, *J*=9.39 Hz, 2 H), 7.46 (d, *J*=8.80 Hz, 1 H), 7.80 (d, *J*=8.80 Hz, 1 H), 8.24 (s, 1 H).

### EXAMPLE 79

### 6-(2,6-Difluorophenyl)-2-(1-methyl-3-(trifluoromethyl)-1H-pyrazol-5-yl)-5H-pyrrolo[2,3-b]pyrazine

3-((2,6-Difluorophenyl)ethynyl)-5-(1-methyl-3-(trifluoromethyl)-1*H*-pyrazol-5-yl)pyrazin-2-amine (Preparation 59) was treated with potassium *tert*-butoxide and *N-*methylpyrrolidone as a solvent according to the method described in Example 10. The crude was purified by reverse phase using Isolera Purification System to obtain the title compound (48% yield) as a solid.
LRMS (m/z): 380 (M+1)⁺.
¹H NMR (300 MHz, CHLOROFORM-*d*) d ppm 4.26 (s, 3 H), 6.83 (s, 1 H), 7.07 (dd, *J*=9.68, 8.51 Hz, 2 H), 7.19 (s, 1 H), 7.29 (d, *J*=1.76 Hz, 2 H), 8.52 (s, 1 H), 9.71 (s, 1 H).

### EXAMPLE 80

### 2-(2-Fluorophenyl)-5-(6-methoxy-4-methylpyridin-3-yl)-1H-pyrrolo[2,3-c]pyridine

2-Iodo-5-(6-methoxy-4-methylpyridin-3-yl)-1-(phenylsulfonyl)-1*H*-pyrrolo[2,3-*c*]pyridine (Preparation 52c) was treated with (2-fluorophenyl)boronic acid, cesium carbonate, [1,1'-bis(diphenylphosphino)ferrocene]dichloropalladium(II), complex with dichloromethane and dioxane as a solvent according to the method of Preparation 3a under standard conditions. The crude was purified by flash cromatography (0% to 80%, hexane-ehtyl acetate) to give the title compound (48% yield) as a solid.
LRMS (m/z): 334 (M+1)⁺.
¹H NMR (300 MHz, DMSO-*d*₆) d ppm 2.34 (s, 3 H), 3.88 (s, 3 H), 6.77 (s, 1 H), 7.01 (d, *J*=2.93 Hz, 1 H), 7.36 - 7.55 (m, 3 H), 8.00 (t, *J*=7.63 Hz, 1 H), 8.16 (s, 1 H), 8.88 (s, 1 H), 12.09 (s, 1 H).

### EXAMPLE 81

### 2-(2-Chloro-5-fluorophenyl)-5-(6-methoxy-4-methylpyridin-3-yl)-1H-pyrrolo[2,3-b]pyridine

2-Iodo-5-(6-methoxy-4-methylpyridin-3-yl)-1*H*-pyrrolo[2,3-*b*]pyridine (Preparation 29c) was treated with (2-chloro-5-fluorophenyl)boronic acid, cesium carbonate, 1,1'-bis(diphenylphosphino)ferrocene-palladium(II) dichloride dichloromethane complex, dioxane as a solvents. The reaction was heating at 110ºC for 3.5 h according to the method described in Preparation 3a under standard conditions. The crude was purified by flash chromatography using SP1 Purification System (0% to 5%, dichloromethane-methanol) to obtain the title compound (45% yield) as a yellow solid.
LRMS (m/z): 368(M+1)⁺.
¹H NMR (300 MHz, DMSO-*d*₆) d ppm 2.26 (s, 3 H), 3.88 (s, 3 H), 6.83 (s, 1 H), 7.03 (s, 1 H), 7.25 - 7.42 (m, 1 H), 7.63 - 7.77 (m, 2 H), 7.97 - 8.09 (m, 2 H), 8.33 (s, 1 H), 12.31 (br. s., 1 H).

### EXAMPLE 82

### 2-(4-Isopropoxy-2-methylphenyl)-5-(1-methyl-3-(trifluoromethyl)-1H-pyrazol-5-yl)-1H-pyrrolo[3,2-b]pyridine

Obtained (58% yield) as a solid from 2-iodo-5-(1-methyl-3-(trifluoromethyl)-1*H*-pyrazol-5-yl)-1*H*-pyrrolo[3,2-*b*]pyridine (Preparation 27) and (4-isopropoxy-2-methylphenyl)boronic acid following the experimental procedure as described in Example 26. The crude was purified by flash chromatography (0%-65%, hexane-ethyl ether) to give the title compound.
LRMS (m/z): 415 (M+1)⁺.
¹H NMR (300 MHz, CHLOROFORM-*d*) d ppm 1.38 (s, 3 H), 1.40 (s, 3 H), 2.51 (s, 3 H), 4.31 (s, 3 H), 4.58 - 4.68 (m, 1 H), 6.80 (s, 2 H), 6.83 - 6.90 (m, 2 H), 7.37 - 7.44 (m, 2 H), 7.75 (d, *J*=9.39 Hz, 1 H), 8.28 (s, 1 H).

### EXAMPLE 83

### 2-(4-Ethoxy-2-methylphenyl)-5-(1-methyl-3-(trifluoromethyl)-1H-pyrazol-5-yl)-1H-pyrrolo[3,2-b]pyridine

Obtained (58% yield) as a solid from 2-iodo-5-(1-methyl-3-(trifluoromethyl)-1*H*-pyrazol-5-yl)-1*H*-pyrrolo[3,2-*b*]pyridine (Preparation 27) and (4-ethoxy-2-methylphenyl)boronic acid following the experimental procedure as described in Example 26. The crude was purified by flash chromatography (0%-100%, hexane-ethyl ether) to give the title compound.
LRMS (m/z): 401 (M+1)⁺.
¹H NMR (300 MHz, CHLOROFORM-*d*) d ppm 1.45 (t, *J*=7.04 Hz, 3 H), 2.52 (s, 3 H), 4.10 (q, *J*=7.04 Hz, 2 H), 4.31 (s, 3 H), 6.80 (s, 2 H), 6.82 - 6.91 (m, 2 H), 7.41 (t, *J*=7.92 Hz, 2 H), 7.75 (d, *J*=8.22 Hz, 1 H), 8.29 (s, 1 H).

### EXAMPLE 84

### 2-(2-Chloro-6-fluorophenyl)-5-(6-methoxy-4-methylpyridin-3-yl)-1H-pyrrolo[2,3-c]pyridine

2-Iodo-5-(6-methoxy-4-methylpyridin-3-yl)-1-(phenylsulfonyl)-1*H*-pyrrolo[2,3-*c*]pyridine (Preparation 52c) was treated with (2-chloro-6-fluorophenyl)boronic acid, potassium phosphate, tris(dibenzylideneacetone)dipalladium(0) (5 mg, 0.01 mmol) and 2-dicyclohexylphosphino-2',6'-dimethoxybiphenyl (5 mg, 0.01 mmol) according to the method described in Example 64. The crude was purified by reverse phase using Isolera Purification System to give the final product (11% yield) as a solid.
LRMS (m/z): 368 (M+1)⁺.
¹H NMR (300 MHz, CHLOROFORM-*d*) d ppm 2.39 (s, 3 H), 3.99 (s, 3 H), 6.70 (s, 1 H), 6.99 (s, 1 H), 7.18 - 7.24 (m, 1 H), 7.35 - 7.44 (m, 2 H), 7.65 (s, 1 H), 8.21 (s, 1 H), 8.92 (s, 1 H), 8.95 (s, 1 H).

### Example 85

### 2-(2,6-difluorophenyl)-5-[1-methyl-3-(trifluoromethyl)-1H-pyrazol-5-yl]-1H-pyrrolo[2,3-c]pyridine

A microwave tube was charged with 5-chloro-2-(2-chloro-6-fluorophenyl)-1-(phenylsulfonyl)-1H-pyrrolo[2,3-c]pyridine (Preparation 64, 25 mg, 0.06 mmol), (1-methyl-3-(trifluoromethyl)-1*H*-pyrazol-5-yl)boronic acid (40 mg, 0.22 mmol), cesium carbonate 2.0M (60 µL, 0.12 mmol), dioxane (0.5 ml). The mixture was degassed with argon and 1,1'-bis(diphenylphosphino)ferrocene-palladium(II) dichloride dichloromethane complex (9 mg, 0.02 mmol) was added. After capping the vial, it was subjected to three further cycles of evacuation-backfilling with argon and heated at 110ºC during 4h. The mixture was filtered through a plug of Celite, washing with ethyl acetate, the organic layer was washed with water and dried (MgSO₄) and evaporated. The crude product was dissolved in ethanol (1.5mL) and 0.2 mL of NaOH 8.0N were added and the mixture was stirred at room temperature 1h. The reaction mixture was concentrated and re-dissolved in ethyl acetate (15 mL), the organic layer was washed with water and dried (MgSO₄) and evaporated. Purification by flash chromatography (0% to 100% hexane- ethyl acetate) afforded the desired product (8 mg, 35%) as a white solid.
LRMS (m/z): 395 (M+1)⁺.
1 H NMR (300 MHz, CHLOROFORM-*d*) ppm 4.21 (s, 3 H), 6.77 (s, 1 H), 7.00 (s, 1 H), 7.13 - 7.26 (m, 1 H), 7.39 (t, J=3.52 Hz, 2 H), 7.86 (s, 1 H), 8.93 (s, 1 H), 9.04 (br. s., 1 H)

### Example 86

### 2-(2-chloro-6-fluorophenyl)-5-(4-methyl-6-(trifluoromethyl)pyridin-3-yl)-1H-pyrrolo[2,3-b]pyridine

Obtained (8% yield) from 5-Bromo-2-(2-chloro-6-fluorophenyl)-1*H*-pyrrolo[3,2-b]pyridine (preparation 61) and 4-methyl-5-(4,4,5,5-tetramethyl-1,3,2-dioxaborolan-2-yl)-2-(trifluoromethyl)pyridine (preparation 63) following the experimental procedure as described in Example 1(110ºC, 4h), followed by purification by flash chromatography (30% to 100% hexane - diethyl ether).
LRMS (m/z): 406 (M+1)⁺.
1 H NMR (400 MHz, DMSO-d6) d ppm 2.39 - 2.48 (s, 3 H), 6.73 (s, 1 H), 7.33 - 7.50 (m, 1 H), 7.48 - 7.69 (m, 2 H), 7.96 (s, 1 H), 8.09 - 8.24 (m, 1 H), 8.29 - 8.42 (m, 1 H), 8.67 (s, 1 H), 12.26 (br. s., 1 H)

### Example 87

### 2-(2-chloro-6-fluorophenyl)-5-(4,6-dimethoxypyridin-3-yl)-1H-pyrrolo[2,3-b]pyridine

Obtained (13% yield) from 5-Bromo-2-(2-chloro-6-fluorophenyl)-1H-pyrrolo[3,2-b]pyridine (preparation 61) and (4,6-dimethoxypyridin-3-yl)boronic acid (preparation 62) at 110ºC for 1.5h following the experimental procedure as described in Example 1 and followed by purification by flash chromatography (0% to 100% hexane - diethyl ether).
LRMS (m/z): 384 (M+1)⁺.
1 H NMR (400 MHz, CHLOROFORM-d) d ppm 3.86 (s, 3 H), 3.99 (s, 3 H), 6.34 (s, 1 H), 6.92 (s, 1 H), 7.12 - 7.23 (m, 1 H), 7.30 - 7.46 (m, 2 H), 8.04 (s, 2 H), 8.41 (s, 1 H), 9.18 (br. s., 1 H).

### EXAMPLE 88

### {2-(2-Chloro-6-fluorophenyl)-5-[1-methyl-3-(trifluoromethyl)-1H-pyrazol-5-yl]-1H-pyrrolo[3,2-b]pyridin-1-yl}methyl dihydrogen phosphate

### a) di-tert-Butyl {2-(2-chloro-6-fluorophenyl)-5-[1-methyl-3-(trifluoromethyl)-1H-pyrazol-5-yl]-1H-pyrrolo[3,2-b]pyridin-1-yl}methyl phosphate

Sodium hydride (14 mg, 0.35 mmol) was added portionwise to a solution of Example 14 (82 mg, 0.21 mmol) in *N,N*-dimethylformamide (3 ml) and the resulting mixture was stirred at room temperature for 20 minutes. Sodium iodide (5 mg, 0.03 mmol) and di-*tert*-butyl chloromethyl phosphate (110 mg, 0.43 mmol) were added sequentially and the mixture stirred at 60 °C for 2.5 hours. The reaction mixture was partitioned between water and ethyl acetate, the organic layer was washed with water, dried (MgSO₄), filtered and the solvent was evaporated in vacuo. Purification by silica flash chromatography (0% to 85% hexanes - diethyl ether) afforded the desired product as a white solid (0.111 g, 82% yield).
LRMS (m/z): 617, 619 (M+1)⁺.
¹H NMR (300 Hz, CHLOROFORM-*d*) δ ppm 1.33 (s, 18 H), 4.32 (s, 3 H), 5.59 - 5.79 (m, 1 H), 5.80 - 5.98 (m, 1 H), 6.86 (d, *J*=14.67 Hz, 2 H), 7.20 (t, *J*=8.22 Hz, 2 H) ,7.30 - 7.61 (m, 2 H), 8.15 (d, *J*=9.39 Hz, 1 H).

### b) {2-(2-Chloro-6-fluorophenyl)-5-[1-methyl-3-(trifluoromethyl)-1H-pyrazol-5-yl]-1H-pyrrolo[3,2-b]pyridin-1-yl}methyl dihydrogen phosphate

Trifluoroacetic acid (0.2 ml, 2.6 mmol) was added to a solution of di-*tert*-butyl {2-(2-chloro-6-fluorophenyl)-5-[1-methyl-3-(trifluoromethyl)-1*H*-pyrazol-5-yl]-1*H*-pyrrolo[3,2-*b*]pyridin-1-yl}methyl phosphate (0.11 g, 0.17 mmol) in dichloromethane (2 ml). The resulting mixture was stirred at room temperature for 40 minutes and then the solvent was removed in vácuo. Purification by silica C18 chromatography (0% to 100% water - methanol) afforded the desired product as a white solid (0.065 g, 76% yield).
LRMS (m/z): 505, 507 (M+1)⁺.
¹H NMR (300 Hz, METHANOL-*d*) δ ppm 4.22 (s, 3 H), 5.55 - 5.72 (m, 1 H), 5.75 - 5.97 (m, 1 H), 6.77 (s, 1 H), 7.00 (s, 1 H), 7.29 (t, *J*=8.80 Hz, 1 H), 7.42 - 7.50 (m, 1 H), 7.51 - 7.60 (m, 1 H), 7.65 (d, J=8.22 Hz, 1 H), 8.36 (d, J=8.80 Hz, 1 H).

### BIOLOGICAL TESTS

### fluorescent calcium flux assay

### Cells:

Jurkat E6-1 cells were obtained from ATCC (number TIB-152™) and maintained as ATCC recommend; in medium RPMI-1640 with 10% FBS at 37°C and 5% CO₂.

### Assay:

Before the assay, Jurkat cells were grown at a density of 2 millions cells/ml in cell culture media (RPMI with 10% FBS) in a T-175 flask at 37°C and 5% CO2. The day of the assay, cells were harvested by centrifugation (5 minutes at 1500 rpm) and the pellet were suspended in Ca²⁺ free assay buffer (HBSS w/o Ca²⁺ and Mg²⁺, 20mM Hepes and 1 mM MgCl2, pH 7.4) at a density of 5 million cells/ml.

Calcium depletion was initiated by the addition to the cell suspension of Thapsigarguin at a final assay concentration of 10µM. 20µl of the cellular suspension containing Thapsigargin (100.000 cells/well) were plated into 384 well plates (corning 3711) and incubated at RT for 15 minutes.

Test compounds were diluted in DMSO 100% to get a 10⁻²M stock solution and serial dilutions 1/3 were performed in the same solvent. Then, 20µl of test compounds, diluted 75 times in Ca²⁺ free assay buffer, were added to the cells and incubated at RT for 1h.

Once this incubation was completed, 5 µl of dye solution (FLIPR Calcium 5 Express Kit, Molecular Devices), prepared as Molecular Devices recommend, were added into the assay plates followed by an extra incubation period of 1 hour at room temperature. After the second incubation period, assay plates were placed into the FLIPR Tetra from Molecular Devices. Fluorescence was measured at a wavelength of 525nm after the dye excitation at 485nm. The initial baseline was measured during 20 seconds, and then 8µl of a 13mM Calcium solution in assay buffer were added (2mM final calcium concentration) except for basal wells, where assay buffer without calcium was added. At this time, changes in the cellular fluorescent were measured for an extra 80 seconds.

Peak and base line were taken for the calculation of the ratio (Peak/Base Line). Total, basal and compound well ratio values were used to calculate %Inhibition.

### In vitro assay of cytokine release from T cells

Jurkat E6-1 cells were obtained from American Type Culture Collection (ATCC) and maintained in complete medium with 10% fetal bovine serum (FBS) at 37°C/5%CO₂. Cells were plated in 100 µL of Dubelcco's Modified Eagle Medium (DMEM) with 1% FBS in a 96 well plate at a density of 1x10⁶ cells/well and 50 µL of test compound was added. Cells were then stimulated by the addition of 50 µL of phytohemagglutinin (PHA) (final concentration 20 µg/ml) and incubated at 37°C/5%CO₂ for 20 hours. On the following day, the supernatants were collected and assayed for IL-2 levels by ELISA according to the manufacturer's protocols. The IC₅₀ value was calculated as the concentration at which 50% of secreted IL-2 in vehicle is inhibited.

| **Example** | **Ca²⁺ flux IC₅₀ (nM)** | **Jurkat IL2 IC₅₀ (nM)** |
|---|---|---|
| Ex. 1 | 71 | 40 |
| Ex. 2 | 357 | 100 |
| Ex. 8 | 800 | 41 |
| Ex. 9 | 370 | 210 |
| Ex. 13 | 120 | 56 |
| Ex. 14 | 53 | 36 |
| Ex. 16 | 390 | 130 |
| Ex. 30 | 360 | 110 |
| Ex. 69 | 580 | 114 |
| Ex. 73 | 88 | 117 |
| Ex. 85 | 640 | 860 |

As it can be seen form the table, compounds of the present invention are potent inhibitors of Ca²⁺ influx in Jurkat cells. Preferred compounds of the invention possess an IC₅₀ value for the inhibition of Ca²⁺ influx of less than 10 µM, preferably less than 1 µM, more preferably less than 500 nM, even more preferably less than 100 nM, being most preferable less than 50 nM.

Compounds described herein have shown activity inhibiting IL2 secretion with an IC₅₀ value of less than 1 µM, preferably less than 500 nM, more preferably less than 100 nM and even more preferably less than 50 nM.

### COMBINATIONS

The compounds of formula (I) can also be used in combination with other drugs known to be effective in the treatment of the diseases or the disorders indicated above. For example the compounds of the present invention can be combined with (a) anticholinergics, such as aclidinium bromide or tiotropium, (b) corticosteroids, or gluococorticoids such as prednisone or methylprednisolone, (c) antihistamines, such as ebastine, ranitidine, ABT-239 or JNJ 7777120; (d) beta-2 agonists, such as salmeterol or formoterol, (e) chemokine receptor antagonists, such as maraviroc or enfuvirtide, (f) CRth2 antagonists, (g) leukotriene receptor antagonists, (h) JAK inhibitors such as tofacitinib or INCB018424, (i) Syk inhibitors such as R-343, (j) phosphosdiesterase IV inhibitors such as roflumilast or revamilast, (k) dual beta-2 adrenoceptor agonist/M3 receptor antagonist (MABA compounds) such as GSK-961081, (l) p38 Inhibitors such as ARRY-797, (m) PKC inhibitors such as NVP-AEB071, (n) 5-lipoxygenase activating protein inhibitors, such as veliflapon, (o) 5-lipoxygenase inhibitors, (p) CYSLTR1 antagonists, such as montelukast, pranlukast or zafirlukast; (q) CYSLTR2 antagonists, such as pranlukast, zafirlukast or tipilukast; (r) BLT1 antagonists, (s) BLT2 antagonists, (t) thromboxane A2 antagonists such as ramatroban, (u) DP1 receptor antagonists, such as laropiprant, (v) DP1 receptor agonists, such as BW-245C, (w) IP receptor agonists, such as RO-1138452, (x) Anti-IgE, such as omalizumab, (y) IL5 antibody, such as mepolizumab, (z) leukotriene formation inhibitors, (aa) bronchodilators, such as pirbuterol, epinephrine, ephedrine or salbutamol; (bb) decongestants, such as ephedrine, levo-methamphetamine, naphazoline, oxymetazoline, phenylephrine, phenylpropanolamine, propylhexedrine, pseudoephedrine, synephrine or tetrahydrozoline; (cc) mucolytics such as acetylcysteine, ambroxol, bromhexine, carbocisteine, domiodol, eprazinone, erdosteine, letosteine, neltenexine, sobrerol, stepronin or tiopronin; (dd) antitussives, such as dextromethorphan, (ee) analgesics such as aspirin, paracetamol, rofecoxid, celecoxib, morphine, codeine, oxycodone, hydrocodone, dihydromorphine or flupirtine; and (ff) expectorants such antimony pentasulfide, guaiacolsulfonate, guaifenesin, potassium iodide or tyloxapol.

Accordingly, another embodiment of the invention is a combination product comprising (i) at least a compound as defined previously, and (ii) one or more active ingredients as described above, for simultaneous, separate or sequential use in the treatment of the human or animal body.

A preferred embodiment of the invention is a combination product as defined before for the treatment or prevention of pathological conditions, diseases and disorders known to be susceptible of amelioration by inhibition of CRAC channel activity, in particular wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease; as well as a method for treating a subject afflicted with a pathological condition or disease susceptible to amelioration by inhibition of CRAC channel activity in particular wherein the pathological condition or disease as described above; which comprises administering to said subject an effective amount of a combination product as defined before.

As indicated above, the compounds or pharmaceutically acceptable salts, N-oxides, or or isotopically-labeled derivates thereof, according to the invention may also be used in combination with another therapeutically active agent as defined above.

The amount of each active which is required to achieve a therapeutic effect will, of course, vary with the particular active, the route of administration, the subject under treatment, and the particular disease or disorder being treated.

The active ingredients may be administered from 1 to 6 times a day, sufficient to exhibit the desired activity. Preferably, the active ingredients are administered once or twice a day, most preferably once a day.

Examples of suitable β2-agonists that can be combined with the compounds of formula (I) are terbutaline sulphate, eformoterol fumarate, formoterol fumarate, formoterol hydrochloride; bambuterol, ibuterol, isoprenaline hydrochloride, dopexamine, metaprotenerol, tulobuterol hydrochloride, procaterol hydrochloride, sibenadet hydrochloride, mabuterol hydrochloride, albuterol sulphate, salbutamol sulphate, salmefamol, salmeterol, salmeterol xinafoate, carmoterol, carmoterol hydrochloride, (R)-albuterol hydrochloride, levalbuterol hydrochloride; levosalbutamol hydrochloride; levalbuterol sulphate; levosalbutamol sulphate; (-)-salbutamol hydrochloride, formoterol, (R,R)-formoterol tartrate; arformoterol tartrate, sulfonterol, bedoradrine sulphate, indacaterol, indacaterol maleate, indacaterol acetate, indacaterol xinafoate, abediterol (LAS100977), abediterol napadislate, abediterol mesylate, trantinterol hydrochloride, milveterol hydrochloride, olodaterol, fenoterol hydrobromide, rimoterol hydrobromide, riproterol hydrochloride, vilanterol, broxaterol, pirbuterol hydrochloride, bitolterol mesylate, clenbuterol hydrochloride, AZD-3199, GSK-159802; GSK-597901, GSK-678007, GSK-961081; 4-[2-[3-(1H-Benzimidazol-1-yl)-1,1-dimethylpropylamino]-1-hydroxyethyl]-2-(4-methoxybenzylamino)phenal, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-domethoxyphenyl)-2-methyl-2-propylamino]ethanol, 1-[2H-5-hydroxy-3-oxo-4H-1,4-benzoxazin-8-yl]-2-[3-(4-n-butyloxyhenyl)-2-methyl-2-propylamino]ethanol, KUL-1248, KUL-7211; HOKU-81, SM-11044, RP-58802B; AR-C68164AA, AR-C68475AA; AR-C89855AA; AR-C69457AA; and compounds described in PCT patent applications Nos. WO 2007/124898, WO 2006/122788A1, WO 2008/046598, WO 2008095720, WO 2009/068177 and WO 2010/072354.

Examples of suitable corticosteroids and glucocorticoids that can be combined with the compounds of formula (I) are prednisolone, methylprednisolone, dexamethasone, dexamethasone acetate, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone, mometasone furoate, rimexolone, prednisolone farnesylate, ciclesonide, butixocort propionate, RS-85095, CGP-13774, GW-250495, deltacortisone, NO-Prednisolone, NO-Budesonide, etiprednol dicloacetate, QAE-397, 7beta-OH-EPIA, RPR-106541, deprodone propionate, fluticasone, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11beta-hydroxy-17alpha-[2-(methylsulfanyl)acetoxy]-4-pregnene-3,20-dione, desisobutyrylciclesonide, hydrocortisone hydrocortisone sodium succinate, prednisolone sodium phosphate and hydrocortisone probutate, prednisolone sodium metasulfobenzoate and clobetasol propionate.

Examples of suitable M3 antagonists (anticholinergics) that can be combined with the compounds of formula (I) are tiotropium salts, oxitropium salts, flutropium salts, ipratropium salts, glycopyrronium salts, trospium salts, zamifenacin, revatropate, espatropate, darotropium bromide, CI-923, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-1 4695), N-[1-(6-Aminopyridin-2-ylmethyl)piperidin-4-yl]-2(R)-[3,3-difluoro-1 (R)-cyctopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1-methyl-1-[2-oxo-2-(3-thienyl)ethyl]pyrrolidinium iodide, N-[1-(3-Hydroxybenzyl)-1-methylpiperidinium-3(S)-yl]-N-[N-(4-(isopropoxycarbonyl)phenyl]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-fluorophenyl)-2-oxoimidazalidin-1-yl]-1-methyl-1-(2-phenylethyl)pyrrolidinium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Examples of suitable are anti-histamines that can be combined with the compounds of formula (I) are methapyrilene, mequitazine, azelastine hydrochloride, acrivastine, emedastine difumarate, emedastine fumarate, loratadine, cyproheptadine hydrochloride, diphenhydramine hydrochloride, doxepin hydrochloride, promethazine hydrochloride, levocabastine hydrochloride, desloratadine, cinnarizine, setastine hydrochloride, mizolastine, ebastine, cetirizine hydrochloride, epinastine hydrochloride, olopatadine hydrochloride, bepotastine besilate, triprolidine hydrochloride, rupatadine fumarate, fexofenadine hydrochloride, levocetirizine dihydrochloride, ketotifen, azatadine maleate, dimethindene maleate, clemastine fumarate, alcaftadine, bilastine, vapitadine hydrochloride, AZD-1744, GSK-1 004723D, GSK-835726 or SUN-1334H.

Examples of suitable PDE4 inhibitors that can be combined with the compounds of formula (I) are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, revamilast, ronomilast, (R)-(+)-4-[2-(3-Cyclopentyloxy-4-methoxyphenyl)-2-phenylethyl]pyridine (CDP-840), N-(3,5-Dichloro-4-pyridinyl)-2-[1-(4-fluorobenzyl)-5-hydroxy-1H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethy))naphthalen-1-yl]-1-(2-methoxyethyl)pyridin-2(1H)-one (T-440), (-)-trans-2-[3'-[3-(N-Cyclopropylcarbamoyl)-4-oxo-1,4-dihydro-1,8-naphthyridin-1-yl]-3-fluorobiphenyl-4-yl]cyclopropanecarboxylic acid, MK-0873, CDC-801, GSK-356278, TA-7906, CP-80633, PL-554, NIK-616, GPD-1116, D4396, UK-500001, BLX-914, 2-carbomethoxy-4-cyano-4-(3-cyclopropylmethoxy-4-difluroromethoxyphenyl) cyclohexan1-one, *cis*[4-cyano-4-(3-cyclopropy)methoxy-4-difluoromethoxyphenyl)cyclohexan-1-ol, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the compounds claimed in the PCT patent applications number WO 03/097613, WO 2004/058729, WO 2005/049581, WO 2005/123693, WO 2005/123692, and WO 2010/069504.

Examples of suitable leukotriene antagonist that can be combined with the compounds of formula (I) are CYSLTR1 antagonists, such as montelukast, pranlukast or zafirlukast; or CYSLTR2 antagonists, such as pranlukast, zafirlukast or tipilukast.

Examples of suitable CRTH₂ antagonist that can be combined with the compounds of formula (I) are ramatroban, AMG-009, OC-000459).

Examples of suitable Syk kinase inhibitors that can be combined with the compounds of formula (I) are fosfamatinib (from Rigel), R-348 (from Rigel), R-343 (from Rigel), R-112 (from Rigel), piceatannol, 2-(2-Aminoethylamino)-4-[3-(trifluoromethyl)phenylamino] pyrimidine-5-carboxamide, R-091 (from Rigel), 6-[5-Fluoro-2-(3,4,5-trimethoxyphenylamino)pyrimidin-4-ylamino]-2,2-dimethyl-3,4-dihydro-2H-pyrido[3,2-b][1,4]oxazin-3-one benzenesulfonate (R-406 from Rigel), 1-(2,4,6-Trihydroxyphenyl)-2-(4-methoxyphenyl)ethan-1-one, N-[4-[6-(Cyclobutylamino)-9H-purin-2-ylamino]phenyl]-N-methylacetamide (QAB-205 from Novartis), CI-1002 (from Pfizer), VRT-750018 (from Vertex), PRT-062607, 2-[7-(3,4-Dimethoxyphenyl)imidazo[1,2-c]pyrimidin-5-ylamino]pyridine-3-carboxamide dihydrochloride (BAY-61-3606 from Bayer) and AVE-0950 (from Sanofi-Aventis).

The compounds of formula (I) and the combinations of the invention may be used in the treatment of respiratory, skin and inflammatory diseases, wherein the use of a CRAC modulador channel inhibitor is expected to have a beneficial effect, for example an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease

The active compounds in the combination product may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be administered in the morning and the other (s) later in the day. Or in another scenario, one or two actives could be administered twice daily and the other (s) once daily, either at the same time as one of the twice-a-day dosing occurred, or separately. Preferably at least two, and more preferably all, of the actives would be administered together at the same time. Preferably, at least two, and more preferably all actives would be administered as an admixture.

### PHARMACEUTICAL COMPOSITIONS

Compounds of the invention intended for pharmaceutical use may be administered as crystalline or amorphous products, or mixtures thereof. They may be obtained, for example, as solid plugs, powders, or films by methods such as precipitation, crystallization, freeze drying, spray drying, or evaporative drying.
Microwave or radio frequency drying may be used for this purpose.

Pharmaceutical compositions according to the present invention comprise the compounds of the invention in association with a pharmaceutically acceptable diluent or carrier.

As used herein, the term pharmaceutical composition refers to a mixture of one or more of the compounds described herein, or physiologically/pharmaceutically acceptable salts, or N-oxides, or isotopically-labeled derivates thereof, with other chemical components, such as physiologically/pharmaceutically acceptable carriers and excipients. The purpose of a pharmaceutical composition is to facilitate administration of a compound to an organism.

As used herein, a physiologically/pharmaceutically acceptable diluent or carrier refers to a carrier or diluent that does not cause significant irritation to an organism and does not abrogate the biological activity and properties of the administered compound.

A pharmaceutically acceptable excipient refers to an inert substance added to a pharmaceutical composition to further facilitate administration of a compound.

The invention further provides pharmaceutical compositions comprising the compounds of the invention in association with a pharmaceutically acceptable diluent or carrier together with one or more other therapeutic agents such as the previously described for use in the treatment of a pathological condition or disease susceptible to amelioration by inhibition of CRAC channel modulators.

The invention is also directed to pharmaceutical compositions of the invention for use in the treatment of a pathological disease or disorder susceptible to amelioration by inhibition of CRAC channel modulators, in particular wherein the pathological disease or disorder is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease.

The invention also provides a method of treatment of a pathological condition or disease susceptible to amelioration by inhibition of CRAC channel modulators, in particular wherein the pathological condition or disease as defined above, comprising administering a therapeutically effective amount of a pharmaceutical composition of the invention.

The present invention also provides pharmaceutical compositions which comprise, as an active ingredient, at least a compound of formula (I) or a pharmaceutically acceptable salt, solvate, N-oxide or deuterated derivative thereof in association with a pharmaceutically acceptable excipient such as a carrier or diluent. The active ingredient may comprise 0.001% to 99% by weight, preferably 0.01 % to 90% by weight, of the composition depending upon the nature of the formulation and whether further dilution is to be made prior to application. Preferably the compositions are made up in a form suitable for oral, inhalation, topical, nasal, rectal, percutaneous or injectable administration.

Pharmaceutical compositions suitable for the delivery of compounds of the invention and methods for their preparation will be readily apparent to those skilled in the art. Such compositions and methods for their preparation can be found, for example, in Remington: The Science and Practice of Pharmacy, 21 st Edition, Lippincott Williams & Wilkins, Philadelphia, Pa., 2001.

The pharmaceutically acceptable excipients which are admixed with the active compound or salts of such compound, to form the compositions of this invention are well-known per se and the actual excipients used depend inter alia on the intended method of administering the compositions. Examples, without limitation, of excipients include calcium carbonate, calcium phosphate, various sugars and types of starch, cellulose derivatives, gelatin, vegetable oils and polyethylene glycols.

Additional suitable carriers for formulations of the compounds of the present invention can be found in Remington: The Science and Practice of Pharmacy, 21 st Edition, Lippincott Williams & Wilkins, Philadelphia, Pa., 2001; or in Handbook of Pharmaceutical Excipients, 6th ed., published by Pharmaceutical Press and American Pharmacists Association, 2009.

### i) Oral Administration

The compounds of the invention may be administered orally (peroral administration; per os (latin)). Oral administration involve swallowing, so that the compound is absorbed from the gut and delivered to the liver via the portal circulation (hepatic first pass metabolism) and finally enters the gastrointestinal (GI) tract.

Compositions for oral administration may take the form of tablets, retard tablets, sublingual tablets, capsules, inhalation aerosols, inhalation solutions, dry powder inhalation, or liquid preparations, such as mixtures, solutions, elixirs, syrups or suspensions, all containing the compound of the invention; such preparations may be made by methods well-known in the art. The active ingredient may also be presented as a bolus, electuary or paste.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For tablet dosage forms, depending on dose, the drug may make up from 1 wt% to 80 wt% of the dosage form, more typically from 5 wt% to 60 wt% of the form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl- substituted hydroxypropyl cellulose, starch, pregelatinized starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinized starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate. Tablets may also optionally include surface active agents, such as sodium lauryl sulfate and polysorbate 80, and glidants such as silicon dioxide and talc. When present, surface active agents are typically in amounts of from 0.2 wt% to 5 wt% of the tablet, and glidants typically from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally are present in amounts from 0.25 wt% to 10 wt%, preferably from 0.5 wt% to 3 wt% of the tablet. Other conventional ingredients include anti-oxidants, colorants, flavoring agents, preservatives and taste-masking agents.

Exemplary tablets contain up to about 80 wt% drug, from about 10 wt% to about 90 wt% binder, from about 0 wt% to about 85 wt% diluent, from about 2 wt% to about 10 wt% disintegrant, and from about 0.25 wt% to about 10 wt% lubricant. Tablet blends may be compressed directly or by roller to form tablets. Tablet blends or portions of blends may alternatively be wet-, dry-, or melt-granulated, melt congealed, or extruded before tabletting. The final formulation may include one or more layers and may be coated or uncoated; or encapsulated.

The formulation of tablets is discussed in detail in "Pharmaceutical Dosage Forms: Tablets, Vol. 1 ", by H. Lieberman and L. Lachman, Marcel Dekker, N.Y., 1980.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Solid formulations for oral administration may be formulated to be immediate and/or modified release. Modified release formulations include delayed-, sustained-, pulsed-, controlled-, targeted and programmed release.

Liquid formulations include suspensions, solutions, syrups and elixirs. Such formulations may be used as fillers in soft or hard capsules and typically include a carrier, for example, water, ethanol, polyethylene glycol, propylene glycol, methylcellulose, or a suitable oil, and one or more emulsifying agents and/or suspending agents. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent. Liquid formulations may also be prepared by the reconstitution of a solid, for example, from a sachet.

### ii) Oral mucosal administration

The compounds of the invention can also be administered via the oral mucosal. Within the oral mucosal cavity, delivery of drugs is classified into three categories: (a) sublingual delivery, which is systemic delivery of drugs through the mucosal membranes lining the floor of the mouth, (b) buccal delivery, which is drug administration through the mucosal membranes lining the cheeks (buccal mucosa), and (c) local delivery, which is drug delivery into the oral cavity.

Pharmaceutical products to be administered via the oral mucosal can be designed using mucoadhesive, quick dissolve tablets and solid lozenge formulations, which are formulated with one or more mucoadhesive (bioadhesive) polymers (such as hydroxy propyl cellulose, polyvinyl pyrrolidone, sodium carboxymethyl cellulose, hydroxy propyl methyl cellulose, hydroxy ethyl cellulose, polyvinyl alcohol, polyisobutylene or polyisoprene); and oral mucosal permeation enhancers (such as butanol, butyric acid, propranolol, sodium lauryl sulphate and others)

### iii) Inhaled administration

The compounds of the invention can also be administered by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomizer (preferably an atomizer using electrohydrodynamics to produce a fine mist), or nebulizer, with or without the use of a suitable propellant, such as 1 ,1 ,1 ,2-tetrafluoraethane or 1 ,1 ,1 ,2,3,3,3-heptafluoropropane. For intranasal use, the powder may include a bioadhesive agent, for example, chitosan or cyclodextrin.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 0.001-5000 mg, more preferably 0.01-1000 mg of active ingredient or the equivalent amount of a pharmaceutically acceptable salt thereof. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (Ex. EP0069715) or disks (Ex. GB 2041763; EP 0424790; DE 4239402 and EP 0674533), rotatable cylinders (Ex. EP 0166294; GB 2165159 and WO 92/09322) and rotatable frustums (Ex. WO 92/00771), all having cavities which have to be filled with powder from the container. Other multi dose devices have measuring slides (Ex. US 5201308 and WO 97/00703) or measuring plungers with a local or circumferential recess to displace a certain volume of powder from the container to a delivery chamber or an air conduit (Ex. EP 0505321, WO 92/04068 and WO 92/04928), or measuring slides such as the Genuair® (formerly known as Novolizer SD2FL), which is described the following patent applications Nos: WO97/000703, WO03/00325 and WO2006/008027.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (Ex. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with. Such atomiser is the respimat® which is described, for example, in PCT Patent Applications Nos. WO 91/14468 and WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetra-fluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants (eg oleic acid or lecithin) and cosolvens (eg ethanol). Pressurised formulations will generally be retained in a canister (e.g. an aluminium canister) closed with a valve (e.g. a metering valve) and fitted into an actuator provided with a mouthpiece.

Medicaments for administration by inhalation desirably have a controlled particle size. The optimum particle size for inhalation into the bronchial system is usually 1-10 µm, preferably 2-5 µm. Particles having a size above 20 µm are generally too large when inhaled to reach the small airways. To achieve these particle sizes the particles of the active ingredient as produced may be size reduced by conventional means e.g. by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate.

Pressurized aerosol compositions will generally be filled into canisters fitted with a valve, especially a metering valve. Canisters may optionally be coated with a plastics material e. g. a fluorocarbon polymer as described in WO96/32150. Canisters will be fitted into an actuator adapted for buccal delivery.

### iv) Nasal mucosal administration

The compounds of the invention may also be administered via the nasal mucosal.

Typical compositions for nasal mucosa administration are typically applied by a metering, atomizing spray pump and are in the form of a solution or suspension in an inert vehicle such as water optionally in combination with conventional excipients such as buffers, anti-microbials, tonicity modifying agents and viscosity modifying agents.

### v) Parenteral Administration

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water.

The preparation of parenteral formulations under sterile conditions, for example, by lyophilization, may readily be accomplished using standard pharmaceutical techniques well known to those skilled in the art. The solubility of compounds of the invention used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

### vi) Topical Administration

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated; see, for example, J Pharm Sci, 88 (10), 955-958 by Finnin and Morgan (October 1999). Other means of topical administration include delivery by electroporation, iontophoresis, phonophoresis, sonophoresis and microneedle or needle-free injection.

Formulations for topical administration may be formulated to be immediate and/or modified release. Modified release formulations include delayed-, sustained-, pulsed-, controlled-, targeted and programmed release.

### vii) Rectal/Intravaginal Administration

Compounds of the invention may be administered rectally or vaginally, for example, in the form of a suppository, pessary, or enema. Cocoa butter is a traditional suppository base, but various alternatives may be used as appropriate. Formulations for rectal/vaginal administration may be formulated to be immediate and/or modified release. Modified release formulations include delayed-, sustained-, pulsed-, controlled-, targeted and programmed release.

### viii) Ocular Administration

Compounds of the invention may also be administered directly to the eye or ear, typically in the form of drops of a micronized suspension or solution in isotonic, pH-adjusted, sterile saline. Other formulations suitable for ocular and aural administration include ointments, biodegradable {e.g. absorbable gel sponges, collagen) and nonbiodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

### ix) Other Technologies

Compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

The amount of the active compound administered will be dependent on the subject being treated, the severity of the disorder or condition, the rate of administration, the disposition of the compound and the discretion of the prescribing physician. However, an effective dosage is typically in the range of 0.01-3000 mg, more preferably 0.5-1000 mg of active ingredient or the equivalent amount of a Pharmaceutical acceptable salt thereof per day. Daily dosage may be administered in one or more treatments, preferably from 1 to 4 treatments, per day.

The pharmaceutical formulations may conveniently be presented in unit dosage form and may be prepared by any of the methods well known in the art of pharmacy.

Preferably the composition is in unit dosage form, for example a tablet, capsule or metered aerosol dose, so that the patient may administer a single dose.

The active substance compositions according to the invention are preferably administered in the form of compositions for inhalation delivered with the help of inhalers, especially dry powder inhalers; however, any other form of nasal, topical, parenteral or oral application is possible. Here, the application of inhaled compositions embodies one of the preferred application form, especially in the therapy of obstructive lung diseases or for the treatment of asthma. Other preferred application form is dermal and transdermal administration, especially in the therapy of atopic dermatitis or psoriasis.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

### FORMULATION EXAMPLES

The following preparations forms are cited as formulation examples:

### Formulation Example 1 (Oral suspension)

| **Ingredient** | **Amount** |
|---|---|
| Active Compound | 3 mg |
| Citric acid | 0,5 g |
| Sodium chloride | 2,0 g |
| Methyl paraben | 0,1 g |
| Granulated sugar | 25 g |
| Sorbitol (70% solution) | 11 g |
| Veegum K | 1,0 g |
| Flavoring | 0,02 g |
| Dye | 0,5 mg |
| Distilled water | q.s. to 100 mL |

### Formulation Example 2 (Hard gelatine capsule for oral administration)

| **Ingredient** | **Amount** |
|---|---|
| Active Compound | 1 mg |
| Lactose | 150 mg |
| Magnesium stearate | 3 mg |

### Formulation Example 3 (Gelatin cartridge for inhalation)

| **Ingredient** | Amount |
|---|---|
| Active Compound (micronized) | 0,2 mg |
| Lactose | 25 mg |

### Formulation Example 4 (Formulation for inhalation with a DPI)

| **Ingredient** | **Amount** |
|---|---|
| Active Compound (micronized) | 15 mg |
| Lactose | 3000 mg |

### Formulation Example 5 (Formulation for a MDI)

| **Ingredient** | **Amount** |
|---|---|
| Active Compound (micronized) | 10g |
| 1,1,1,2,3,3,3-heptafluoro-n-propane | q.s. to 200 ml |

Modifications, which do not affect, alter, change or modify the essential aspects of the compounds, combinations or pharmaceutical compositions described, are included within the scope of the present invention.

## Claims

1. A compound of formula (I), or pharmaceutically acceptable salts, N-oxides and isotopically-labeled derivates thereof. 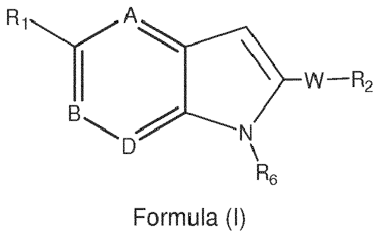 wherein,
A, B and D each independently represent a nitrogen atom or a -CH group, wherein at least one of A, B and D represent a nitrogen atom, and wherein at least one of A, B and D represent a -CH group;
W represents a linker selected from a direct bond and a -CH₂- group,
R₁ represents a monocyclic or bicyclic C₆-C₁₄ aryl group, a monocyclic or bicyclic 5- to 14- membered heteroaryl group containing at least one heteroatom selected from O, S and N, a tetrahydropyridinyl group or a bicyclyl group which is a monocyclic C₆-C₉aryl or 5- to 9- membered heteroaryl group fused to a saturated or unsaturated C₅₋₉ cycloalkyl or to a saturated or unsaturated 5- to 9- membered heterocyclyl group, said heteroaryl or heterocyclyl group containing at least one heteroatom selected from O, S and N,
wherein the aryl, heteroaryl, tetrahydropyridinyl and the bicyclyl group are unsubstituted or substituted with one or more substituents selected from a halogen atom, a cyano group, a -(CH₂)₁₋₃CN group, a -(CH₂)_{n'}-OR₃ group, a -(CH₂)_{n'}-C(O)-(CH₂)₁₋₃-CN group, a -(CH₂)_{n'}-C(O)-(CH₂)ₙ-R₃ group, a -(CH₂)_{n'}-C(O)-(CH₂)ₙ-NR₄R₅ group, a -(CH₂)_{n'}-S(O)₂(CH₂)ₙR₃ group or a -(CH₂)_{n'}-S(O)₂(CH₂)ₙNR₄R₅ group, a linear or branched C₁-C₆ alkyl group, a C₁-C₄ haloalkyl group, a C₁-C₄ hydroxyalkyl group, a C₃-C₇ cycloalkyl group, a monocyclic or bicyclic C₆-C₁₄ aryl group, a monocyclic or bicyclic 5- to 14- membered heteroaryl group containing at least one heteroatom selected from O, S and N, and a 5- to 14- membered heterocyclyl group containing at least one heteroatom selected from O, S and N,
R₂ represents a linear or branched C₁-C₉ alkyl group, a monocyclic or bicyclic C₃-C₁₀ cycloalkyl group, a monocyclic or bicyclic C₃-C₁₀ cycloalkenyl group, a monocyclic or bicyclic C₆-C₁₄ aryl group, a monocylic or bicyclic 5- to 14-membered heteroaryl group containing at least one heteroatom selected from N, O and S, or a monocylic saturated or unsaturated 4- to 8- membered heterocyclyl group containing at least one heteroatom selected from O, S and N,
wherein the cycloalkyl, cycloalkenyl, aryl, heteroaryl and heterocyclyl groups are unsubstituted or substituted by one or more substituents selected from a halogen atom; a cyano group; a hydroxyl group; an oxo group, an ethylenedioxy group, a - (CH₂)₁-₃-CN group, a -(CH₂)_{n'}-OR₃ group, a -(CH₂)_{n'}-C(O)-(CH₂)₁₋₃-CN group, a - (CH₂)_{n'}-C(O)OR₃ group, a -(CH₂)_{n'}-C(O)-(CH₂)ₙ-R₃ group, a -(CH₂)_{n'}-C(O)-(CH₂)ₙ-NR₄R₅ group, a -(CH₂)_{n'}-S(O)₂(CH₂)ₙR₃ group or a -(CH₂)_{n'}-S(O)₂(CH₂)ₙNR₄R₅ group, a linear or branched C₁-C₆ alkyl group; a linear or branched C₁-C₄ haloalkyl group; a linear or branched C₁-C₄ hydroxyalkyl group; a C₃-C₇ cycloalkyl group; a monocyclic or bicyclic C₆-C₁₄aryl group; a 5- to 14- membered heteroaryl group containing at least one heteroatom selected from O, S and N; and a 5- to 14-membered heterocyclyl group containing at least one heteroatom selected from O, S and N;
each n' and n are 0, 1 or 2
R₃ represents a linear or branched C₁-C₄ haloalkyl group, a linear or branched C₁-C₄ hydroxyalkyl group or a linear or branched C₁-C₆ alkyl group,
R₄ and R₅ each independently represent a hydrogen atom, a linear or branched C₁-C₄ haloalkyl group, a linear or branched C₁-C₄ hydroxyalkyl group or a linear or branched C₁-C₆ alkyl group, or R₄ and R₅ together with the nitrogen atom to which both R₄ and R₅ groups are bonded form a 4- to 7- membered, saturated N-containing heterocyclyl group optionally containing a further heteroatom selected from O, N and S and which heterocyclyl group is unsubstituted or substituted with one or more substituents selected from a halogen atom, a hydroxyl group, a cyano group, a -CHF₂ group or a - CF₃ group;
R₆ is selected from the group consisting of a hydrogen atom and a C group, wherein C represents a prodrug-functional group,
wherein the compound of formula (I) is not one of 2,5-diphenyl-3H-pyrrolo[2,3-c]pyridine, 2-methyl-5-phenyl-1H-pyrrolo[2,3-b]pyridine, 2-ethyl-5-(3-fluorophenyl)-1H-pyrrolo[2,3-b]pyridine and 2-methyl-5-phenyl-1H-pyrrolo[2,3-c]pyridine.

2. A compound according to claim 1, wherein
A is a nitrogen atom, B is a -CH group and D is a -CH group; or A is a nitrogen atom, B is a -CH group and D is a nitrogen atom; or A is a -CH group, B is a -CH group and D is a nitrogen atom; or A is a -CH group, B is a nitrogen atom and D is a -CH group; or A is a nitrogen atom, B is a nitrogen atom and D is a -CH group; or A is a -CH group, B is a nitrogen atom and D is a nitrogen atom.

3. A compound according to claim 1 or claim 2, wherein
A is a nitrogen atom, B is a -CH group and D is a -CH group; or A is a nitrogen atom, B is a -CH group and D is a nitrogen atom; or A is a -CH group, B is a -CH group and D is a nitrogen atom; or A is a -CH group, B is a nitrogen atom and D is a -CH group.

4. A compound according to any one of the preceding claims, wherein W represents a direct bond.

5. A compound according to any one of the preceding claims, wherein R₁ represents a monocyclic C₆-C₈ aryl group, a monocyclic 5- to 8- membered heteroaryl group containing at least one heteroatom selected from O, S and N, a tetrahydropyridinyl group or a bicyclyl group which is a monocyclic C₆-C₉aryl fused to a monocyclic C₅₋₉ cycloalkyl or to a monocyclic 5- to 9- membered heterocyclyl group, said heterocyclyl group containing at least one heteroatom selected from O, S and N,
wherein the aryl, heteroaryl, tetrahydropyridinyl and the bicyclyl group are unsubstituted or substituted by one or more substituents selected from a halogen atom, a cyano group, a -(CH₂)_{n'}-OR₃ group, a -(CH₂)_{n'}-C(O)-(CH₂)ₙ-R₃ group, a - (CH₂)_{n'}-C(O)-(CH₂)ₙ-NR₄R₅ group, a -(GH₂)_{n'}-S(O)2(CH₂)ₙR₃ group or a -(CH₂)_{n'}-S(O))₂(CH₂)ₙNR₄R₅ group, a linear or branched C₁-C₄ alkyl group, a C₁-C₄ haloalkyl group, a C₁-C₄ hydroxyalkyl group, a C₃-C₇ cycloalkyl group, a monocyclic C₆-C₈ aryl group, a monocyclic 5- to 8- membered heteroaryl group containing at least one heteroatom selected from O, S and N, a 5- to 14- membered heterocyclyl group containing at least one heteroatom selected from O, S and N, wherein n, n', R₃, R₄ and R₅ are as defined in claim 1.

6. A compound according to claim 5, wherein R₁ represents a monocyclic C₆-C₈ aryl group, a monocyclic 5- to 8- membered heteroaryl group containing at least one heteroatom selected from O, S and N or a bicyclyl group which is a monocyclic C₆-C₉ aryl fused to a 5- to 9- membered heterocyclyl group, said heterocyclyl group containing at least one heteroatom selected from O, S and N,
wherein the aryl, heteroaryl and the bicyclyl group are unsubstituted or substituted by one or more substituents selected from a halogen atom, a -(CH₂)_{n'}-OR₃ group, a - (CH₂)_{n'}-C(O)-(CH₂)ₙ-NR₄R₅ group or a -(CH₂)_{n'}-S(O)₂(CH₂)ₙNR₄R₅ group, a C₁-C₂ alkyl group, and a C₁-C₂ haloalkyl group, wherein R₃ represent a C₁-C₂ haloalkyl group, a C₁-C₂ hydroxyalkyl group and a C₁-₂ alkyl group, and R₄ and R₅ independently are selected from the group consisting of a hydrogen atom, a C₁-C₂ haloalkyl group, and a C₁-₂ alkyl group, or R₄ and R₅ together with the nitrogen atom to which both R₄ and R₅ groups are bonded form a 4- to 6- membered, saturated N-containing heterocyclyl group optionally containing a further heteroatom selected from O, N and S and which heterocyclyl group is unsubstituted or substituted with one or more substituents selected from a halogen atom, a hydroxyl group, a cyano group or a -CF₃ group; wherein n' and n are as defined in claim 1.

7. A compound according to claim 6, wherein R₁ represents a monocyclic 5- to 8-membered N-containing heteroaryl group and further containing at least one heteroatom selected from O, S and N or a bicyclyl group which is a phenyl group fused to a 5- to 6- membered heterocyclyl group containing at least one heteroatom selected from O, S and N,
wherein the heteroaryl and the bicyclyl group are unsubstituted or substituted by one or two substituents selected from a chlorine atom, fluorine atom, a -OR₃ group, a-S(O)₂NR₄R₅ group, a C₁-C₂ alkyl group, and a C₁-C₂ haloalkyl group, wherein R₃ represents a C₁₋₂ alkyl group and a C₁-C₂ haloalkyl group, and R₄ and R₅ independently are selected from the group consisting of a hydrogen atom and a C₁₋₂ alkyl group, preferably the aryl and the bicyclyl group are unsubstituted or substituted by one or two substituents selected from a chlorine atom, fluorine atom, a methyl group, a methoxy group, a CF₃ group and a -S(O)₂NR₄R₅ group, wherein R₄ and R₅ independently are selected from the group consisting of a hydrogen atom and a methyl group.

8. A compound according to any one of the preceding claims, wherein R₂ represents a linear or branched C₁-C₆ alkyl group, a monocyclic C₃-C₇ cycloalkyl group, a monocyclic C₆-C₈aryl group, a monocylic 5- to 8-membered heteroaryl group containing at least one heteroatom sleected from O and N, a saturated or unsaturated 4- to 8- membered heterocyclyl group containing at least one heteroatom selected from O and N,
wherein the cycloalkyl, aryl, heteroaryl and heterocyclyl groups are unsubstituted or substituted by one or more substituents selected from a halogen atom; a cyano group; a hydroxyl group; an oxo group, a -(CH₂)_{n'}-OR₃ group, a -(CH₂)_{n'}-C(O)O-R₃ group, a -(CH₂)_{n'}-C(O)-(CH₂)ₙ-NR₄R₅ group, a -(CH2)_{n'}-S(O)₂(CH₂)R₃ group or a - (CH₂)_{n'}-S(O)₂(CH₂)ₙNR₄R₅ group, a linear or branched C₁-C₄ alkyl group; a C₁-C₂ haloalkyl group; a C₁-C₂ hydroxyalkyl group; wherein R₃ represent a linear or branched C₁-C₄ haloalkyl group, a linear or branched C₁-C₄ hydroxyalkyl group and a C₁₋₂ alkyl group, and R₄ and R₅ independently are selected from the group consisting of a hydrogen atom, a C₁-C₂ haloalkyl group, and a C₁₋₂ alkyl group, or R₄ and R₅ together with the nitrogen atom to which both R₄ and R₅ groups are bonded form a 4- to 6- membered, saturated N-containing heterocyclyl group optionally containing a further heteroatom selected from O and N and which heterocyclyl group is unsubstituted or substituted with one or more substituents selected from a halogen atom, a hydroxyl group, a cyano group or a -CF₃ group; wherein n' and n are as defined in claim 1.

9. A compound according to claim 8, wherein R₂ represents a monocyclic C₄-C₆ cycloalkyl group and a monocyclic C₆-C₈ aryl group, wherein the cycloalkyl, and the aryl groups are unsubstituted or substituted with one or two substituents selected from a fluorine atom, chlorine atom, a hydroxyl group; a methoxy group, a methyl group and a -CF₃ group, preferably R₂ represents a phenyl group , a cyclopentyl group or a cyclohexyl group which groups are optionally substituted with one or two substituents selected from a chlorine atom, a fluorine atom, a methyl group and a methoxy group.

10. A compound according to any one of the preceding claims, wherein C is selected from the group consisting of a -(CH₂)-O-P(O)(OH)₂ and a -(CH₂)-O-(CO)R₇, wherein R₇ is selected from the group consisting of a linear or branched C₁₋₄ alkyl group and a C₃₋₇ cycloalkyl group, preferably, C represents a -(CH₂)-O-P(O)(OH)₂ group.

11. A compound according to any one of the preceding claims, wherein R₆ is selected from the group consisting of a hydrogen atom and a -(CH₂)-O-P(O)(OH)₂ group.

12. A compound of according to claim 1 or 2, selected from compounds of formula (Ia); 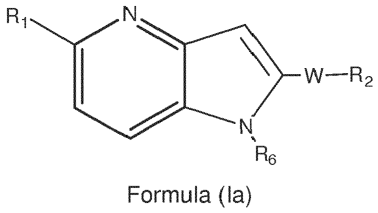 compounds of formula (Ib); 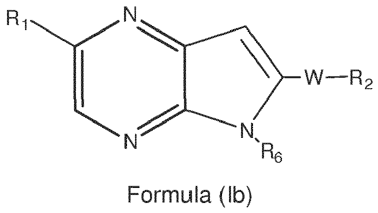 compounds of formula (Ic); 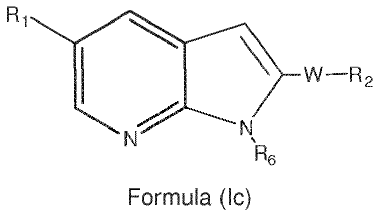 compounds of formula (Id); 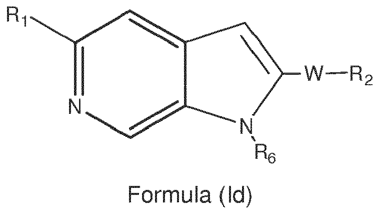 compounds of formula (Ie); 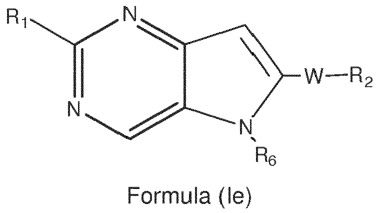 and compounds of formula (If); 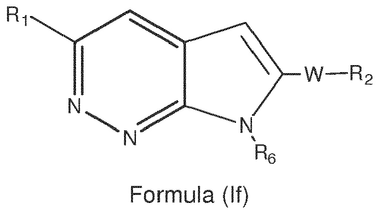 wherein W, R₁, R₂ and R₆ are as defined in any one of the preceding claims.

13. A compound according to claim 12, selected from compounds of formula (Ia); 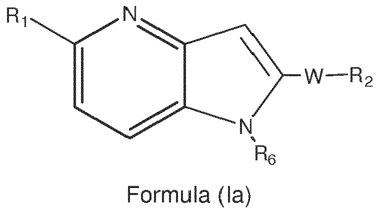 compounds of formula (Ib); 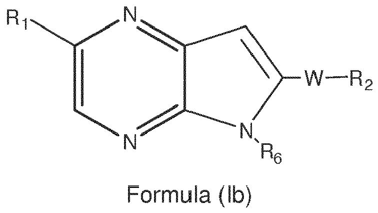 or compounds of formula (Ic);

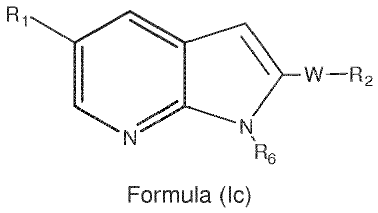

14. A compound according to claim 3, wherein:
W represents a direct bond or a -CH₂- group,
R₁ represents a phenyl group, a monocyclic 5- to 6- membered N-containing heteroaryl group and optionally further containing one or two nitrogen atom as heteroatom, or a bicyclyl group which is a phenyl group fused to a 5- to 9-membered saturated or unsaturated heterocyclyl group, said heterocyclyl group containing at least one heteroatom selected from O and N, wherein the phenyl, heteroaryl and the bicyclyl group are substituted with two or three substituents selected from a halogen atom, a -OR₃ group, a -S(O)₂NR₄R₅ group, a linear or branched C₁-C₆ alkyl group, a C₁-C₄ haloalkyl group and a monocyclic 5- to 7- membered heteroaryl group containing at least one heteroatom selected from O and N,
R₂ represents a linear or branched C₁-C₄ alkyl group, a C₃-C₁₀ cycloalkyl group, a C₄-C₆ cycloalkenyl group, monocyclic C₆-C₁₄aryl group, a monocylic a 5- to 6-membered heteroaryl group containing at once heteroatom selected from O and N or a monocyclic saturated or unsaturated 4- to 8- membered heterocyclyl group containing at least one heteroatom selected from O and N,
wherein the cycloalkyl, cycloalkenyl, aryl, heteroaryl and heterocyclyl groups are unsubstituted or substituted with one or more substituents selected from a halogen atom; a cyano group; a hydroxyl group; an oxo group, an ethylenedioxy group, a - OR₃ group, a -C(O)O-R₃ group, a -S(O)₂NR₄R₅ group; a linear or branched C₁-C₆ alkyl group or a C₁-C₄ hydroxyalkyl group;
R₃ represents a C₁-C₄ haloalkyl group, a C₁-C₄ hydroxyalkyl group or a linear or branched C₁-C₄alkyl group,
R₄ and R₅ each independently represent a hydrogen atom, or a linear or branched C₁-C₆ alkyl group, or R₄ and R₅ together with the nitrogen atom to which both R₄ and R₅ groups are bonded form an unsubstituted 4- to 7- membered saturated N-containing heterocyclyl group optionally containing a further heteroatom selected from O.
R₆ is selected from the group consisting of a hydrogen atom and a -(CH₂)-OP(O)(OH)₂ group.

15. A compound according to claim 1 which is one of:
2-((2-chloro-6-fluorophenyl)-5-(6-methoxy-4-methylpyridin-3-yl)-1H-pyrrolo[2,3-b]pyridine,
6-((2-chloro-6-fluorophenyl)-2-(6-methoxy-4-methylpyridin-3-yl)-5H-pyrrolo[2,3-b]pyrazine,
6-((2-chloro-6-fluorophenyl)-2-(1-methyl-3-(trifluoromethyl)-1H-pyrazol-5-yl)-5H-pyrrolo[2,3-b]pyrazine,
2-((2,6-difluorophenyl)-5-(6-methoxy-4-methylpyridin-3-yl)-1H-pyrrolo[2,3-b]pyridine, 2-cyclohexyl-5-(6-methoxy-4-methylpyridin-3-yl)-1H-pyrrolo[2,3-b]pyridine,
5-((6-methoxy-4-methylpyridin-3-yl)-2-(4-methylpyridin-3-yl)-1H-pyrrolo[2,3-b]pyridine,
6-((2-chloro-6-fluorophenyl)-2-(1-ethyl-3-(trifluoromethyl)-1H-pyrazol-5-yl)-5H-pyrrolo[2,3-b]pyrazine,
4-((6-(2-chloro-6-fluorophenyl)-5H-pyrrolo[2,3-b]pyrazin-2-yl)-N,N,3-trimethylbenzenesulfonamide,
3-((3,5-difluoro-4-(5-(6-methoxy-4-methylpyridin-3-yl)-1H-pyrrolo[2,3-b]pyridin-2-yl)phenoxy)propan-1-ol,
6-cyclohexyl-2-(1-methyl-3-(trifluoromethyl)-1H-pyrazol-5-yl)-5H-pyrrolo[2,3-b]pyrazine,
2-((2-chloro-6-fluorophenyl)-5-(6-methoxy-4-methylpyridin-3-yl)-1H-pyrrolo[3,2-b]pyridine,
2-((2-chloro-6-fluorophenyl)-5-(1-methyl-3-(trifluoromethyl)-1H-pyrazol-5-yl)-1H-pyrrolo[2,3-b]pyridine,
5-((6-chloro-2,2-difluorobenzo[d][1,3]dioxol-5-yl)-2-(2-chloro-6-fluorophenyl)-1H-pyrrolo[2,3-b]pyridine,
2-((2-chloro-6-fluorophenyl)-5-(1-methyl-3-(trifluoromethyl)-1H-pyrazol-5-yl)-1H-pyrrolo[3,2-b]pyridine,
6-cyclohexyl-2-(6-methoxy-4-methylpyridin-3-yl)-5H-pyrrolo[2,3-b]pyrazine,
2-cyclohexyl-5-(1-methyl-3-(trifluoromethyl)-1H-pyrazol-5-yl)-1H-pyrrolo[3,2-b]pyridine,
2-cyclohexyl-5-(6-methoxy-4-methylpyridin-3-yl)-1H-pyrrolo[3,2-b]pyridine,
2-((2-chloro-6-fluorophenyl)-5-(1,3-dimethyl-1H-pyrazol-5-yl)-1H-pyrrolo[3,2-b]pyridine,
2-((4-methyl-3-(2-propyl-1H-pyrrolo[3,2-b]pyridin-5-yl)phenyl)oxazole,
2-((2-chloro-6-fluorophenyl)-5-(1-ethyl-3-(trifluoromethyl)-1H-pyrazol-5-yl)-1H-pyrrolo[3,2-b]pyridine,
2-((2,6-difluorophenyl)-5-(1-methyl-3-(trifluoromethyl)-1H-pyrazol-5-yl)-1H-pyrrolo[3,2-b]pyridine,
5-((1-methyl-3-(trifluoromethyl)-1H-pyrazol-5-yl)-2-propyl-1H-pyrrolo[3,2-b]pyridine,
5-((5-methyl-3-(trifluoromethyl)-1H-pyrazol-1-yl)-2-propyl-1H-pyrrolo[3,2-b]pyridine,
2-cyclopentyl-5-(1-methyl-3-(trifluoromethyl)-1H-pyrazol-5-yl)-1H-pyrrolo[3,2-b]pyridine,
2-cyclohexyl-5-(5-methyl-3-(trifluoromethyl)-1H-pyrazol-1-yl)-1H-pyrrolo[3,2-b]pyridine,
2-((2-chlorophenyl)-5-(1-methyl-3-(trifluoromethyl)-1H-pyrazol-5-yl)-1H-pyrrolo[3,2-b]pyridine,
1-((5-(1-methyl-3-(trifluoromethyl)-1H-pyrazol-5-yl)-1H-pyrrolo[3,2-b]pyridin-2-yl)cyclohexanol,
5-((1-methyl-3-(trifluoromethyl)-1H-pyrazol-5-yl)-2-(4-methylpyridin-3-yl)-1H-pyrrolo[3,2-b]pyridine,
2-((2,6-difluorophenyl)-5-(5-methyl-3-(trifluoromethyl)-1H-pyrazol-1-yl)-1H-pyrrolo[3,2-b]pyridine,
2-cyclohexyl-5-(5-(difluoromethoxy)-3-(trifluoromethyl)-1H-pyrazol-1-yl)-1H-pyrrolo[3,2-b]pyridine,
5-((1-methyl-3-(trifluoromethyl)-1H-pyrazol-5-yl)-2-(1,4-dioxaspiro[4.5]dec-7-en-8-yl)-1 H-pyrrolo[3,2-b]pyridine,
tert-butyl 4-(5-(5-methyl-3-(trifluoromethyl)-1H-pyrazol-1-yl)-1H-pyrrolo[3,2-b]pyridin-2-yl)piperidine-1-carboxylate,
2-((5-(1-methyl-3-(trifluoromethyl)-1H-pyrazol-5-yl)-1H-pyrrolo[3,2-b]pyridin-2-yl)benzonitrile,
4-((5-(1-methyl-3-(trifluoromethyl)-1H-pyrazol-5-yl)-1H-pyrrolo[3,2-b]pyridin-2-yl)cyclohexanol,
2-((2-fluorophenyl)-5-(1-methyl-3-(trifluoromethyl)-1H-pyrazol-5-yl)-1H-pyrrolo[3,2-b]pyridine,
(2-((5-(1-methyl-3-(trifluoromethyl)-1H-pyrazol-5-yl)-1H-pyrrolo[3,2-b]pyridin-2-yl)phenyl)methanol,
2-((3,5-difluoro-4-(5-(1-methyl-3-(trifluoromethyl)-1H-pyrazol-5-yl)-1H-pyrrolo[3,2-b]pyridin-2-yl)phenoxy)ethanol,
3-((3,5-difluoro-4-(5-(1-methyl-3-(trifluoromethyl)-1H-pyrazol-5-yl)-1H-pyrrolo[3,2-b]pyridin-2-yl)phenoxy)propan-1-ol,
4-(((4-(2-(2-chloro-6-fluorophenyl)-1H-pyrrolo[2,3-b]pyridin-5-yl)-3-methylphenyl)sulfonyl)morpholine,
5-((1-methyl-3-(trifluoromethyl)-1H-pyrazol-5-yl)-2-(o-tolyl)-1H-pyrrolo[3,2-b]pyridine,
5-((1-methyl-3-(trifluoromethyl)-1H-pyrazol-5-yl)-2-(1,4-dioxaspiro[4.5]decan-8-yl)-1 H-pyrrolo[3,2-b]pyridine,
2-((3,6-dihydro-2H-pyran-4-yl)-5-(1-methy)-3-(trifluoromethyl)-1H-pyrazol-5-yl)-1H-pyrrolo[3,2-b]pyridine,
4-((5-(1-methyl-3-(trifluoromethyl)-1H-pyrazol-5-yl)-1H-pyrrolo[3,2-b]pyridin-2-yl)cyclohexanone,
4-((2-(2-chloro-6-fluorophenyl)-1H-pyrrolo[2,3-b]pyridin-5-yl)-N,N,3-trimethylbenzenesulfonamide,
2-((2-methoxyphenyl)-5-(1-methyl-3-(trifluoromethyl)-1H-pyrazol-5-yl)-1H-pyrrolo[3,2-b]pyridine,
5-((1-methyl-3-(trifluoromethyl)-1H-pyrazol-5-yl)-2-(tetrahydro-2H-pyran-4-yl)-1H-pyrrolo[3,2-b]pyridine,
N,N,3-trimethyl-4-(5-(1-methyl-3-(trifluoromethyl)-1H-pyrazol-5-yl)-1H-pyrrolo[3,2-b]pyridin-2-yl)benzenesulfonamide,
5-fluoro-2-(5-(1-methyl-3-(trifluoromethyl)-1H-pyrazol-5-yl)-1H-pyrrolo[3,2-b]pyridin-2-yl)benzonitrile,
2-((4,4-difluorocyclohexyl)-5-(1-methyl-3-(trifluoromethyl)-1H-pyrazol-5-yl)-1H-pyrrolo[3,2-b]pyridine,
5-((1-methyl-3-(trifluoromethyl)-1H-pyrazol-5-yl)-2-phenyl-1H-pyrrolo[3,2-b]pyridine,
2-((4-butoxy-2-methylphenyl)-5-(1-methyl-3-(trifluoromethyl)-1H-pyrazol-5-yl)-1H-pyrrolo[3,2-b]pyridine,
2-isobutyl-5-(1-methyl-3-(trifluoromethyl)-1H-pyrazol-5-yl)-1H-pyrrolo[3,2-b]pyridine,
2-((4-fluoro-2-methylphenyl)-5-(1-methyl-3-(trifluoromethyl)-1 H-pyrazol-5-yl)-1H-pyrrolo[3,2-b]pyridine,
2-((5-fluoro-2-methylphenyl)-5-(1-methyl-3-(trifluoromethyl)-1H-pyrazol-5-yl)-1H-pyrrolo[3,2-b]pyridine,
2-((2,3-difluorophenyl)-5-(1-methyl-3-(trifluoromethyl)-1H-pyrazol-5-yl)-1H-pyrrolo[3,2-b]pyridine,
2-fluoro-6-(5-(1-methyl-3-(trifluoromethyl)-1H-pyrazol-5-yl)-1H-pyrrolo[3,2-b]pyridin-2-yl)benzonitrile,
2-((5-Chloro-2-methylphenyl)-5-[1-methyl-3-(trifluoromethyl)-1*H*-pyrazol-5-yl]-1*H-*pyrrolo[3,2-*b*]pyridine,
2-((3-fluoropyridin-4-yl)-5-(1-methyl-3-(trifluoromethyl)-1H-pyrazol-5-yl)-1H-pyrrolo[3,2-b]pyridine,
2-((2-chloro-6-fluorophenyl)-5-(5-methyl-3-(trifluoromethyl)-1H-pyrazol-1-yl)-1H-pyrrolo[2,3-b]pyridine,
4-((2-(2-chlorophenyl)-1H-pyrrolo[3,2-b]pyridin-5-yl)-N,N,3-trimethylbenzenesulfonamide,
4-(((4-(6-(2-chloro-6-fluorophenyl)-5H-pyrrolo[2,3-b]pyrazin-2-yl)-3-methylphenyl)sulfonyl)morpholine,
2-((3-fluoro-2-methylphenyl)-5-(1-methyl-3-(trifluoromethyl)-1H-pyrazol-5-yl)-1H-pyrrolo[3,2-b]pyridine,
2-((2-chlorophenyl)-5-(6-methoxy-4-methylpyridin-3-yl)-1H-pyrrolo[2,3-c]pyridine,
2-((2-fluoro-6-methylphenyl)-5-(1-methyl-3-(trifluoromethyl)-1H-pyrazol-5-yl)-1H-pyrrolo[3,2-b]pyridine,
4-((6-(2-chloro-6-fluorophenyl)-5H-pyrrolo[2,3-b]pyrazin-2-yl)-3-methylbenzenesulfonamide,
2-((2-chloro-4-fluorophenyl)-5-(1-methyl-3-(trifluoromethyl)-1H-pyrazol-5-yl)-1H-pyrrolo[3,2-b]pyridine,
6-cyclopentyl-2-(6-methoxy-4-methylpyridin-3-yl)-5H-pyrrolo[2,3-b]pyrazine, 6-(2,6-difluorophenyl)-2-(6-methoxy-4-methylpyridin-3-yl)-5H-pyrrolo[2,3-b]pyrazine,
4-((6-(2-chloro-6-fluorophenyl)-5H-pyrrolo[2,3-b]pyrazin-2-yl)-N,3-dimethylbenzenesulfonamide,
2-((2-chloro-6-methylphenyl)-5-(1-methyl-3-(trifluoromethyl)-1H-pyrazol-5-yl)-1H-pyrrolo[3,2-b]pyridine,
2-((2-chloro-5-fluorophenyl)-5-(1 -methyl-3-(trifluoromethyl)-1H-pyrazol-5-yl)-1H-pyrrolo[3,2-b]pyridine,
2-benzyl-5-(1-methyl-3-(trifluoromethyl)-1H-pyrazol-5-yl)-1H-pyrrolo[3,2-b]pyridine,
5-chloro-6-(2-(2-chloro-6-fluorophenyl)-1H-pyrrolo[2,3-b]pyridin-5-yl)-2-methylbenzo[d]oxazole,
2-((2-chloro-3-fluorophenyl)-5-(1-methyl-3-(trifluoromethyl)-1H-pyrazol-5-yl)-1H-pyrrolo[3,2-b]pyridine,
2-((2-chlorophenyl)-5-(6-methoxy-4-methylpyridin-3-yl)-1H-pyrrolo[2,3-b]pyridine, 2-(2-fluorophenyl)-5-(6-methoxy-4-methylpyridin-3-yl)-1H-pyrrolo[2,3-b]pyridine,
2-((2-chloro-6-fluorophenyl)-5-(5-methyl-3-(trifluoromethyl)-1H-1,2,4-triazol-1-yl)-1H-pyrrolo[3,2-b]pyridine,
3,5-dimethyl-4-(5-(1-methyl-3-(trifluoromethyl)-1H-pyrazol-5-yl)-1H-pyrrolo[3,2-b]pyridin-2-yl)isoxazole,
6-((2,6-difluorophenyl)-2-(1-methyl-3-(trifluoromethyl)-1H-pyrazol-5-yl)-5H-pyrrolo[2,3-b]pyrazine,
2-((2-fluorophenyl)-5-(6-methoxy-4-methylpyridin-3-yl)-1H-pyrrolo[2,3-c]pyridine,
2-((2-chloro-5-fluorophenyl)-5-(6-methoxy-4-methylpyridin-3-yl)-1H-pyrrolo[2,3-b]pyridine,
2-((4-isopropoxy-2-methylphenyl)-5-(1-methyl-3-(trifluoromethyl)-1H-pyrazol-5-yl)-1 H-pyrrolo[3,2-b]pyridine,
2-((4-ethoxy-2-methylphenyl)-5-(1-methyl-3-(trifluoromethyl)-1H-pyrazol-5-yl)-1H-pyrrolo[3,2-b]pyridine,
2-((2-chloro-6-fluorophenyl)-5-(6-methoxy-4-methylpyridin-3-yl)-1H-pyrrolo[2,3-c]pyridine,
2-((2,6-difluorophenyl)-5-[1-methyl-3-(trifluoromethyl)-1*H*-pyrazol-5-yl]-1*H-*pyrrolo[2,3-*c*]pyridine,
2-((2-chloro-6-fluorophenyl)-5-(4-methyl-6-(trifluoromethyl)pyridin-3-yl)-1H-pyrrolo[2,3-b]pyridine,
2-((2-chloro-6-fluorophenyl)-5-(4,6-dimethoxypyridin-3-yl)-1H-pyrrolo[2,3-b]pyridine,
{2-((2-Chloro-6-fluorophenyl)-5-[1-methyl-3-(trifluoromethyl)-1*H*-pyrazol-5-yl]-1*H-*pyrrolo[3,2-*b*]pyridin-1-yl}methyl dihydrogen phosphate,
2-Cyclopentyl-5-(1-methyl-3-(trifluoromethyl)-1*H*-pyrazol-5-yl)-1*H*-pyrrolo[2,3-b]pyridine,
2-((2-Chloro-6-fluorophenyl)-5-(4,6-dimethoxypyridin-3-yl)-1*H*-pyrrolo[3,2-*b*]pyridine, 2-(2,6-Difluorophenyl)-5-(1-methyl-3-(trifluoromethyl)-1*H*-pyrazol-5-yl)-1*H-*pyrrolo[2,3-*b*]pyridine,
2-((2-Chloro-6-fluorophenyl)-5-(2-methyl-4-(methylsulfonyl)phenyl)-1*H*-pyrrolo[3,2-b]pyridine,
2-((2-Chlorobenzyl)-5-(1-methyl-3-(trifluoromethyl)-1*H*-pyrazol-5-yl)-1*H*-pyrrolo[3,2-b]pyridine,
2-((2-Chloro-6-fluorophenyl)-5-(1-methyl-5-(trifluoromethyl)-1*H*-1,2,4-triazol-3-yl)-1*H-*pyrrolo[3,2-*b*]pyridine,
4-((2-(2-Chlorophenyl)-1*H*-pyrrolo[3,2-*b*]pyridin-5-yl)-N,3-dimethylbenzenesulfonamide,
5-Chloro-6-(2-(2-chloro-6-fluorophenyl)-1H-pyrrolo[3,2-*b*]pyridin-5-yl)-2-methylbenzo[*d*]oxazole,
4-((2-(2-Chloro-6-fluorophenyl)-1*H*-pyrrolo[3,2-*b*]pyridin-5-yl)-*N*-methyl-3-(trifluoromethyl)benzenesulfonamide,
2-((2-Chloro-6-fluorophenyl)-5-(4-methyl-6-(trifluoromethyl)pyridin-3-yl)-1*H-*pyrrolo[3,2-*b*]pyridine,
4-((2-(2-Chloro-6-fluorophenyl)-1H-pyrrolo[3,2-b]pyridin-5-yl)-N,3-dimethylbenzamide,
Methyl 3-(2-(2-chloro-6-fluorophenyl)-1*H*-pyrrolo[2,3-*b*]pyridin-5-yl)-4-methyl-5,6-dihydropyridine-1 (2*H*)-carboxylate,
5-((2-(2-Chloro-6-fluorophenyl)-1*H*-pyrrolo[2,3-b]pyridin-5-yl)-*N*,4-dimethylpyridin-2-amine,
1-((4-(2-(2-Chloro-6-fluorophenyl)-1*H*-pyrrolo[2,3-*b*]pyridin-5-yl)-3-methylphenyl)-*N-*methylmethanesulfonamide,
2-Cyclopentyl-5-(6-(difluoromethoxy)-4-methylpyridin-3-yl)-1*H*-pyrrolo[3,2-b]pyridine,
2-((*tert*-Butyl)-5-(1-methyl-3-(trifluoromethyl)-1*H*-pyrazol-5-yl)-1*H*-pyrrolo[3,2-b]pyridine,
5-Chloro-6-(2-cyclopentyl-1*H*-pyrrolo[2,3-*b*]pyridin-5-yl)-2-methylbenzo[*d*]oxazole,
6-((2-Chloro-6-fluorophenyl)-2-(4-methyl-6-(trifluoromethyl)pyridin-3-yl)-5*H-*pyrrolo[2,3-*b*]pyrazine,
4-((6-(2-Chloro-6-fluorophenyl)-5*H*-pyrrolo[2,3-*b*]pyrazin-2-yl)-*N*-methyl-3-(trifluoromethyl)benzenesulfonamide, and
2-((2-Chloro-6-fluorophenyl)-5-(3-methyl-1-(phenylsulfonyl)-1*H*-pyrazol-4-yl)-1*H-*pyrrolo[2,3-*b*]pyridine
and pharmaceutically acceptable salts, N-oxides and isotopically-labeled derivates thereof.

16. A compound as defined in any one of claims 1 to 15, for use in the treatment of the human or animal body by therapy.

17. A compound as defined in any one of claims 1 to 15, for use in the treatment of a pathological condition or disease susceptible to amelioration by inhibiting calcium release-activated calcium channel (CRAC) activity.

18. A compound for use according to claim 17, wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease.

19. A pharmaceutical composition comprising a compound as defined in any one of claims 1 to 15 in association with a pharmaceutically acceptable diluent or carrier.

20. Use of a compound as defined in any one of claims 1 to 15, for the manufacture of a medicament for the treatment of a pathological condition or disease as defined in any one of the claims 17 and 18.

21. A method for treating a subject afflicted with a pathological condition or disease as defined in any one of claims 17 to 18, which comprises administering to said subject a therapeutically effective amount of a compound as defined in any one of claims 1 to 15, or a pharmaceutical composition as defined in claim 19.

22. A combination product comprising (i) a compound as defined in any one of claims 1 to 15; and (ii) another compound selected from:
a. Anticholinergics, such as aclidinium bromide or tiotropium,
b. Corticosteroids, or gluococorticoids such as prednisone or methylprednisolone,
c. Antihistamines, such as ebastine, ranitidine, ABT-239 or JNJ 7777120;
d. Beta-2 agonists, such as salmeterol or formoterol,
e. Chemokine receptor antagonists, such as maraviroc or enfuvirtide,
f. CRth2 antagonists,
g. Leukotriene receptor antagonists,
h. JAK inhibitors such as tofacitinib or INCB018424,
i. Syk inhibitors such as R-343,
j. Phosphosdiesterase IV inhibitors such as roflumilast or revamilast,
k. Dual beta-2 adrenoceptor agonist/M3 receptor antagonist (MABA compounds) such as GSK-961081,
l. p38 Inhibitors such as ARRY-797,
m. PKC inhibitors such as NVP-AEB071,
n. 5-lipoxygenase activating protein inhibitors, such as veliflapon,
o. 5-lipoxygenase inhibitors,
p. CYSLTR1 antagonists, such as montelukast, pranlukast or zafirlukast;
q. CYSLTR2 antagonists, such as pranlukast, zafirlukast or tipilukast;
r. BLT1 antagonists,
s. BLT2 antagonists,
t. Thromboxane A2 antagonists such as ramatroban,
u. DP1 receptor antagonists, such as laropiprant,
v. DP1 receptor agonists, such as BW-245C,
w. IP receptor agonists, such as RO-1138452,
x. Anti-IgE, such as omalizumab,
y. IL5 antibody, such as mepolizumab,
z. Leukotriene formation inhibitors,
aa. Bronchodilators, such as pirbuterol, epinephrine, ephedrine or salbutamol;
bb. Decongestants, such as ephedrine, levo-methamphetamine, naphazoline, oxymetazoline, phenylephrine, phenylpropanolamine, propylhexedrine, pseudoephedrine, synephrine or tetrahydrozoline;
cc. Mucolytics such as acetylcysteine, ambroxol, bromhexine, carbocisteine, domiodol, eprazinone, erdosteine, letosteine, neltenexine, sobrerol, stepronin or tiopronin;
dd. Antitussives, such as dextromethorphan,
ee. Analgesics such as aspirin, paracetamol, rofecoxid, celecoxib, morphine, codeine, oxycodone, hydrocodone, dihydromorphine or flupirtine; and
ff. Expectorants such antimony pentasulfide, guaiacoisulfonate, guaifenesin, potassium iodide or tyloxapol,
for simultaneous, separate or sequential use in the treatment of the human or animal body.
